# EUROPEAN PATENT SPECIFICATION

(11) **EP 3 952 852 B1**
(45) Date of publication and mention of the grant of the patent: **22.07.2026**
(21) Application number: 20786878.7
(22) Date of filing: 08.04.2020
(51) Int. Cl.: A61K 31/4525, A61K 31/454, A61K 31/138, A61K 31/401, A61K 31/42, A61P 25/22, A61P 25/24, A61K 31/445, A61K 31/453, A61K 45/06

(54) **PREVENTION OF STRESS-INDUCED FEAR AND DEPRESSIVE-LIKE BEHAVIOR WITH SEROTONIN 4 RECEPTOR AGONISTS**
VORBEUGUNG VON STRESSINDUZIERTER ANGST UND DEPRESSIVEM VERHALTEN MIT SEROTONIN-4-REZEPTORAGONISTEN
PRÉVENTION DE LA PEUR INDUITE PAR LE STRESS ET DU COMPORTEMENT DE TYPE DÉPRESSIF AVEC DES AGONISTES DU RÉCEPTEUR DE LA SÉROTONINE 4

(30) Priority: 09.04.2019 US 201962831517 P; 04.06.2019 US 201962857075 P; 04.10.2019 US 201962910859 P
(43) Date of publication of application: 16.02.2022
(73) Proprietor: The Trustees of Columbia University in the City of New York, New York, NY 10027 (US); The Research Foundation For Mental Hygiene, Inc., Menands, NY 12204 (US); Université Paris-Saclay, 91190 Saint-Aubin (FR); Institut National de la Santé et de la Recherche Médicale, 75013 Paris (FR)
(72) Inventor: DENNY, Christine, A., New York, NY 10032 (US); GARDIER, Alain, M., 91190 Saint-Aubin (FR); DAVID, Denis, J., 91190 Saint-Aubin (FR); MENDEZ-DAVID, Indira, 91190 Saint-Aubin (FR); FAYE, Charlène, 91190 Saint-Aubin (FR); CHEN, Briana, K., New York, NY 10032 (US)
(74) Representative: Elkington and Fife LLP
(86) International application number: PCT/US2020/027321
(87) International publication number: WO 2020/210393

(56) References cited:
- US-A1- 2006 116 341
- US-A1- 2009 170 899
- US-A1- 2009 170 899
- US-A1- 2018 325 844
- FAYE C ET AL: "Rapid anxiolytic effects of a serotonin type 4 receptor agonist involve prefrontal cortex/brainstem neural circuit recruitment", EUROPEAN NEUROPSYCHOPHARMACOLOGY, ELSEVIER SIENCE PUBLISHERS BV , AMSTERDAM, NL, vol. 28, 14 March 2018 (2018-03-14), XP085360174, ISSN: 0924-977X, DOI: 10.1016/J.EURONEURO.2017.12.040
- MENDEZ-DAVID INDIRA, DAVID DENIS J, DARCET FLAVIE, WU MELODY V, KERDINE-RÖMER SAADIA, GARDIER ALAIN M, HEN RENÉ: "Rapid Anxiolytic Effects of a 5-HT4 Receptor Agonist Are Mediated by a , Neurogenesis-Independent Mechanism", NEUROPSYCHOPHARMACOLOGY, vol. 39, no. 6, 24 June 2017 (2017-06-24), pages 1366 - 1378, XP055749228, DOI: 10.1038/npp.2013.332

## Description

### FIELD OF THE INVENTION

The present invention relates to a serotonin 4 receptor (5-hydroxytryptamine (serotonin) receptor 4, or 5-HT₄R) agonist composition, specifically a pharmaceutical composition comprising 4-amino-5-chloro-2,3-dihydro-N-[1-3-methoxypropyl)-4-piperidinyl]-7-benzofuran carboxamide monohydrochloride (prucalopride), 4-[4-[4-Tetrahydrofuran-3-yloxy)-benzo[d]isoxazol-3-yloxymethyl]-piperidin-1-ylmethyl]-tetrahydropyran-4-ol (PF-04995274), or combinations thereof, for use in a method for preventing stress-induced affective disorders or psychopathologies in a subject prior to a stressor, wherein the pharmaceutical composition is administered to the subject 48 hours to 3 weeks prior to the stressor.

### BACKGROUND OF THE INVENTION

Stress exposure is a significant factor for the development of major depressive disorder (MDD) and post-traumatic stress disorder (PTSD). According to the National Comorbidity Study, approximately 60% of men and 51% of women have been exposed to one or more traumatic events during their lifetime. It is estimated that 7.8% of the overall population experiences PTSD at some point in their lives, with females (10.4%) experiencing the disorder at significantly higher rates than males (5.0%) [1]. Women are 2-3 times more at risk of suffering from stress-related anxiety or depressive disorders than men [61]. Traditionally, affective disorders have been treated from a symptom-suppression approach. Existing drugs aim to mitigate the impact of these chronic diseases, but do not cure or prevent the disease itself. However, if drugs were developed that enhance stress resilience, they could potentially be used in at-risk populations to protect against stress-induced psychiatric disorders.

Anxiety disorders are among the most common psychiatric disorders, with a lifetime prevalence of over 25 % (A1) and an annual financial burden of more than $40 billion (A2). Benzodiazepines (BZDs) are effective at treating most anxiety disorders and have been the standard treatment for years, with over an 80% response in reducing acute anxiety in patients (A3). However, their long-term daily use has been associated with a risk for dependency and amnesia. Consequently, they are often replaced by chronic treatment with serotonergic agents such as Selective Serotonin Reuptake Inhibitors (SSRIs) pointing out a role of serotonin (5-HT) to treat anxiety. However, as SSRIs have a delayed onset of action of several weeks and a 40% non-responders rate in anxious patients (A4), there is a need to develop novel fast-acting anxiolytics.

We and others have recently reported that (*R*,*S*)-ketamine acts as a resilience enhancing drug (e.g., prophylactic) against stress when administered 1 week before stress in mice [2-6]. In addition, limited data in human patients have demonstrated (*R*,*S*)-ketamine's potential in preventing psychiatric disorders such as PTSD [7] and, perhaps in a dose-specific manner, post-partum depression (PPD) [8,9]. Prophylactic drug efficacy has been limited to (*R,S*)-ketamine until recently when Gould and colleagues reported that group II metabotropic glutamate receptor (mGlu_{2/3}) antagonists are also protective [10]. We have previously reported that the SSRI Flx is ineffective as a prophylactic, but it remains to be determined if other serotonergic drugs could be effective prophylactics and/or if the serotonergic system is involved in prophylactic efficacy.

The 5-HT₄Rs are a promising target for treating depression and anxiety. 5-HT₄Rs are metabotropic G-protein coupled receptors that stimulate the Gₛ/cyclic adenosine monophosphate (cAMP)/protein kinase A (PKA) signaling pathway in response to 5-HT [11-15]. 5-HT₄Rs are highly expressed in the periphery, including the heart and adrenal gland, as well as in the brain in areas such as the amygdala (AMG), medial prefrontal cortex (mPFC), nucleus accumbens (NAc), and hippocampus (HPC) [16,17]. 5-HT₄R knockout mice display increased anxiety-like behavior and depressive-like behavior, while activation of 5-HT₄Rs stimulates neurogenesis in the HPC and produces rapid-acting antidepressant-like effects [18-21]. However, if and how 5-HT₄Rs are involved in stress resilience has yet to be determined.

US 2009/0170899 A1 discloses a combination of a serotonin selective re-uptake inhibitor (SSRI) and an agonist of the serotonin 4 (5-HT₄) receptor to augment and/or provide faster onset of the therapeutic effect of the SSRI alone or administered with any other compound which causes an elevation in the level of extracellular serotonin (5-HT).
US 2009/0170899 A1 also discloses a pharmaceutical formulation comprising the afore-mentioned combination and a method and use of the combination in the treatment of depression, anxiety, obsessive compulsive disorder (OCD) or other disease or disorder responsive to a SSRI. Faye et al. (European Neuropsychopharmacology, Elsevier Science Publishers BV, Amsterdam, NL, (20180314), vol. 28) disclose that rapid anxiolytic effects of a serotonin type 4 receptor agonist involve prefrontal cortex/brainstem neural circuit recruitment.

There is an unmet need for effective prophylactic therapies to prevent the onset of stress-induced affective disorders.

### SUMMARY OF THE INVENTION

The present disclosure provides for pharmaceutical compositions for use in preventing or delaying a stress-induced affective disorder or stress-induced psychopathology in a subject in need thereof. The pharmaceutical compositions comprise an effective amount of prucalopride and/or PF-04995274, which are activators of serotonin 4 receptor (5-HT₄R) (e.g., an agonist of serotonin 4 receptor (5-HT₄R)) and are administered to the subject prior to a stressor.

The invention is set out in the appended claims. Specifically, the invention relates to a pharmaceutical composition comprising 4-amino-5-chloro-2,3-dihydro-N-[1-3-methoxypropyl)-4-piperidinyl]-7-benzofuran carboxamide monohydrochloride (prucalopride), 4-[4-[4-Tetrahydrofuran-3-yloxy)-benzo[d]isoxazol-3-yloxymethyl]-piperidin-1-ylmethyl]-tetrahydropyran-4-ol (PF-04995274), or combinations thereof, for use in a method for preventing a stress-induced affective disorder or stress-induced psychopathology selected from stress-induced fear and depressive-like behavior and associated affective disorders in a subject prior to a stressor, wherein the pharmaceutical composition is administered to the subject 48 hours to 3 weeks prior to the stressor. In certain embodiments, the pharmaceutic composition is administered to the subject about 72 hours to about 2 weeks prior to a stressor. In certain embodiments, the pharmaceutic composition is administered to the subject about 1 week prior to a stressor.

In certain embodiments, the pharmaceutic composition is administered to the subject once prior to a stressor.

In certain embodiments, the pharmaceutic composition is administered orally, intravenously, intranasally, or via injection to the subject.

Use of the pharmaceutical composition may further comprise administering to the subject an effective amount of an anti-depressant, an anxiolytic, or combinations thereof.

Use of the pharmaceutical composition may further comprise administering an effective amount of a selective serotonin reuptake inhibitor (SSRI), or a pharmaceutically acceptable salt or derivative thereof.

Use of the pharmaceutical composition may further comprise administering an effective amount of fluoxetine, paroxetine, sertraline, lithium, riluzole, prazosin, lamotrigine, ifenprodil, or combinations thereof.

The subject may be a mammal. In certain embodiments, the subject is a human. The subject may be female or male.

### BRIEF DESCRIPTION OF THE DRAWINGS

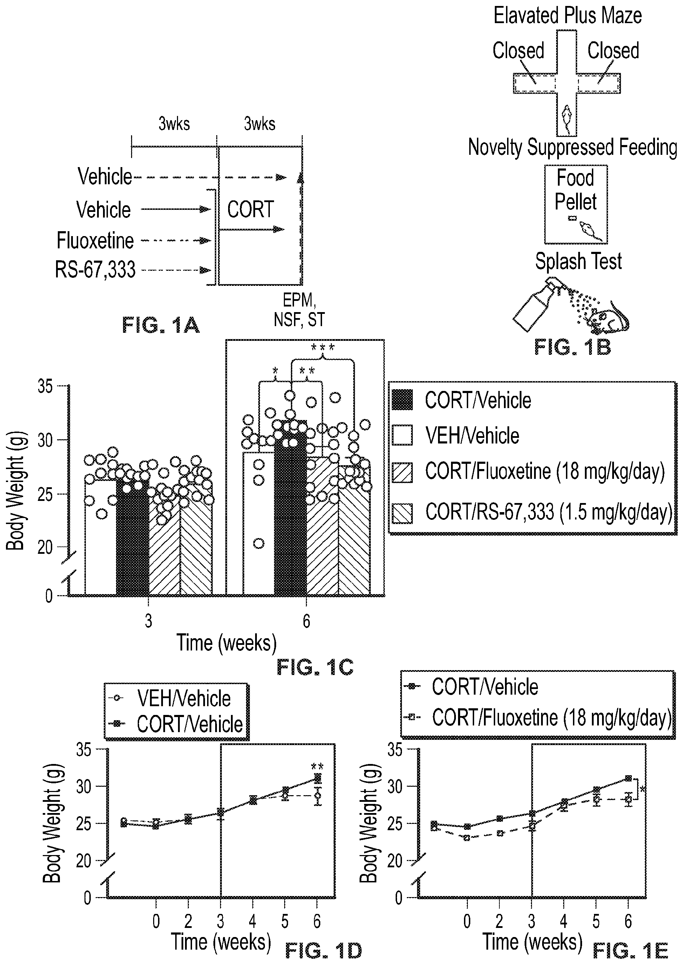

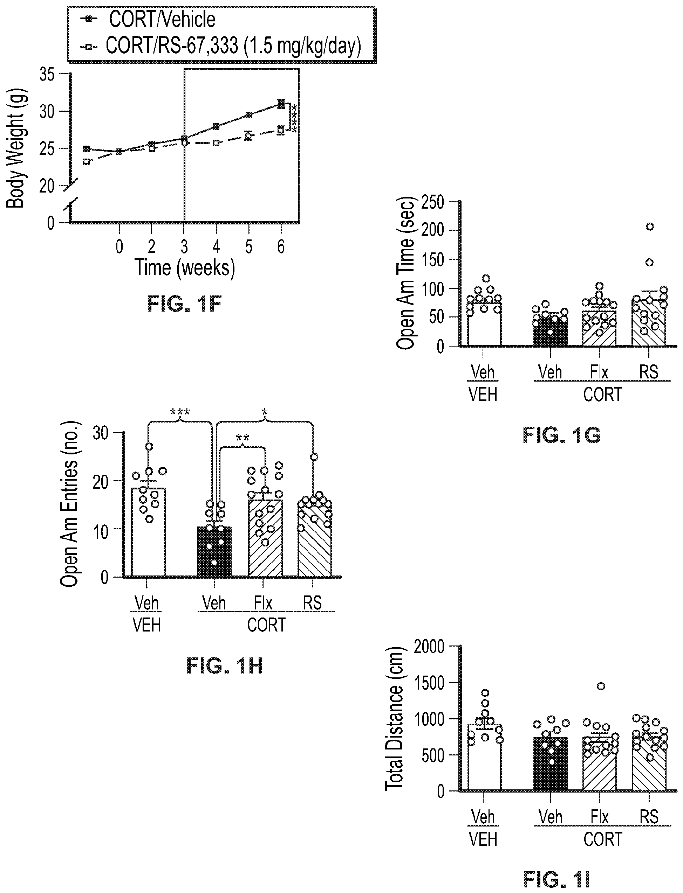

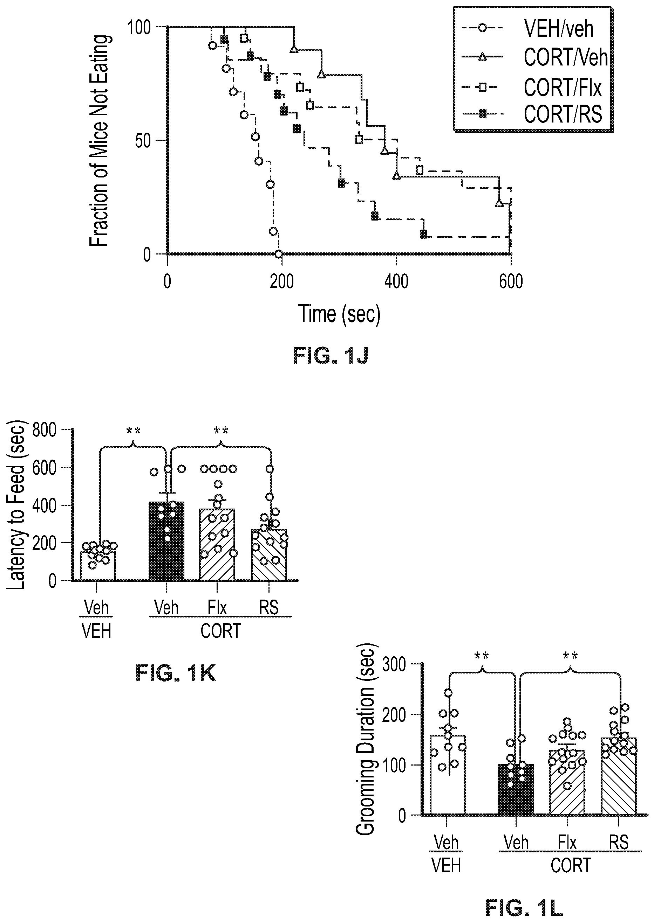

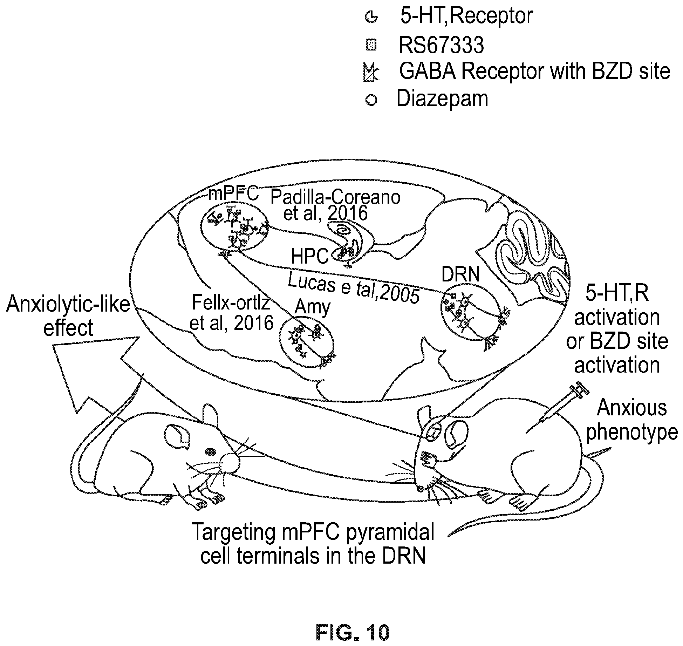

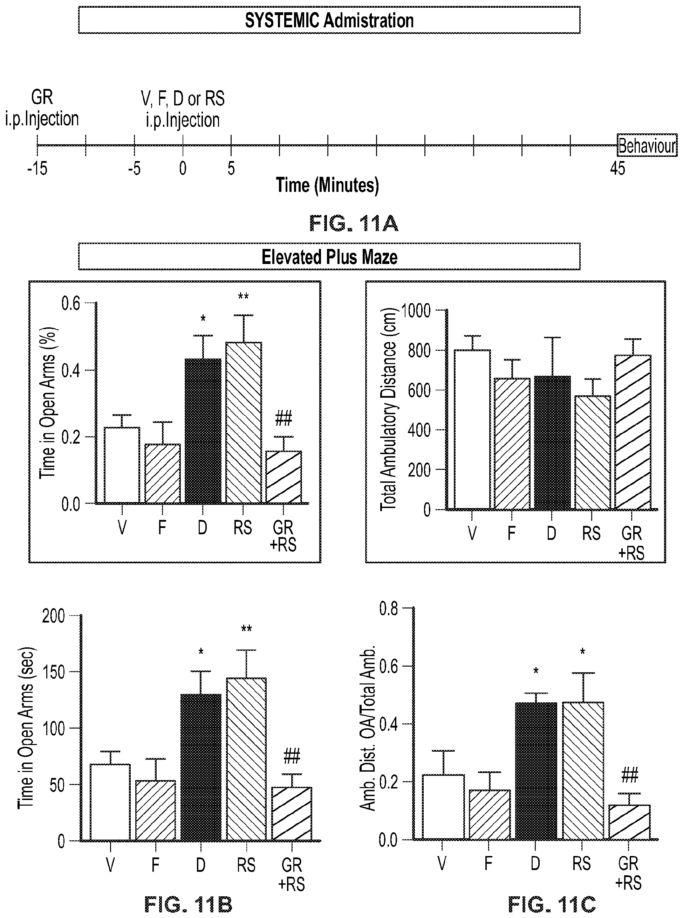

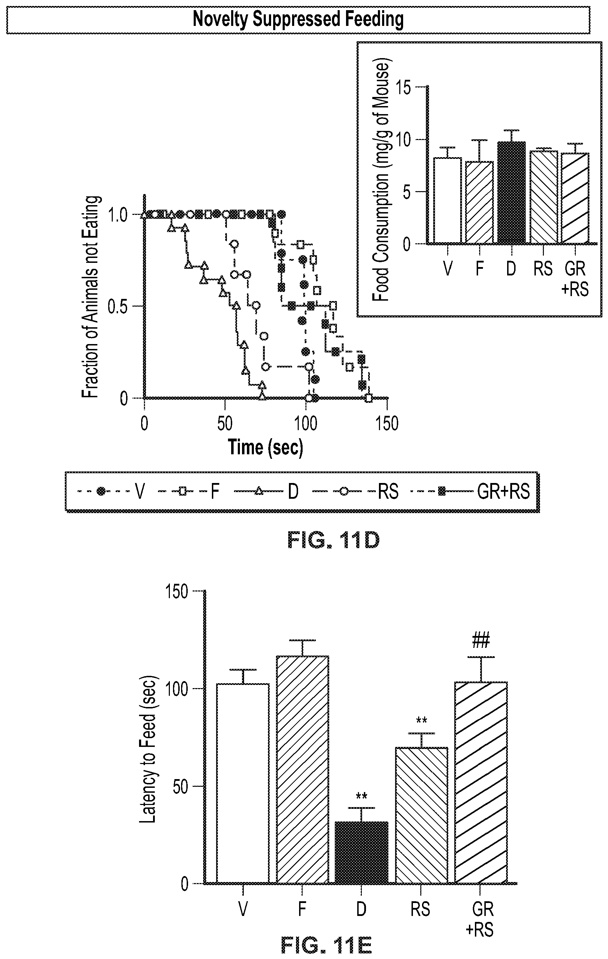

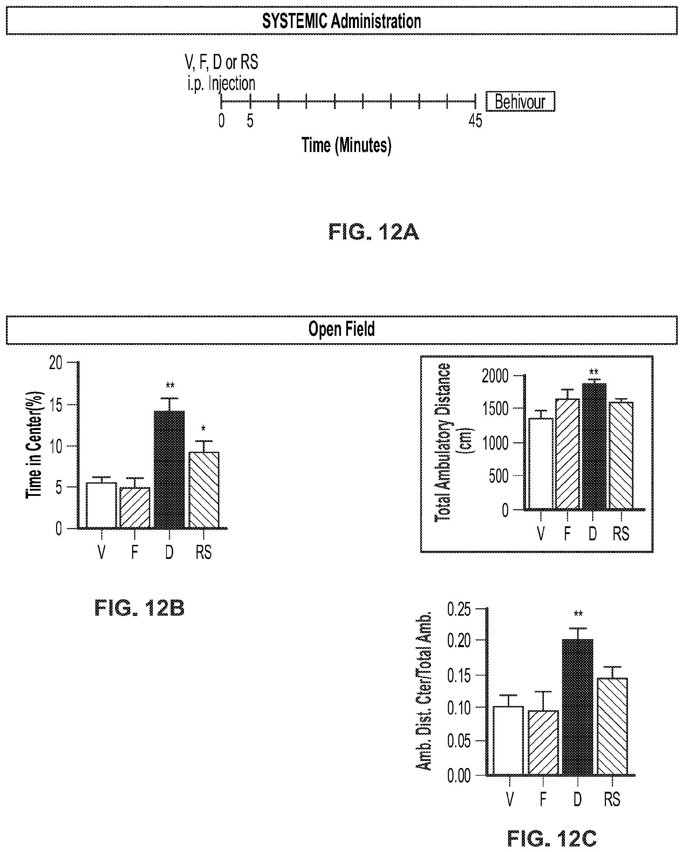

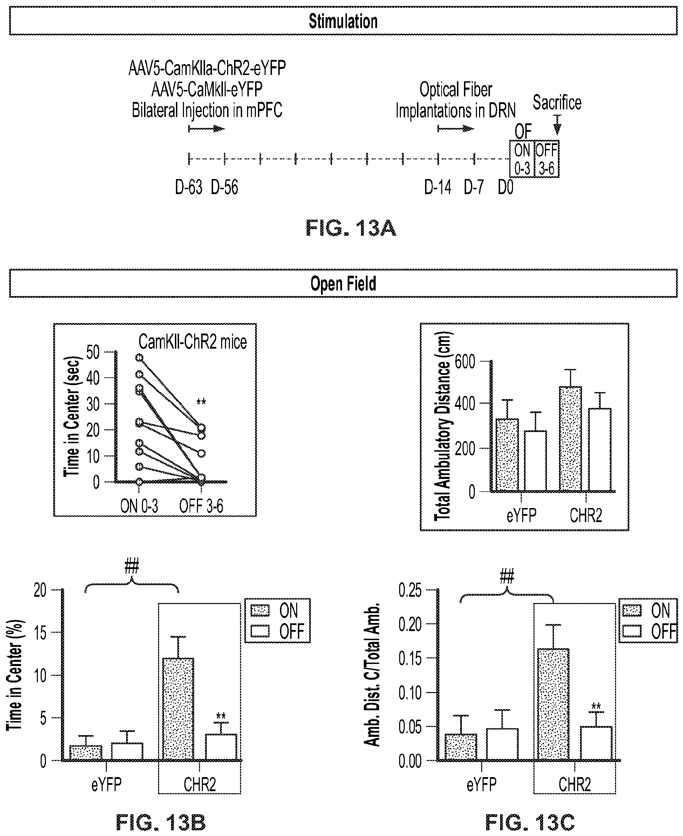

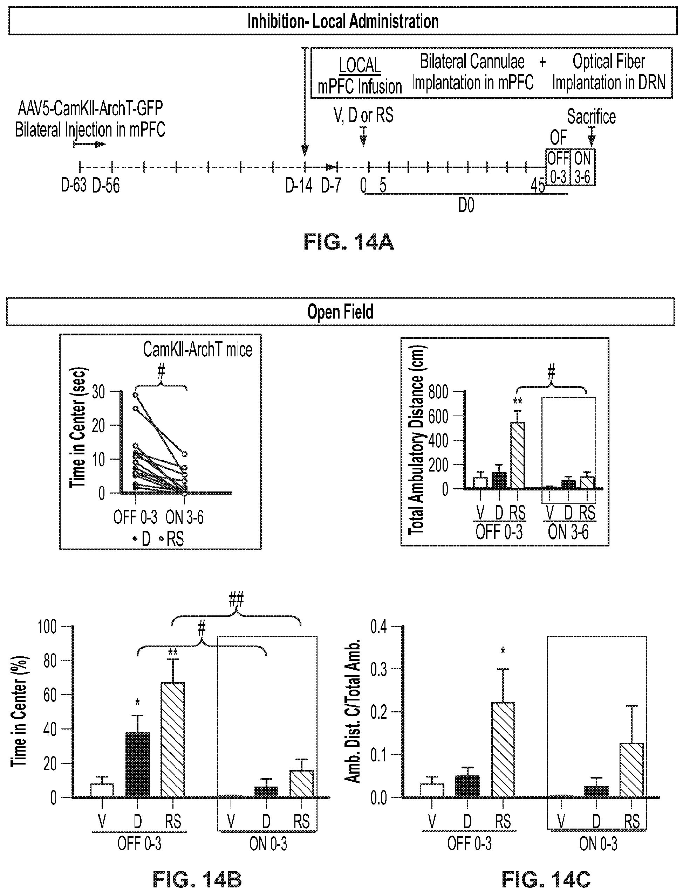

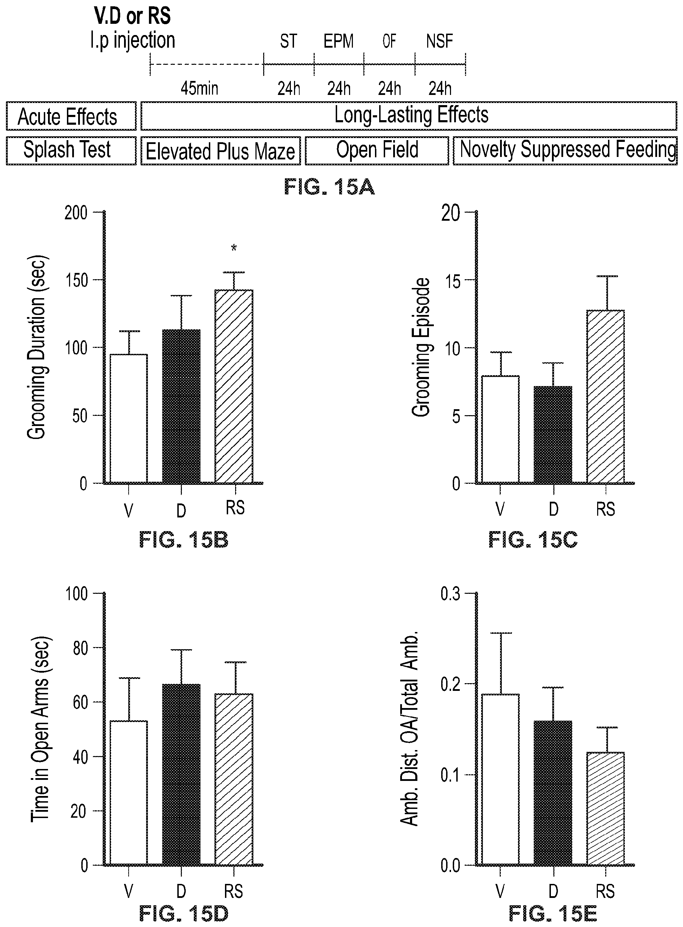

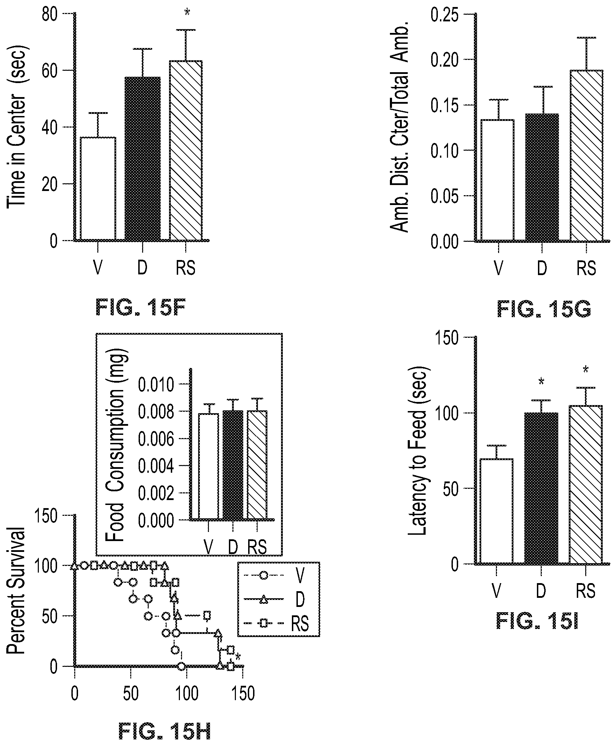

**Figures 1A-1L****. RS-67,333 protects against depressive- and anxiety-like behavior induced with a neuroendocrine model in male C57BL/6NTac mice.** (1A) Experimental design. (1B) Behavioral assays to test anxiety-like behavior (EPM, NSF) and depressive-like behavior (ST). (1C-1F) By Week 6, CORT administration resulted in increased body weight when compared with VEH administration. RS and Flx administration resulted in decreased body weight in CORT-treated mice. (1G) All groups of mice exhibited comparable amounts of time in the open arms of the EPM. (1H) CORT + Veh mice had significantly less entries into the open arms of the EPM when compared with VEH + Veh mice. RS, but not Flx administration increased the number of entries into the open arms of the EPM in CORT-treated mice. (1I) All groups of mice traveled a similar distance in the EPM. (1J-1K) CORT administration increased the latency to feed in the NSF when compared with the VEH administration. RS, but not Flx administration decreased the latency to feed in CORT-treated mice. (1L) CORT administration decreased grooming duration in the ST when compared with VEH administration. RS, but not Flx administration increased the grooming duration in CORT-treated mice. (n = 9-14 male mice per group). Error bars represent ± SEM. *, p < 0.05; ** p < 0.01; ***, p < 0.001; ****, p < 0.0001. VEH, vehicle; Veh, vehicle; CORT, corticosterone; Flx, fluoxetine; RS, RS-67,333; EPM, elevated plus maze; NSF, novelty suppressed feeding; ST, splash test; sec, seconds; no., number; cm, centimeters; g, grams.

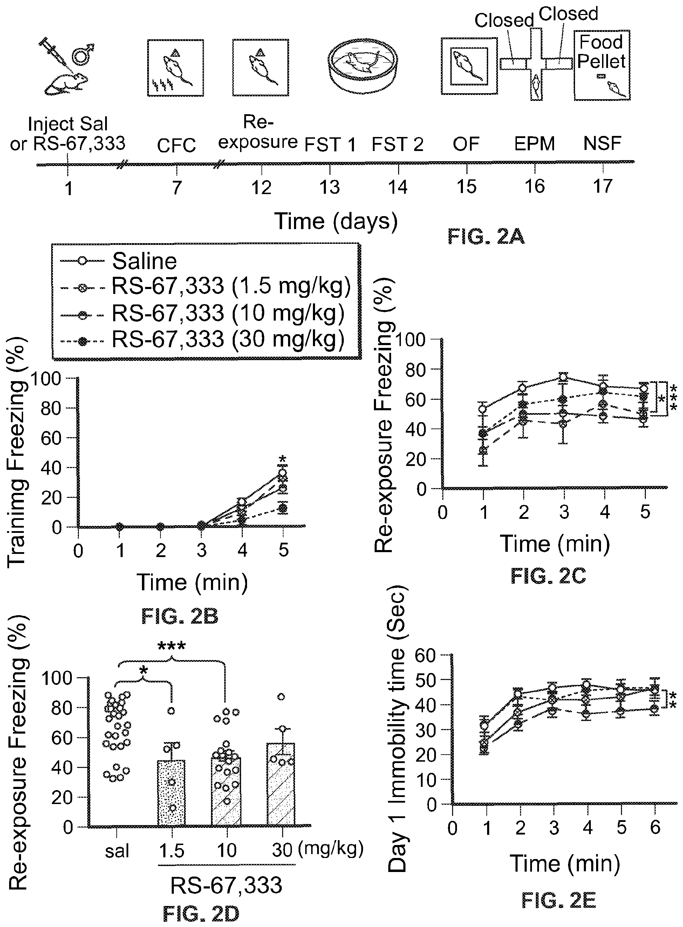

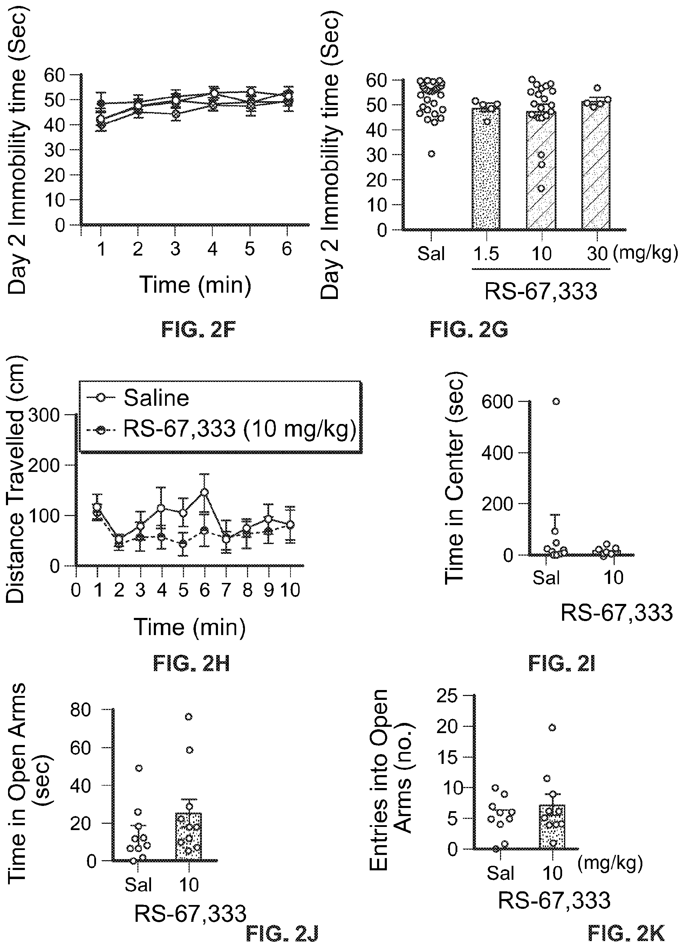

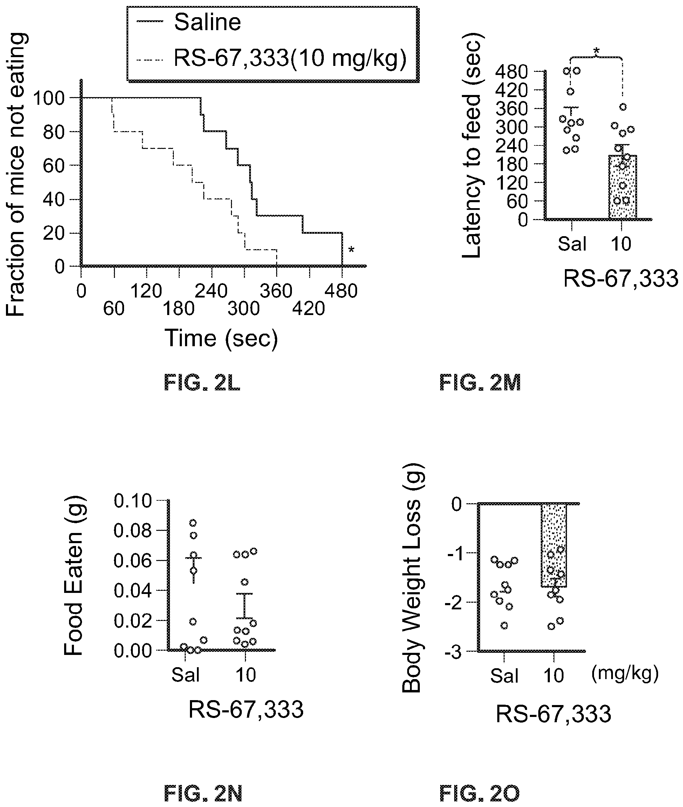

**Figures 2A-2O**. **A single, prophylactic injection of RS-67,333 attenuates learned fear and prevents novelty-induced hypophagia in male 129S6/SvEv mice.** (2A) Experimental design. (2B) Mice administered 30, but not 1.5 or 10 mg/kg of RS-67,333 exhibited significantly less freezing during CFC training when compared with mice administered saline. (2C-2D) Mice administered 1.5 or 10, but not 30 mg/kg of RS-67,333 at exhibited significantly less freezing when compared with mice administered saline. (2E) Mice administered 10, but not 1.5 or 30 mg/kg of RS-67,333, exhibited reduced immobility when compared with mice administered saline during day 1 of the FST. (2F-2G) All groups of mice had comparable amounts of immobility during day 2 of the FST. (2H-2I) RS-67,333 (10 mg/kg) did not alter distance travelled or time spent in the center of the OF when compared to saline mice. (2J-2K) Both groups of mice had comparable time spent in the open arms and entries into the open arms of the EPM. (2L-2M) Mice administered RS-67,333 (10 mg/kg) exhibited a significantly reduced latency to feed when compared to saline mice. (2N) Mice in both groups ate a comparable amount of food in the home cage following the NSF. (2O) Following food deprivation, mice in both groups lost a comparable amount of weight. (n = 5-29 male mice per group). Error bars represent ± SEM. *, p < 0.05; ***, p < 0.001. Sal, saline; CFC, contextual fear conditioning; FST, forced swim test; OF, open field; EPM, elevated plus maze; NSF, novelty suppressed feeding; min, minutes; sec, seconds; g, grams; mg, milligram; kg, kilogram; no., number; cm, centimeter.

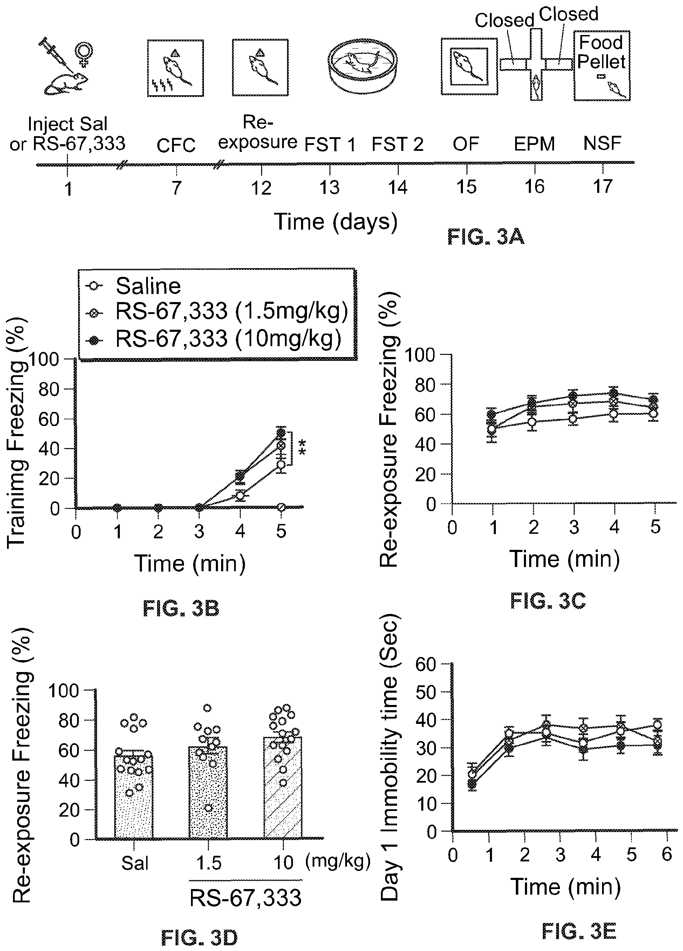

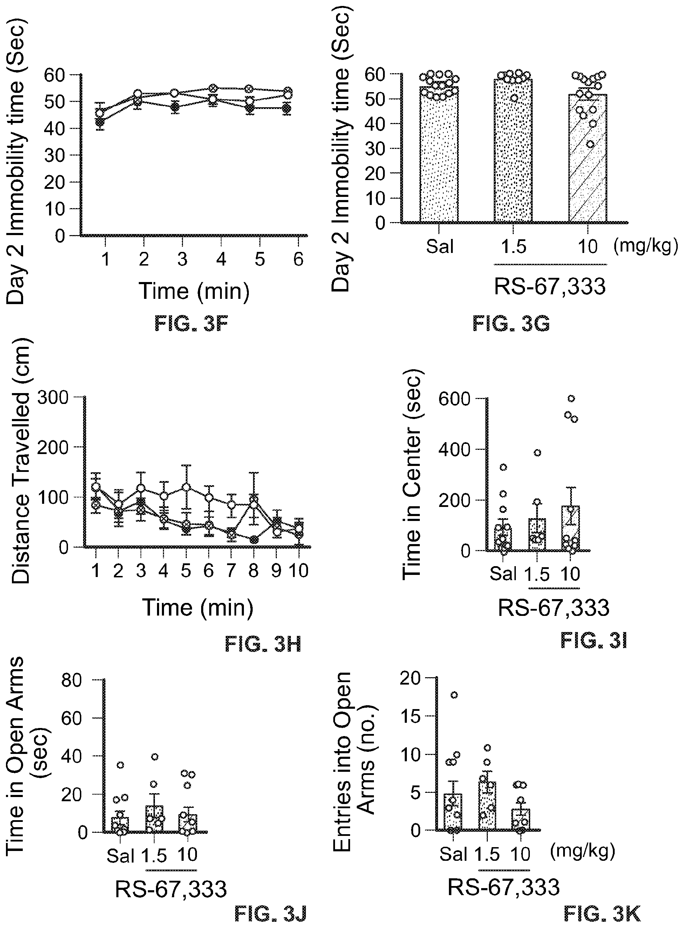

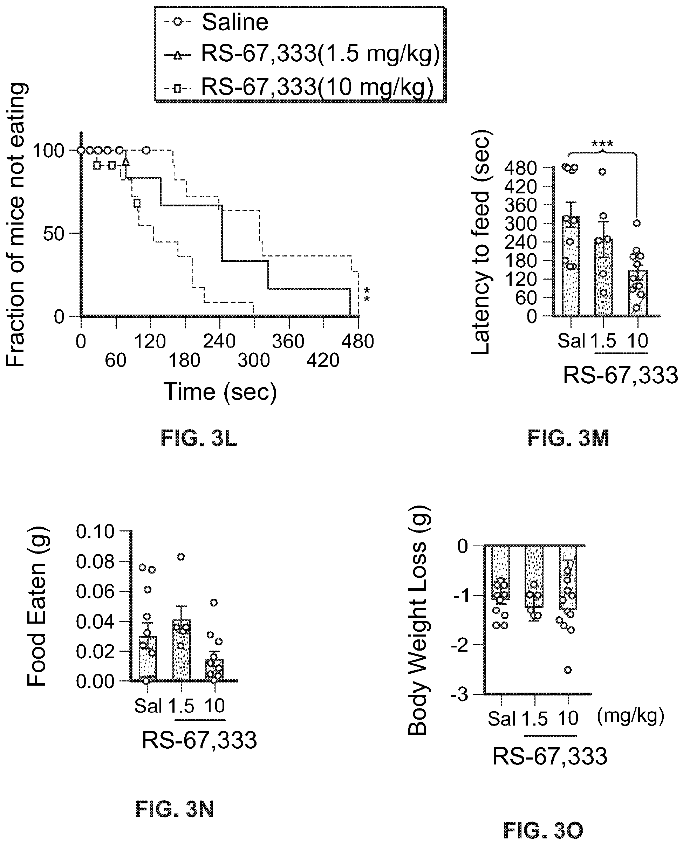

**Figures 3A-3O****. A single, prophylactic administration of RS-67,333 prevents novelty-induced hypophagia, but does not alter fear- or depressive-like behavior, in female 129S6/SvEv mice.** (3A) Experimental design. (3B) All mice exhibited comparable levels of freezing during CFC training. (3C-3D) All groups exhibited comparable levels of freezing during re-exposure. (3E) All groups of mice had comparable amounts of immobility during day 1 of the FST. (3F-3G) All groups of mice had comparable amounts of immobility during day 2 of the FST. (3H-3I) RS-67,333 did not alter distance travelled or time spent in the center of the OF. (3J) Time spent in the open arms of the EPM was comparable between all groups of mice. (3K) Entries into the open arms of the EPM was comparable between all groups of mice. (3L-3M) A single, prophylactic dose of RS-67,333 (10 mg/kg) significantly reduced latency to feed in the NSF. (3N) A single, prophylactic dose of RS-67,333 (10 mg/kg) did not alter the amount of food eaten in the home cage or (3O) body weight loss. (n = 6-11 female mice per group). Error bars represent ± SEM. *, p < 0.05; ***, p < 0.001. Sal, saline; CFC, contextual fear conditioning; FST, forced swim test; OF, open field; EPM, elevated plus maze; NSF, novelty suppressed feeding; min, minutes; sec, seconds; cm, centimeters; no., number; g, grams; mg, milligram; kg, kilogram.

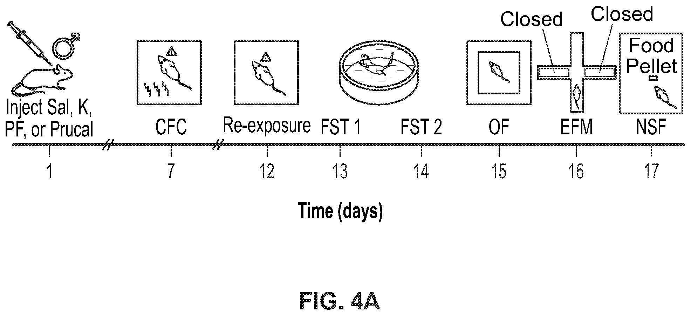

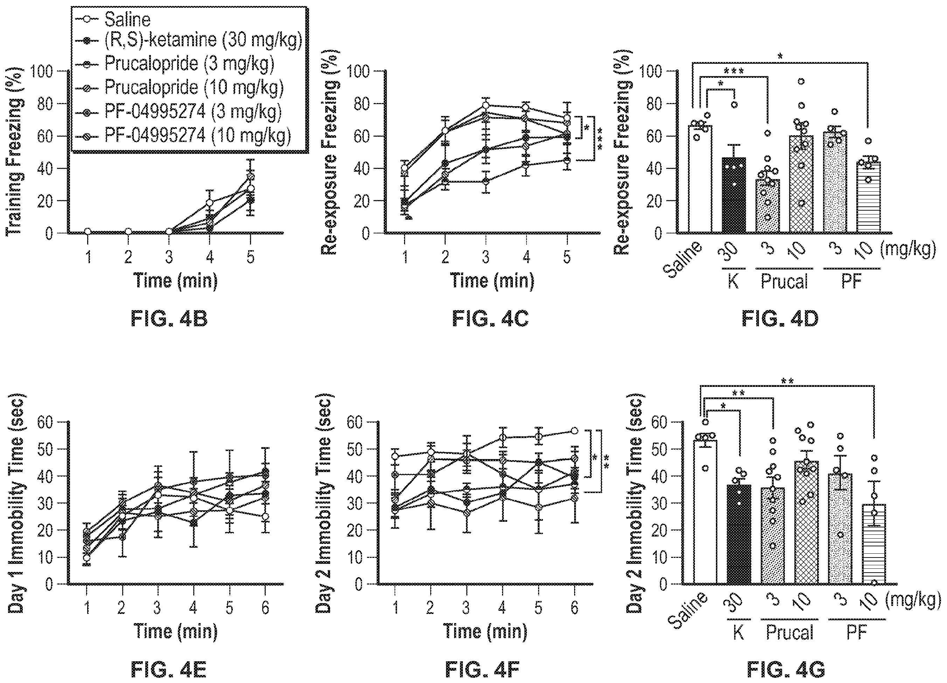

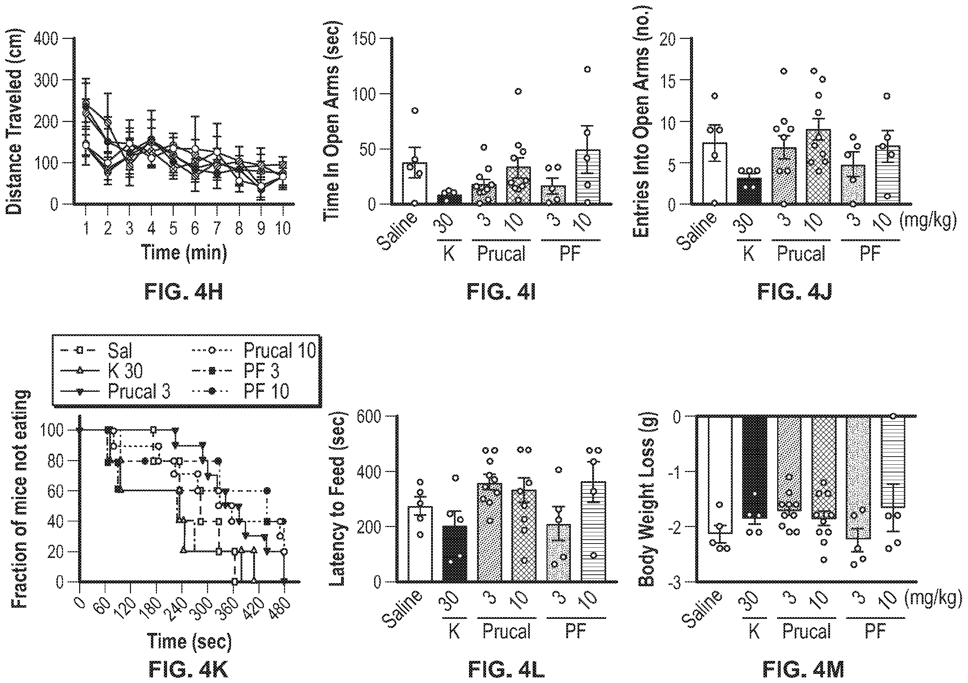

**Figures 4A-4M****. A single, prophylactic administration of prucalopride or PF-04995274 attenuates learned fear and decreases depressive-like behavior in male 129S6/SvEv mice.** (4A) Experimental design. (4B) All mice exhibited comparable levels of freezing during CFC training. (4C-4D) (*R*,*S*)-ketamine (30 mg/kg), prucalopride (3 mg/kg), and PF-04995274 (10 mg/kg), but not prucalopride (10 mg/kg) or PF-04995274 (3 mg/kg), administration attenuated learned fear when compared with saline administration. (4E) All groups of mice had comparable amounts of immobility during day 1 of the FST. (4F-4G) (*R*,*S*)-ketamine (30 mg/kg), prucalopride (3 mg/kg), and PF-04995274 (10 mg/kg), but not prucalopride (10 mg/kg) or PF-04995274 (3 mg/kg) significantly decreased immobility time during day 2 of the FST. (4H) All groups of mice traveled a comparable amount of distance in the OF. (4I) All groups of mice spent a comparable amount of time in the open arms of the EPM. (4J) All groups of mice had a comparable number of entries into the open arms of the EPM. (4K-4L) All groups of mice had a comparable latency to approach the pellet in the NSF. (4M) All groups of mice lost a comparable amount of weight following food deprivation for the NSF. (n = 5-10 male mice per group). Error bars represent ± SEM. *, p < 0.05; ** p < 0.01; ***, p < 0.001. Sal, saline; K, (*R,S*)-ketamine; Prucal, prucalopride; PF, PF-04995274; CFC, contextual fear conditioning; FST, forced swim test; OF, open field; EPM; elevated plus maze; NSF, novelty suppressed feeding; min, minutes; sec, seconds; cm, centimeters; no, number; mg, milligram; kg, kilogram.

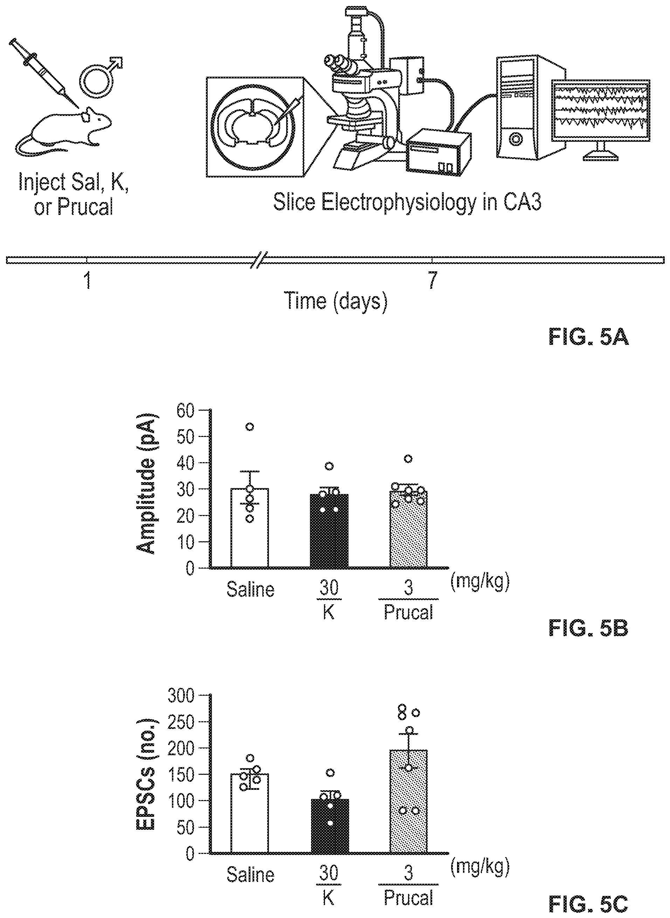

**Figures 5A-5F****. (*R*,*S*)-ketamine and prucalopride exhibit a common mechanism by reducing large AMPA-driven synaptic bursts in CA3.** (5A) Experimental design. (5B) The average EPSC amplitude did not differ between the groups. (5C) The average number of EPSCs (within a 20-second recording window) did not differ between the groups. (5D) Saline-treated mice typically displayed large bursts of EPSCs (-590.8±13.85 pA), which were blocked by the AMPA receptor antagonist NBQX. These large AMPA receptor-mediated signals were not present in either (5E) (*R,S*)-ketamine- or (5F) prucalopride-treated mice. (n = 5-7 mice per group). Error bars represent ± SEM. Sal, saline; K, (*R,S*)-ketamine; Prucal, prucalopride; CA3, Cornu Ammonis 3; pA, picoamps; EPSCs, excitatory postsynaptic currents; no., number; 00NBQX, 2,3-dihydroxy-6-nitro-7-sulfamoyl-benzo[f]quinoxaline; mg, milligram; kg, kilogram; ms, millisecond.

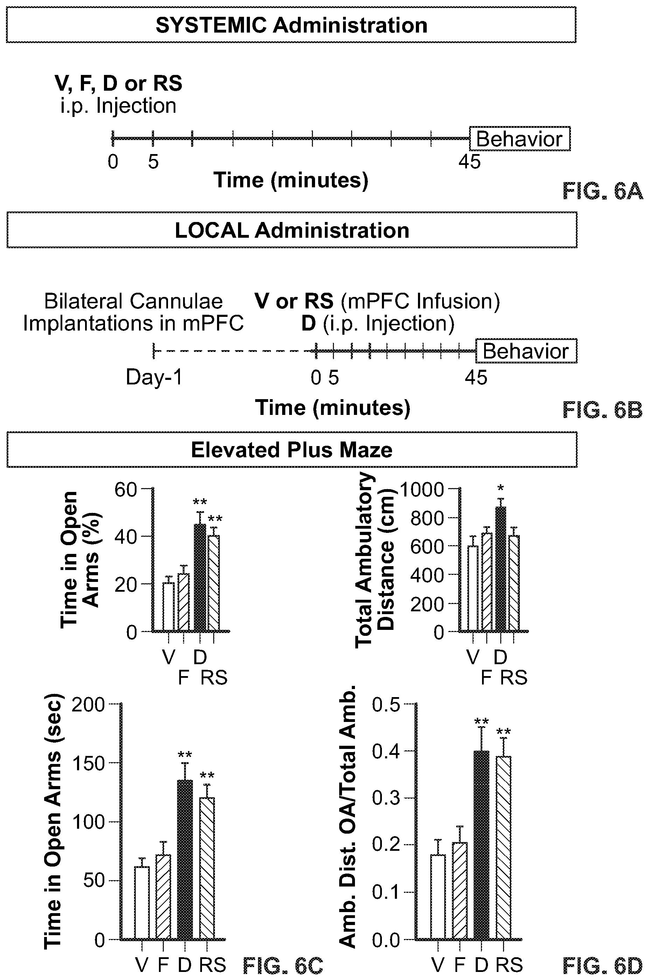

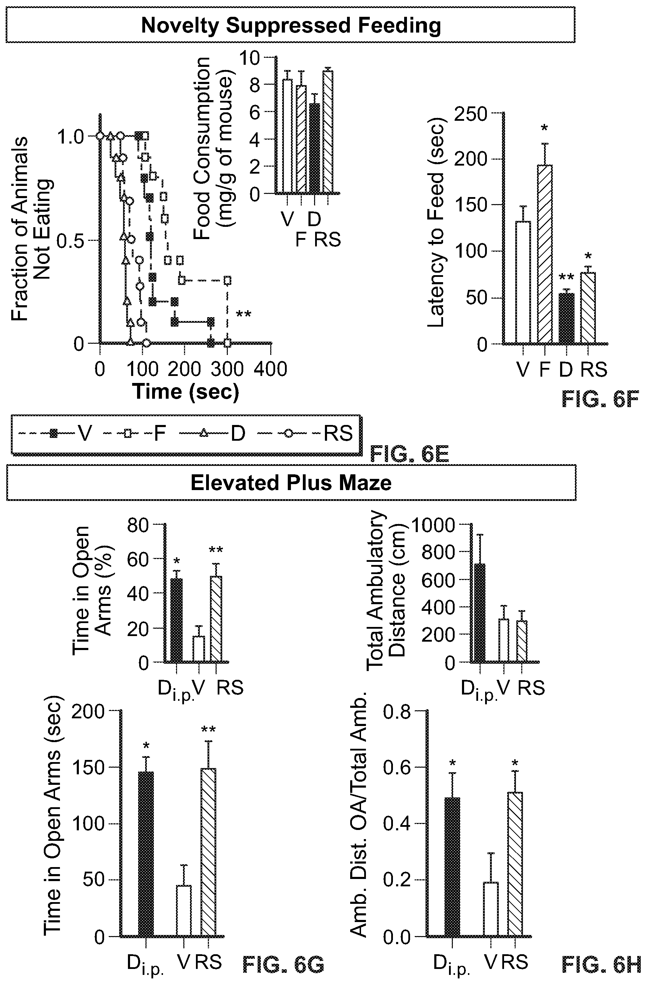

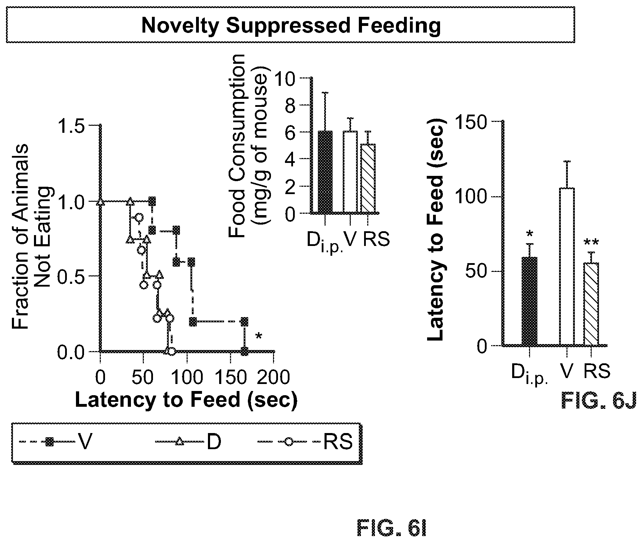

**Figures 6A-6J****. Acute 5-HT₄ receptor stimulation induces fast anxiolytic-like effects in an anxious BALB/cJRj mouse strain.** (6A, 6B) Experimental design. (6A) In a first cohort of animals, vehicle (V), fluoxetine (F, 18 mg/kg), diazepam (D, 1.5 mg/kg), or RS67333 (RS, 1.5 mg/kg) were administered via intraperitoneal (i.p.) injection 45 minutes before behavioral testing. (6B) In a second cohort of animals, treatments (V or RS, 0.5 µg/side) were infused in medial prefrontal cortex (mPFC) and diazepam (D, 1.5 mg/kg) administered via i.p. injection 45 minutes before the start of behavioral paradigms. (6C-6D and 6G-6H) Anxiolytic-like effect was measured in the elevated plus maze (EPM) as mean time or percent time spent in open arms (6C, 6G and inset), or as mean ratio ambulatory distance in open arms/total distance (6D, 6H) and as mean total ambulatory distance (inset) (n = 10 mice per group and n = 5-9 mice per group for systemic and local injection study respectively). (6E-6F and 6I-6J) In the Novelty Suppressed Feeding (NSF) paradigm, anxiolytic-like effect is expressed as fraction of animals that have not eaten over 10 minutes (6E, 6I), as mean of the food consumption (inset) or as mean of latency to feed (6F, 6J) (n = 10 mice per group, n = 4-8 mice per group). (6C-6D and 6E-6F) Systemic administration. (6G-6H and 6I-6J) local administration. Values plotted are mean ± SEM. *p<0.05, **p<0.01 *vs.* vehicle group.

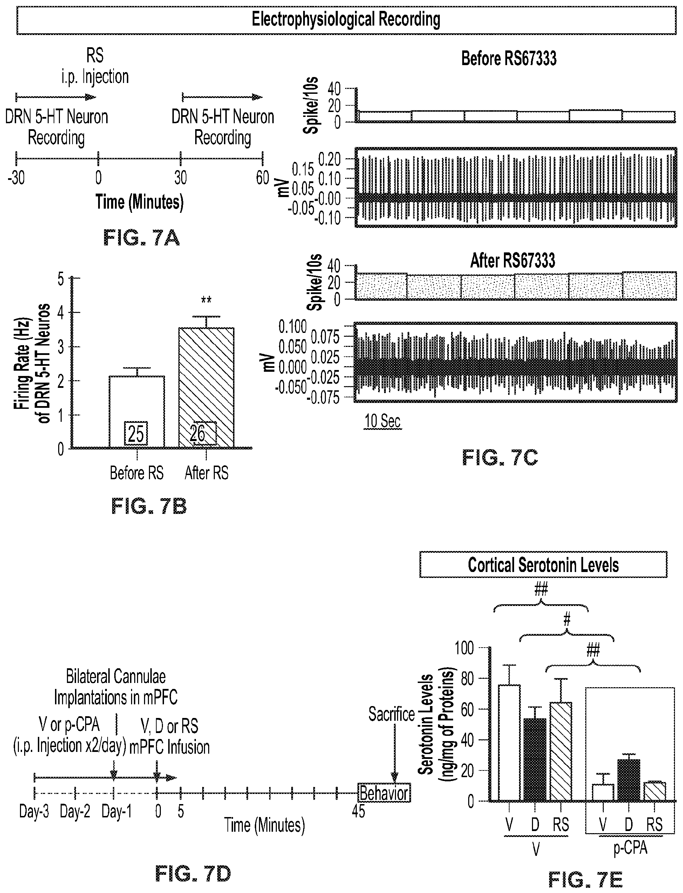

**Figures 7A-7G****. Acute 5-HT₄ receptor stimulation induces fast anxiolytic-like effects on 5-HT function through a modulation of the mPFC in an anxious BALB/cJRj mouse strain.** (7A) Before the administration of RS67333 (RS, 1.5 mg/kg), different tracks were performed to record 5-HT neurons for 30 min. At the end of this period, RS67333 was administered i.p. and 30 min after, two or three subsequent tracks were realized. (7B) Discharge frequency of DRN 5-HT neurons is assessed as mean firing rate. The number of neurons tested is indicated in each histogram (n = 25 and 26 before and after RS injection for a total of n = 5 mice). Data are mean ± frequency (Hz) of DRN 5-HT neurons determined before the administration of RS67333. (7C) Typical recordings of DRN 5-HT neurons in the different experimental conditions. Histograms in the upper panels represent the number of action potentials (APs) per 10 seconds (scale bar). Lower panels represent the well-characterized regular discharge of serotonergic neurons in both conditions. **p<0.01 *vs.* before RS67333 administration. (7D) p-CPA was injected i.p. twice a day during 3 days and treatments [vehicle (V), diazepam (D, 1.5 µg/side) and RS (0.5 µg/side)] were infused 24 hours after the final p-CPA administration in the medial prefrontal cortex (mPFC) and 45 minutes before the start of behavioral paradigms. (7E) Cortical 5-HT depletion by p-CPA pre-treatment is measured as mean 5-HT levels (n = 6-8 mice per group). (7F and 7G) Anxiety is measured in the elevated plus maze (EPM) as mean time or percent time spent in the open arms (F and inset), as mean ratio ambulatory distance in open arms/total distance (7G) and as mean total ambulatory distance (inset) (n = 8-11 mice per group). Values plotted are mean ± SEM. *p<0.05, **p<0.01 *vs.* vehicle group, #p<0.05, ##p<0.01 vs. appropriate group.

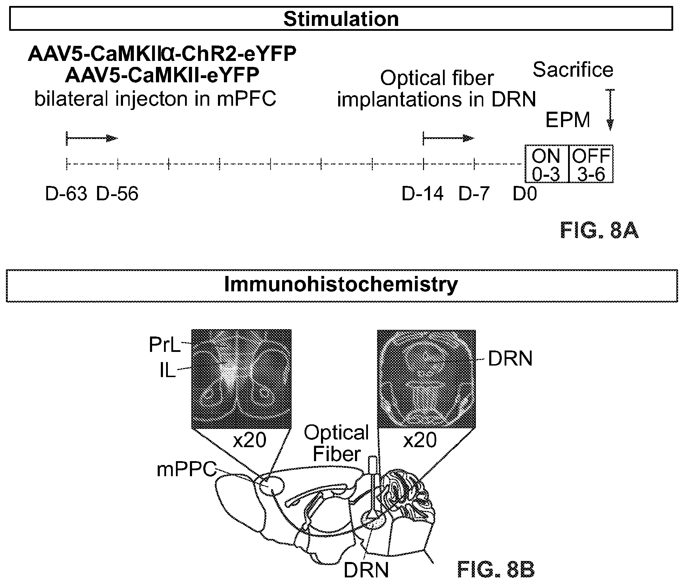

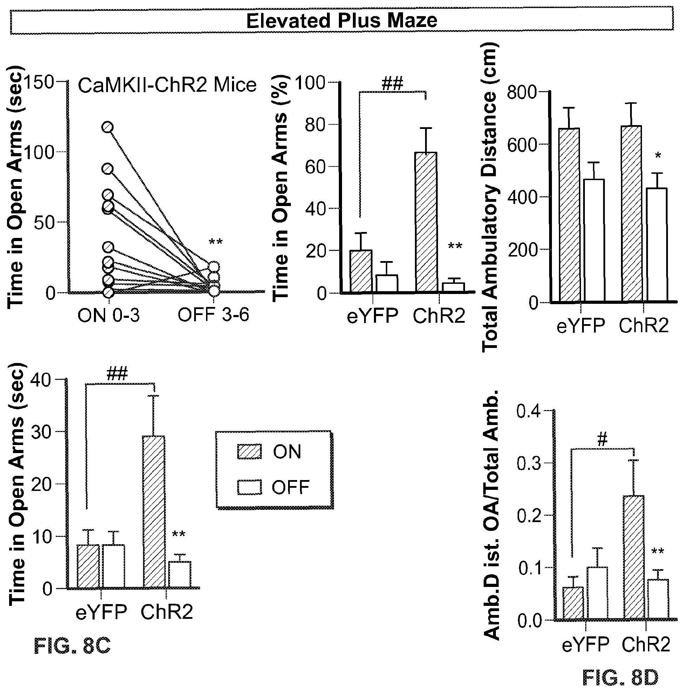

**Figures 8A-8D****. Effects of cortical terminals stimulation in the dorsal raphe nucleus of anxious BALB/cJRj mouse strain.** (8A) Timeline regarding the behavioral consequences after stimulation of glutamatergic terminals in the DRN. AAV5-CamKIIα-ChR2-eYFP or AAV5-CamKII-eYFP virus were bilaterally injected in the medial prefrontal cortex (mPFC) and an optic fiber was implanted in the dorsal raphe nucleus (DRN), respectively 7 weeks and 1 week before testing in the Elevated Plus Maze (EPM). (8B) Expression of virus was confirmed in the mPFC (left) and in the DRN (right). (8C-8D) After optogenetic stimulation (3-min ON and 3-min OFF), anxiolytic-like effect is measured in the EPM as time or percent time spent in the open arms between laser ON and laser OFF (8C and inset), the distribution of time spent in open arms during and following cortical terminals stimulation in the dorsal raphe nucleus (inset), as mean ratio of ambulatory distance in the open arms divided by total distance (8D) and as mean total ambulatory distance (inset) or (eYFP: n = 12 mice per group; ChR2: n = 19 mice per group). Values plotted are mean ± SEM. *p<0.05, **p<0.01 between laser ON and laser OFF; #p<0.05, ##p<0.01 between CHR2 and eYFP group.

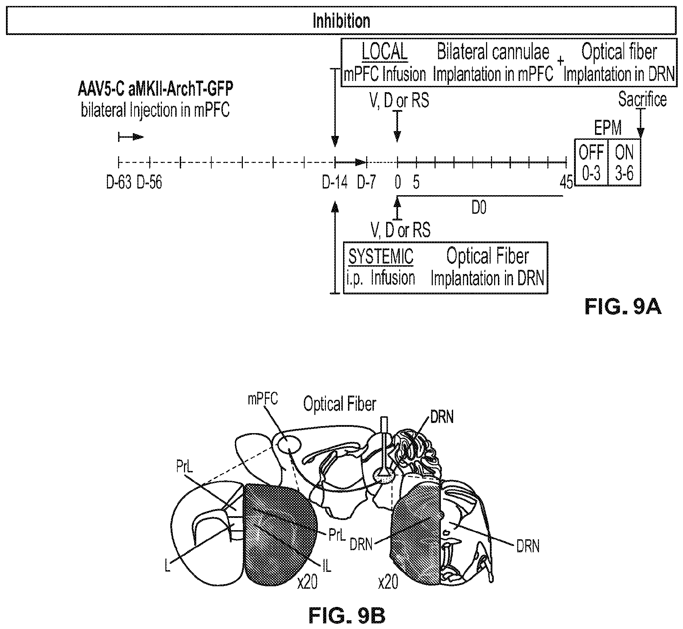

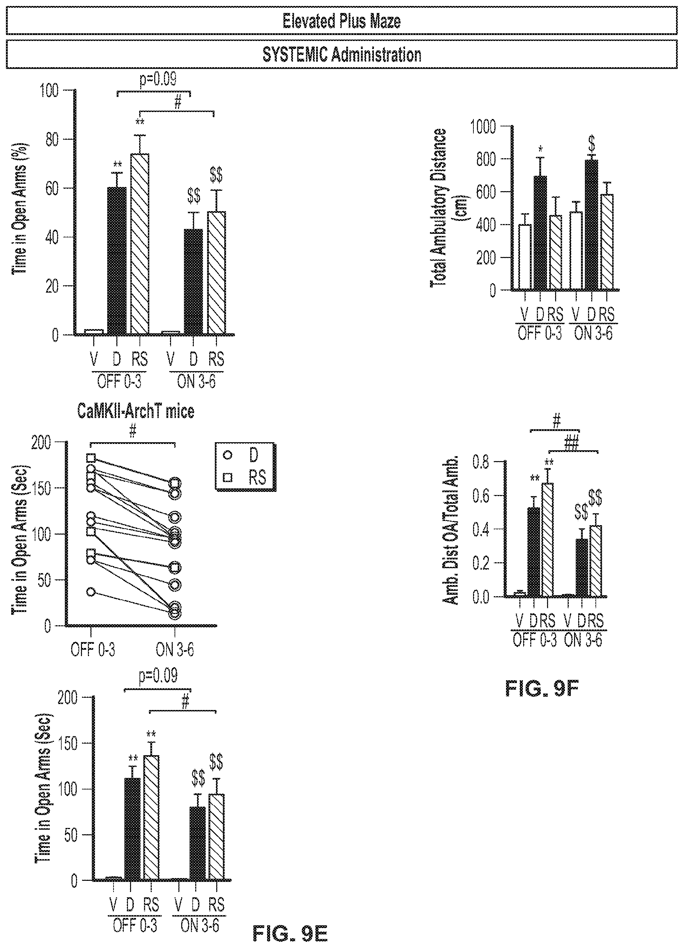

**Figures 9A-9F****. Modulation of anxiolytic-like activity after optogenetic inhibition of glutamatergic terminals in the dorsal raphe nucleus (DRN) of the anxious BALB/cJRj mouse strain.** (9A) Timeline regarding the behavioral consequences after inhibition of glutamatergic terminals in the DRN after medial prefrontal cortex (mPFC) infusion (diazepam [D] [1.5 mg/side] or RS67333 [RS] [0.5 mg/side]) or systemic administration of diazepam (1.5 mg/kg intraperitoneally [i.p.]), RS67333 (1.5 mg/kg i.p.) or vehicle (V). AAV5-CamKII-ArchT-GFP virus was injected bilaterally in the mPFC 7 weeks before testing. An optic fiber was implanted in the DRN 1 week before testing. For the local injection protocol, 2 injection cannulae were also implanted in the mPFC. Drug treatments were infused in the mPFC or injected i.p. 45 minutes before testing. (9B) Expression of control CaMKII-ArchT-GFP virus was confirmed in the mPFC (left) and in the DRN (right). (9C-9F) For the behavioral consequences of a local infusion (9C, 9D) or systemic administration (9E, 9F) with vehicle, diazepam, or RS, the anxiolytic-like effect is measured in the elevated plus maze (EPM) as time (9C, 9E) or percent time (insets in panels 9C and 9E) spent in the open arms across 2-period stimulation (3-minute light/dark cycle), as the distribution of time spent in open arms before and after cortical terminals inhibition in the DRN (insets in panels 9C and 9E), as mean time in open arms (OA) divided by total time (insets in panels 9C and 9E), as mean total ambulatory distance (insets in panels 9D and 9F), or as mean ratio of ambulatory distance in the OA divided by total distance (9D, 9F) (n = 7-11 mice per group and n = 7-9 mice per group for local and systemic administration, respectively). Values plotted are mean ± SEM. *p < .05 and **p < .01 vs. vehicle group; #p < .05 and ##p < .01 vs. the appropriate group during lights off. ^{$}p < .05 and ^{$$}p < .01 vs. vehicle group during lights on.
**Figure 10****. The neuronal circuits involved in fast anxiolytic-like effects induced by acute systemic RS67333 and diazepam administration.** Acute systemic administration with RS67333, a serotonin type 4 receptor (5-HT4R) agonist, induces fast anxiolytic-like effects in BALB/cJRj mice through at least activation of the cortical glutamatergic terminals in the dorsal raphe nucleus (DRN) confirming previous studies (19). Similarly, diazepam, a benzodiazepine (BZD), induces fast anxiolytic-like effects through a similar neuronal circuit recruitment. Our data also demonstrated that other brain structures might be involved in the fast anxiolytic-like activity of a 5-HT₄R agonist. Previous studies demonstrated that the hippocampus (HPC)/medial prefontal cortex (mPFC) (46) circuit and the mPFC/amygdala (Amy) pathway (48) are recruited to modulate anxiety-like phenotypes, suggesting that these circuits should be also investigated for fast anxiolytic-like effects induced by 5-HT4R. GABA, gamma-aminobutyric acid.
**Figures 11A-11E****. Acute 5-HT₄R antagonist administration prevents RS67333-induced fast anxiolytic-like effects.** (11A) Treatments (Fluoxetine 18 mg/kg, F; Diazepam 1.5 mg/kg, D; RS67333 1.5 mg/kg, RS; Vehicle, V) were administered i.p. 45 minutes before behavioral testing, except for GR125487 (GR, 1 mg/kg, i.p.) administered 15 minutes before RS67333 administration. (11B and 11C) After treatment administration, anxiety is measured in the EPM as mean time or percent time spent in the open arms (11B and inset), as mean ratio of ambulatory distance in the open arms divided by total distance or as mean ambulatory distance (11C and inset) (n = 5-6 mice per group). (11D and 11E) In the NSF, anxiety parameter is expressed as fraction of animals that have not eaten over 10 minutes (11D), as mean of the food consumption (inset) or mean of latency to feed (E) (n = 4-6 mice per group). All statistical tests and p values are mean ± SEM. *p<0.05, **p<0.01 *vs.* vehicle group (V), ##p<0.01 *vs.* RS group.
**Figures 12A-12C****. Acute systemic 5-HT₄R stimulation induces fast anxiolytic-like effects in the Open Field Paradigm.** (12A) Experimental design. Vehicle (V), fluoxetine (F, 18 mg/kg), diazepam (D, 1.5 mg/kg), or RS67333 (RS, 1.5 mg/kg) were administered i.p. 45 minutes before behavioral testing in BALB/cJRj mouse strain. (12B-12C) Anxiolytic-like effect was measured in the OF as mean percent time spent in center (12B), as mean ratio ambulatory distance in center/total distance (12C) and as mean total ambulatory distance (inset) (n = 5-9 mice per group for systemic). All statistical tests and p values are mean ± SEM. *p<0.05, **p<0.01 *vs.* vehicle group (V).
**Figures 13A-13C****. Optogenetic stimulation of mPFC terminals in the DRN induces fast anxiolytic-like effects in the Open Field Paradigm.** (13A) Timeline regarding the behavioral consequences after stimulation of cortical glutamatergic terminals in the DRN. AAV5-CamKIIα-ChR2-eYFP or AAV5-CamKII-eYFP virus were bilaterally injected in the medial prefrontal cortex (mPFC) and an optic fiber was implanted in the dorsal raphe nucleus (DRN), respectively 7 weeks and 1 week before testing in the OF. (13B-13C) Anxiolytic-like effect is measured in the OF as percent time spent in the center between laser ON and laser OFF (inset: time in seconds) (13B), as mean ratio of ambulatory distance in the center divided by total distance (inset: total ambulatory distance) (13C) (eYFP: n = 8 mice per group; ChR2: n = 13 mice per group). Values plotted are means ± SEM. **p<0.01 between laser ON and laser OFF; ##p<0.01 between CHR2 and eYFP group during laser ON.
**Figures 14A-14C****. Optogenetic inhibition of mPFC terminals in the DRN blocks anxiolytic activity of mPFC infusion with RS67333 and Diazepam.** (14A) Timeline regarding the behavioral consequences of inhibition of medial prefrontal cortex (mPFC) terminals in the dorsal raphe nucleus (DRN) after mPFC infusion of diazepam (D, 1.5 µg/side), RS672333 (RS, 0.5 µg/side) or vehicle (V). AAV5-CamKII-ArchT-GFP virus was bilaterally injected in the mPFC 7 weeks before testing. An optic fiber was implanted in the DRN, 1 week before testing. (14B-14C) For the behavioral consequences of a local infusion with V, D or RS, anxiolytic-like effect is measured in the open field (OF) as percent time spent in the center across 2 epoch stimulation (3-min OFF and 3-min ON) (14B), the distribution of time spent in center prior to and following inhibition of glutamatergic axon terminals arising from the mPFC to the dorsal raphe nucleus (inset), as mean ratio of ambulatory distance in the center divided by total distance (14C) and as mean total ambulatory distance (inset) (n = 6-8 mice per group). Values plotted are mean ± SEM. *p<0.05, **p<0.01 *vs.* vehicle group, #p<0.05, ##p<0.01 *vs.* appropriate group during light ON.
**Figures 15A-15I****. Single stimulation of the 5-HT4 receptor could lead to long-lasting anxiolytic and antidepressant effects in the BALB/cJRj mice.** (15A) The BALB/cJRj mice were systemically injected with a single dose of RS67333 (1.5 mg/kg) or diazepam (1.5 mg/kg), 45 minutes before performing the Splash Test. The following day, the mice underwent the EPM without having received another dose of RS67333, and then at the open field 24 hours later, and, finally, to NSF 24 hours after the open field. (15B-15C) RS67333 increased the grooming time (t = 2.294; p<0.05) in the Splash Test, without affecting the number of episodes (t = 1.546; p = 0.1531), following single administration. (15D-15E) We have not identified the anxiolytic effects expected of RS67333 in the EPM (t = 0.4990; p = 0.6286), 24 hours after the injection. However, the RS67333 has increased the time spent in the center of the OF (t = 1.924; p<0.05), without affecting the ratio of the distance in the center to the total walking distance (t = 1.281; p = 2292) (15F-15G), 48 hours after the injection, and reduced the lag for feeding in the NSF (t = 2.520; p<0.05), without affecting the consumption of food in a familiar environment (t = 0.2203; p = 0.4151) (15H-15I), 72 hours after the injection. (15B-15C) Splash test (acute effects). (15D-15E) Elevated plus maze (long-lasting effects). (15F-15G) Open field (long-lasting effects). (15H-15I) Novelty suppressed feeding (long-lasting effects). Abbreviations: *p<0.05 versus carrier; D: diazepam 1.5 mg/kg; i.p.: intraperitoneal injection RS: RS 67333 1.5 mg/kg; V: carrier.

### DETAILED DESCRIPTION OF THE INVENTION

The present disclosure provides pharmaceutical compositions for prophylactically treating a stress-induced affective disorder or stress-induced psychopathology in a subject. The invention is set out in the appended claims. Specifically, the invention relates to a pharmaceutical composition comprising 4-amino-5-chloro-2,3-dihydro-N-[1-3-methoxypropyl)-4-piperidinyl]-7-benzofuran carboxamide monohydrochloride (prucalopride), 4-[4-[4-Tetrahydrofuran-3-yloxy)-benzo[d]isoxazol-3-yloxymethyl]-piperidin-1-ylmethyl]-tetrahydropyran-4-ol (PF-04995274), or combinations thereof, for use in a method for preventing a stress-induced affective disorder or stress-induced psychopathology selected from stress-induced fear and depressive-like behavior and associated affective disorders in a subject prior to a stressor, wherein the pharmaceutical composition is administered to the subject 48 hours to 3 weeks prior to the stressor.

Administration of an effective amount of the pharmaceutical composition prevents the stress-related disorders from developing or being exacerbated into more serious conditions.

In certain embodiments, the pharmaceutical composition is administered prior to recurrence of the stressor. In certain embodiments, the pharmaceutical composition is administered prior to the onset of a particular symptom.

The benefit to a subject to be treated is either statistically significant or at least perceptible to the patient or to the physician.

A "prophylactically effective amount" refers to an amount effective, at dosages and for periods of time necessary, to achieve the desired prophylactic result.

The pharmaceutical composition may be administered by any method known in the art, including, without limitation, intranasal, oral, transdermal, ocular, intraperitoneal, inhalation, intravenous, intracerebroventricular (ICV), intracisternal injection or infusion, subcutaneous, implant, vaginal, sublingual, urethral (e.g., urethral suppository), subcutaneous, intramuscular, intravenous, rectal, sub-lingual, mucosal, ophthalmic, spinal, intrathecal, intra-articular, intra-arterial, sub-arachinoid, bronchial and lymphatic administration. Topical formulation may be in the form of gel, ointment, cream, aerosol, etc.; intranasal formulation can be delivered as a spray or in a drop; transdermal formulation may be administered via a transdermal patch or iontorphoresis; inhalation formulation can be delivered using a nebulizer or similar device. Compositions can also take the form of tablets, pills, capsules, semisolids, powders, sustained release formulations, solutions, suspensions, elixirs, aerosols, or any other appropriate compositions.

In certain embodiments, a subject is treated with the pharmaceutical composition, via intravenous, oral, transdermal or intranasal administration. In certain embodiments, a subject is injected with the pharmaceutical composition.

In certain embodiments, a subject is treated with a single dose of an effective amount of the pharmaceutical composition. In some aspects, a subject is treated with multiple doses of an effective amount of the pharmaceutical composition.

Provided herein is a pharmaceutical composition that comprises prucalopride and/or PF-04995274), or pharmaceutically acceptable salts or solvates thereof, and a pharmaceutically acceptable carrier, excipient or diluent, for use in the prophylactic treatment of the afore-mentioned stress-induced affective disorders.

"Patient" or "subject" refers to mammals and includes human and veterinary subjects. In certain embodiments, the subject is mammalian.

Prucalopride and/or PF-04995274 may activate 5-HT₄R through any mechanism, including, but not limited to, activating/increasing 5-HT₄R activity, activating/increasing 5-HT₄R level, and/or activating/increasing 5-HT₄R gene expression. The terms "activator of 5-HT₄R", "activator of the 5-HT4 receptor", "5-HT4 receptor activator", and "5-HT₄R activator" are used interchangeably herein.

By "activation", "up-regulation" or "increase" is meant any positive effect on the condition being studied; this may be total or partial. Thus, where the level or activity of a protein (e.g., 5-HT4 receptor or 5-HT₄R) is being detected, the pharmaceutical composition is capable of activating, up-regulating, or increasing the level or activity of the protein (e.g., 5-HT4 receptor or 5-HT₄R). The activation or up-regulation of the level or activity of the protein achieved by prucalopride or PF-04995274may be at least 10%, such as at least 20%, at least 30%, at least 40%, at least 50%, at least 60%, at least 70%, at least 80%, at least 90% or more compared to the level or activity of the protein (e.g., 5-HT4 receptor or 5-HT₄R) in the absence of pharmaceutical composition.

Half maximal effective concentration (EC50) refers to the concentration of an agent which induces a response halfway between the baseline and maximum after a specified exposure time. The pEC50 is defined as the negative logarithm of the EC50:$pEC 50 = - {log}_{10} \left(EC50\right) .$

Prucalopride or PF-04995274 may have a pEC50 in activating the 5-HT4 receptor activity ranging from about 3 to about 13, from about 4 to about 12, from about 5 to about 11, from about 6 to about 10, from about 6 to about 9, from about 6 to about 8, from about 6 to about 7, from about 7 to about 10, from about 7 to about 9, from about 7 to about 8, from about 8 to about 10, from about 8 to about 9, from about 9 to about 10, from about 5 to about 10, about 6, about 6.5, about 7, about 7.5, about 8, about 8.5, about 9, about 9.5, or about 10.

Ki denotes the affinity of an agent (e.g., an activator such as an agonist) for a receptor. When measured using a radioligand competition binding assay, it is the molar concentration of the competing ligand that would occupy 50% of the receptors if no radioligand was present. The pKi is the negative logarithm of the Ki value.

Prucalopride or PF-04995274may have a pKi for the 5-HT4 receptor ranging from about 3 to about 13, from about 4 to about 12, from about 5 to about 11, from about 6 to about 10, from about 6 to about 9, from about 6 to about 8, from about 6 to about 7, from about 7 to about 10, from about 7 to about 9, from about 7 to about 8, from about 8 to about 10, from about 8 to about 9, from about 9 to about 10, from about 5 to about 10, about 6, about 6.5, about 7, about 7.5, about 8, about 8.5, about 9, about 9.5, or about 10.

### 5-HT₄R Agonists

The 5-HT₄R is a G-protein coupled receptor (GPCR) that activates G protein Gs and stimulates the cAMP/PKA signaling pathway, resulting in the phosphorylation of cAMP response element binding protein (CREB) and as a consequence the expression of a number of genes involved in neuroplasticity (A10). The majority of 5-HT₄Rs are expressed in the brain of primates and rodents specifically in the medium spiny neurons of the striatum, the ammon's horns (CA1 and CA3) of the hippocampus, the granule cells of the dentate gyrus and glutamatergic neurons in the cortex and amygdala (A11). In addition, 5-HT₄Rs are also found in hypothalamus, ventral pallidum, olfactory bulbs, septal area, and substantia nigra. Mice lacking the 5-HT₄R display anhedonia and a context-dependent anxiety-like behavior (A12) and various 5-HT₄R agonists can exert an antidepressant and anxiolytic-like activity (A6).

Whether in humans or in rodents, the expression of the serotoninergic type 4 receptor (5-HT₄) is found in the limbic regions (ₘPFC, HPC and NAc). In addition, the basal ganglia, i.e., the caudate nucleus and the lenticular nucleus (putamen and pallidum), the black matter, and the amygdala, also express the 5-HT₄ receptor. The 5-HT₄ receptor is expressed at the somatodendritic level and at the level of the axon terminals of efferent spinal GABAergic neurons of the striatum, the Amon horns (CA1 and CA3) of the hippocampus, the granular cells of the dentate gyrus, and glutamatergic neurons of the cortex, the hippocampus and the amygdala.

5-HT₄ receptor is also found at the peripheral level, in particular at the cardiac level, where activation thereof exerts a positive inotropic effect, at the level of the gastro-intestinal tract where it is involved in intestinal motility, at the level of the adrenal glands where it plays a role in secretion of corticosterone, and at the level of the bladder where it causes contraction of the smooth muscles.

The 5-HT₄ receptor is a receptor having seven transmembrane domains. The N-terminal region faces towards the extracellular environment, whereas the C-terminal domain, coupled to a Gs protein, faces towards the cytoplasm. The activation of the 5-HT₄ receptor, e.g., by an agonist, can lead to the recruitment of the Gs protein which stimulates adenylate cyclase (AC) which is responsible for the production of cAMP. Protein kinase A (PKA), activated by the cAMP, modulates different ionic currents and in particular potassium currents, the inhibition of which results in neuronal hyperexcitability. The PKA is also capable of phosphorylating the protein binding the response element to the cAMP (CREB - cAMP response element binding protein), which results in an increase in the transcription of neurotrophic brain factor (BDNF, brain-derived neurotrophic factor), involved in cognition, mood and cell survival.

The term "agonist" may refer to a substance, an agent or a compound capable of binding to and activating one or more receptors, such as 5-HT₄R. The term "agonist" may refer to a compound having the ability to initiate or enhance a biological function of a target protein (e.g., one or more receptors, such as 5-HT₄R), whether by enhancing or initiating the activity or expression of the target protein. 5-HT₄R agonists may be compounds that activate the action of the 5-HT4 receptor. The term "agonist" may be defined in the context of the biological role of the target protein. An agonist may be an agent that binds to a receptor (e.g., 5-HT₄R) and activates the receptor to produce a biological response. While agonists provided herein can specifically interact with (e.g., bind to) the target protein, compounds that initiate or enhance a biological activity of the target protein by interacting with other members of the signal transduction pathway of which the target protein is a member are also specifically included within this definition. A 5-HT₄R agonist may be a compound or an agent that activates the action of 5-HT₄R. A 5-HT₄R agonist may be any agent that acts directly or indirectly through or upon 5-HT₄R to produce a pharmacological effect. The terms "agonist of 5-HT₄R", "agonist of the 5-HT4 receptor", "5-HT4 receptor agonist", and "5-HT₄R agonist" are used interchangeably herein.

The 5-HT₄R agonist may be selective for 5-HT4 receptors or it may be non-selective, exhibiting agonist or antagonist activity at other serotonin receptors. The 5-HT₄R agonist may be selective for 5-HT4 receptors.

The 5-HT₄R agonists may include full agonists, partial agonists, mixed 5-HT₄R agonists/antagonists, etc.

"Full agonists" may refer to agents bind to and activate a receptor with the maximum response that an agonist can elicit at the receptor. An agent may act as a full agonist in some tissues and as a partial agonist in other tissues, depending upon the relative numbers of receptors and differences in receptor coupling.

"Partial agonists" may refer to compounds able to bind and activate a given receptor, but having only partial efficacy at the receptor relative to a "full agonist" or complete agonist. Partial agonists can act as antagonists when competing with a full agonist for receptor occupancy and producing a net decrease in the receptor activation compared to the effects or activation observed with the full agonist alone. Partial agonists may refer to mixed agonists/antagonists, which differentially affect a receptor function within different dose ranges. For example, partial agonists may serve as agonists at lower doses, and as antagonists at higher doses. Partial agonists may be compounds that have reduced efficacy for inducing conformational change in receptors (typically 40-80%) relative to full agonists, and which may induce agonist effects at low dose but antagonist effects at high dose.

Prucalopride is a selective, high affinity 5-HT₄R agonist [24]. Prucalopride is a derivative of the family of benzofurans which exhibits increased selectivity for 5-HT₄ receptor but no affinity for the hERG (human Ether-a-go-go Related Gene) channels. In 2018, it was approved by the FDA for chronic constipation and is currently being tested for chronic intestinal pseudo-obstruction. Prucalopride has also been tested in two separate clinical trials to investigate its effects on emotional processing in health volunteers after an acute (e.g., single dose) or chronic (e.g., 1 week) administration [25,26].

PF-04995274 is a potent, partial 5-HT₄R agonist [27]. A clinical trial was conducted to evaluate PF-04995274, alone or in combination with donepezil, on scopolamine-induced deficits in psychomotor and cognitive function in healthy adults; however, this trial was terminated, but not due to safety concerns [28]. Currently, a clinical trial is underway to test whether adjunctive administration of PF-04995247 has positive effects on emotional processing and neural activity in mediated, treatment-resistant (TRD) depressed patients compared to placebo [29].

The terms "serotonin," "5-hydroxytryptamine" and "5-HT" refers to a phenolic amine neurotransmitter produced from tryptophan by hydroxylation and decarboxylation in serotonergic neurons of the central nervous system and enterochromaffin cells of the gastrointestinal tract. Serotonin is a precursor of melatonin.

The term "pharmaceutically acceptable derivative" refers to any pharmaceutically acceptable salt, solvate, prodrug, e.g. ester, or other precursors, of a compound which upon administration to the recipient is capable of providing (directly or indirectly) the active compound or an active metabolite or residue thereof. Such salts include pharmaceutically acceptable basic or acid addition salts as well as pharmaceutically acceptable metal salts, ammonium salts and alkylated ammonium salts. Such derivatives are recognizable to those skilled in the art, without undue experimentation. Derivatives are described, for example, in Burger's Medicinal Chemistry and Drug Discovery, 5th Edition, Vol 1: Principles and Practice

The pharmaceutical composition may be administered by various routes, including oral, intravenous (i.v. or IV), intranasal (i.n. or IN), intramuscular (i.m. or IM), caudal, intrathecal, and subcutaneous (s.c.) routes.

### Pharmaceutical Compounds

The pharmaceutical compositions described herein may include hydrates, solvates, and complexes of t prucalopride and/or PF-04995274.

The present disclosure is also intended to include use of all isotopes of atoms occurring on prucalopride and PF-04995274Isotopes include those atoms having the same atomic number but different mass numbers. Isotopically-labeled compounds can generally be prepared by conventional techniques known to those skilled in the art or by processes analogous to those described herein using an appropriate isotopically-labeled reagents in place of the non-labeled reagents employed.

Prucalopride or PF-04995274 may be in a salt form. As used herein, a "salt" is a salt of the instant compound which has been modified by making acid or base, salts of the compounds. In the case of compounds used for treatment of mammals, the salt is pharmaceutically acceptable. Examples of pharmaceutically acceptable salts include, but are not limited to, mineral or organic acid salts of basic residues such as amines; alkali or organic salts of acidic residues such as phenols. The salts can be made using an organic or inorganic acid. Such acid salts are chlorides, bromides, sulfates, nitrates, phosphates, sulfonates, formates, tartrates, maleates, malates, citrates, benzoates, salicylates, ascorbates, and the like. Phenolate salts are the alkaline earth metal salts, sodium, potassium or lithium. The term "pharmaceutically acceptable salt" in this respect, refers to the relatively non-toxic, inorganic and organic acid or base addition salts of prucalopride or PF-04995274. These salts can be prepared in situ during the final isolation and purification of prucalopride or PF-04995274, or by separately treating prucalopride or PF-04995274in its free base or free acid form with a suitable organic or inorganic acid or base, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, e.g., Berge et al. (1977) "Pharmaceutical Salts", J. Pharm. Sci. 66:1-19).

As used herein, the term "physiologically functional derivative" refers to a compound (e.g., a drug precursor) that is transformed *in vivo* to yield the compound or its active metabolite, or a pharmaceutically acceptable salt, hydrate or solvate of the compound. The transformation may occur by various mechanisms (*e*.*g*., by metabolic or chemical processes), such as, for example, through hydrolysis in blood. Prodrugs are such derivatives, and a discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

### Dosages

An effective amount of prucalopride or PF-04995274 may a dose of about 0.01 to about 3 mg per kilogram of body weight of the subject (mg/kg), i.e., from about 0.01 mg/kg to about 3 mg/kg body weight. An effective amount of prucalopride or PF-04995274may range from 0.001 to approximately 3 mg/kg body weight, 0.001 to approximately 2 mg/kg body weight, from about 0.01 mg/kg to about 3 mg/kg body weight, from about 0.01 to about 2 mg/kg of body weight, about 0.01 to about 1.5 mg/kg of body weight, about 0.05 to about 1.4 mg/kg of body weight, about 0.05 to about 1.3 mg/kg of body weight, about 0.05 to about 1.2 mg/kg of body weight, about 0.05 to about 1.1 mg/kg of body weight, about 0.01 to about 1 mg/kg of body weight, or about 0.05 to about 0.7 mg/kg of body weight. In some aspects, the dose is about 0.05 to about 0.5 mg/kg. In some aspects, the dose is less than about 0.5 mg/kg, less that about 0.4 mg/kg, or less than about 0.3 mg/kg body weight. In some aspects, the effective amount of prucalopride or PF-04995274is a dose in the range of from about 0.01 mg/kg to about 1.5 mg/kg body weight. In some aspects, the effective amount of prucalopride or PF-04995274is a dose in the range of from about 0.01 mg/kg to about 1 mg/kg body weight. In some aspects, the effective amount of prucalopride or PF-04995274is a dose in the range of from about 0.01 mg/kg to about 0.75 mg/kg body weight. In some aspects, the effective amount of prucalopride or PF 04995274 is a dose in the range of from about 0.75 mg/kg to about 1.5 mg/kg body weight. In some aspects, the effective amount of prucalopride or PF 04995274 is a dose in the range of from about 0.5 mg/kg to about 1.2 mg/kg body weight. In some aspects, the effective amount of prucalopride or PF 04995274 is a dose in the range of from about 0.05 mg/kg to about 0.5 mg/kg. In some aspects, the effective amount of prucalopride or PF 04995274 is a dose of about 0.2 mg/kg or about 0.4 mg/kg body weight. In some aspects, the dose of prucalopride or PF 04995274 is, about 0.01 to about 1 mg/kg, about 0.1 to about 0.5 mg/kg, about 0.8 to about 1.2 mg/kg, about 0.7 to about 1.1 mg/kg, about 0.05 to about 0.7 mg/kg, about 0.01 mg/kg, about 0.05 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, or about 3 mg/kg body weight.

A dose of prucalopride or PF 04995274 per administration may be from about 1 to about 250 mg, from about 10 mg to about 300 mg, about 10 mg to about 250 mg, about 10 to about 200 mg, about 15 to about 175 mg, about 20 to about 175 mg, about 8 mg to about 32 mg, about 50 mg to about 75 mg, about 25 to about 150 mg, about 25 to about 125 mg, about 25 to about 100 mg, about 50 to about 100 mg, about 50 mg to about 75 mg, about 75 mg to about 100 mg, or about 75 mg to about 200 mg, about 1 mg, 2 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, and 250 mg. In some aspects, the dose of prucalopride or PF 04995274 is about 50 mg. In some aspects, the dose of prucalopride or PF 04995274 is about 75 mg. In some aspects, the total dose of prucalopride or PF 04995274 is about 100 mg.

In some aspects, the effective amount of prucalopride or PF-04995274is about 0.01 mg to about 1000 mg, from about 0.01 mg to about 500 mg, from about 0.1 mg to about 250 mg, or any amount or range therein. In another aspect, the effective amount of prucalopride or PF-04995274is, e.g., 0.01 mg, 0.025 mg, 0.05 mg, 0.1 mg, 0.5 mg, 1 mg, 5 mg, 10 mg, 25 mg, 50 mg, 55 mg, 60 mg, 65 mg, 70 mg, 75 mg, 80 mg, 90 mg, 95 mg, 100 mg, 150 mg, 200 mg, 250 mg, or 500 mg.

A therapeutically effective dose of prucalopride or PF-04995274may be adjusted depending on condition to be prophetically treated, the age, body weight, general health conditions, sex, and diet of the subject, dose intervals, administration routes, excretion rate, and combinations of drugs.

An initial dose of prucalopride or PF 04995274 may be larger, followed by one or more smaller maintenance doses. Other ranges are possible, depending on the subject's response to the treatment. An initial dose may be the same as, or lower or higher than subsequently administered doses.

The pharmaceutical composition may be administered daily, weekly, biweekly, several times daily, semi-weekly, every other day, bi-weekly, quarterly, several times per week, semi-weekly, monthly etc. The duration and frequency of treatment may depend upon the subject's response to treatment.

A subject may be administered 1 dose, 2 doses, 3 doses, 4 doses, 5 doses, 6 doses or more of the pharmaceutical composition. In certain embodiments, a single dose of the pharmaceutical composition is administered. In certain embodiments, multiple doses of the pharmaceutical composition (e.g., 2 doses, 3 doses, 4 doses, 5 doses, 6 doses, 7 doses, 8 doses, 9 doses, 10 doses or more) are administered.

In certain embodiments, when there are more than one doses of the pharmaceutical composition administered to a subject, the second dose is lower than the first dose. In certain embodiments, the second dose is an amount that is at most one-half, one-quarter, or one-tenth the amount of the first dose.

The number and frequency of doses may be determined based on the subject's response to administration of the composition, e.g., if one or more of the patient's symptoms improve and/or if the subject tolerates administration of the composition without adverse reaction.

In certain embodiments, the pharmaceutical composition is administered at least once a day, at least twice a day, at least three times per day, or more. In certain embodiments, the pharmaceutical composition is administered at least once a week, at least twice a week, at least three times per week, or more frequently. In certain embodiments, the pharmaceutical composition is administered at least twice per month, or at least once per month.

Treatment can continue as long as needed.

### Dosing Time Frame

The pharmaceutical composition is administered to a subject prior to a stressor. In certain embodiments, the pharmaceutical composition is administered to a subject both prior to and after a stressor. In certain embodiments, the pharmaceutical composition is administered to a subject prior to a stressor, and again prior to a recurrence of the stressor or a different stressor.

In certain embodiments, the pharmaceutical composition is administered to the subject about 2 days to about 3 weeks, about 3 days to about 3 weeks, about 4 days to about 3 weeks, about 5 days to about 3 weeks, about 6 days to about 3 weeks, about 2 days to about 2.5 weeks, about 3 days to about 2.5 weeks, about 4 days to about 2.5 weeks, about 5 days to about 2.5 weeks, about 6 days to about 2.5 weeks, about 1 week to about 2.5 weeks, about 1 week to about 2.5 weeks, about 1 week to about 2 weeks, about 2 days, about 3 days, about 4 days, about 5 days, about 6 days, about 1 week, about 8 days, about 9 days, about 10 days, about 11 days, about 12 days, about 13 days, about 2 weeks, about 2.5 weeks, or about 3 weeks, prior to a stressor.

In certain embodiments, the administration of the pharmaceutical composition is continued over a period of up to 2 days, up to 3 days, up to 4 days, up to 5 days, up to 6 days, up to 1 week, up to 2 weeks, up to 3 weeks, up to 4 weeks, about 2 days, about 3 days, about 4 days, about 5 days, about 6 days, about 1 week, about 2 weeks, about 3 weeks, about 4 weeks, about 5 weeks, about 6 weeks, about 7 weeks, about 8 weeks, about 9 weeks, about 10 weeks, or longer.

In certain embodiments, the pharmaceutical composition is administered once, twice, at least twice, at least three times, at least four times, at least five time, at least six times, at least seven times, at least eight times, at least nine times, or more per treatment.

In certain embodiments, the pharmaceutical composition is administered at least once a day, at least twice a day, at least three times per day, at least once a week, at least twice a week, at least three times a week, at least once per month, at least twice per month, or more frequently. Treatment can continue as long as needed. The pharmaceutical composition may be administered daily, weekly, biweekly, several times daily, semi-weekly, every other day, bi-weekly, quarterly, several times per week, semi-weekly, monthly etc. The duration and frequency of treatment may depend upon the subject's response to treatment.

### Stressors

A stressor is a stimulus that causes stress. It can be an event or other factor that disrupts the body's homeostasis of temperature, blood pressure, and/or other functions. In certain embodiments, a stressor is a traumatic or stressful event. Because humans have sophisticated brains and thought processes, anticipating a disruption can also be a stressor. In certain embodiments, a stressor is injury, trauma, combat, warfare, surgery, an accident, a criminal assault, child abuse, natural or human-caused disasters, a crash, grief, hunger, heat, cold, chemical exposure, autoimmune disease, infectious disease, viral infection, cancer, exhaustion, physical distress, neuropathy, hyperalgesia, allodynia, emotional distress, or depression. A traumatic event may be an event or something that threatens the person's life or the life of a close one or it could be something witnessed. U.S. Patent Application No. 20140018339.

A stressor may be acute, or may be chronic.

There are numerous physiological processes that are altered in response to stress. Among these are altered cortisol, corticotropin, catecholamine and serotonin levels. These levels return to baseline after an acute stressor is removed (McEwen N Eng J Med 1998 338(3):171-179). These biochemical markers of stress in turn lead to ill health and psychosocial disorders. Consequently, stress plays a major role in physical and mental health. Stress can affect the onset of, or susceptibility to disease. It can also affect the progression or course of disease even when there is another underlying pathophysiology of the disease. Recovery from an existing disease can also be delayed due to stress. For example, stress is a contributing factor to high blood pressure, heart disease, headaches, colitis, irritable bowel syndrome, temporo-mandibular joint disorder, cancer, peptic ulcers, insomnia, skin disorders and asthma. Stress can also aggravate other conditions such as multiple sclerosis, diabetes, herpes, mental illness, substance abuse and psychiatric disorders characterized by the presence of violent or aggressive tendencies. Particularly, stress contributes to functional somatic disorders, affective disorders and major depressive disorder (MDD). These include disorders such as chronic fatigue syndrome (CFS), fibromyalgia (FMS), Gulf War Syndrome, anxiety and post-traumatic stress disorder (PTSD). Stressors that disrupt normal exercise or sleep patterns.

Additional examples of use include administration prior to military deployment to protect Service members (active combat soldiers, battlefield surgeons, etc.) and even military working dogs against stress. Potential non-military use cases include, but are not limited to: police, firefighters, first responders, emergency medical technicians (EMTs), emergency room (ER) doctors, prison guards (and prisoners), humanitarian aid workers, and refugees.

A subject may be administered the pharmaceutical composition prior to a situation in which the subject (such as an early responder or military personnel) is likely to be exposed to traumatic stress, immediately after exposure to traumatic stress, and/or when the subject feels that his or her PTSD symptoms are likely to appear.

### Resilience to Stress

Resilience to stress refers to the capacity of a subject to adapt or change successfully, and/or to maintain physiological, neurological, or psychological homeostasis, in the face of a stressor (e.g., adversity). As used herein, the term "enhancing resilience" refers to increasing the ability of a subject to experience a stressor (e.g., a traumatic event) without suffering a stress-induced affective disorder, and/or with less post-event symptomatology or disruption of homeostasis and/or normal activities of daily living. Improving resilience can prevent a stress-induced affective disorder. Improving resilience can reduce at least one of the signs, symptoms, or symptom clusters of a stress-induced affective disorder. The pharmaceutical composition may enhance a subject's resilience to stress, help protect against developing stressor-related psychopathology, decrease the functional consequences of stressor-induced affective disorders or psychopathologies (e.g., PTSD, etc.), and reduce medical morbidity and mortality.

The Connor-Davidson Resilience Scale (CD-RISC) is a 25-item self-report scale, each rated on a 5-point scale (0-4), with higher scores reflecting greater resilience (Connor K M & Davidson, J R T. Development of a new resilience scale: the Connor-Davidson Resilience Scale (CD-RISC). Depression and Anxiety, 2003: 18: 71-82).

Resilience, psychological growth and life satisfaction may be measured with the CD-RISC, the Purpose in Life Scale, the abbreviated MOS Social Support Survey, the PTGI, and the Q-LES-Q.

### Combination Therapy

The pharmaceutical composition may be administered to a subject alone, or may be administered to a subject in combination with one or more other treatments/agents.

In certain embodiments, the second agent is an anti-depressant, an anxiolytic, or combinations thereof. In certain embodiments, the second agent is a serotonin reuptake inhibitor (SRI), or a selective serotonin reuptake inhibitor (SSRI). In certain embodiments, the second agent is fluoxetine, paroxetine, sertraline, lithium, riluzole, prazosin, lamotrigine, ifenprodil, or combinations thereof. In certain embodiments, the second agent is a dual serotonin norepinephrine reuptake inhibitor compound (DRI). In certain embodiments, the second agent is venlafaxine, duloxetine, milnacipran, or combinations thereof. In certain embodiments, the second agent is a non-tricyclic triple reuptake inhibitor (TRI).

In certain embodiments, the pharmaceutical composition is administered to a subject in combination with one or more treatments/agents such as antidepressants, analgesics, muscle relaxants, anorectics, stimulants, antiepileptic drugs, and sedative/hypnotics. Non-limiting examples of compounds that can be administered in combination with the pharmaceutical composition include, neurontin, pregabalin, pramipexole, L-DOPA, amphetamine, tizanidine, clonidine, tramadol, morphine, tricyclic antidepressants, codeine, carbamazepine, sibutramine, amphetamine, valium, trazodone and combinations thereof.

In certain embodiments, combination therapy means simultaneous administration of the agents in the same dosage form, simultaneous administration in separate dosage forms, or separate administration of the agents.

In certain embodiments, the second agent/treatment is used as adjunctive therapy to the pharmaceutical composition. In certain embodiments, the treatment includes a phase wherein treatment with the second agent/treatment takes place after treatment with the pharmaceutical composition has ceased. In certain embodiments, the treatment includes a phase where treatment with the pharmaceutical composition and treatment with the second agent/treatment overlap.

Combination therapy can be sequential or can be administered simultaneously. In either case, these drugs and/or therapies are said to be "co-administered." It is to be understood that "co-administered" does not necessarily mean that the drugs and/or therapies are administered in a combined form (i.e., they may be administered separately (e.g., as separate compositions or formulations) or together (e.g., in the same formulation or composition) to the same or different sites at the same or different times).

In certain embodiments, a subject is treated concurrently (or concomitantly) with the pharmaceutical composition and a second agent. In certain embodiments, a subject is treated initially with the pharmaceutical composition, followed by cessation of the treatment with the pharmaceutical composition and initiation of treatment with a second agent. In certain embodiments, the pharmaceutical composition is used as an initial treatment, e.g., by administration of one, two or three doses, and a second agent is administered to prolong the effect of the pharmaceutical composition, or alternatively, to boost the effect of the pharmaceutical composition. A person of ordinary skill in the art will recognize that other variations of the presented schemes are possible, e.g., initiating treatment of a subject with the pharmaceutical composition, followed by a period wherein the subject is treated with a second agent as adjunct therapy to the treatment with the pharmaceutical composition, followed by cessation of the treatment with the pharmaceutical composition.

The pharmaceutical composition and the other pharmaceutically active agent(s) may be administered together or separately and, when administered separately this may occur simultaneously or sequentially in any order. The amounts of the pharmaceutical composition and the other pharmaceutically active agent(s) and the relative timings of administration will be selected in order to achieve the desired combined therapeutic effect.

In various embodiments, the pharmaceutical and a second agent are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In certain embodiments, the pharmaceutical and the second agent are administered no more than 24 hours apart or no more than 48 hours apart. In certain embodiments, the pharmaceutical and the second agent are administered within the same patient visit. In other embodiments, the pharmaceutical composition and the second agent are administered concurrently. In other embodiments, the pharmaceutical composition and the second agent are administered at about 2 to 4 days apart, at about 4 to 6 days apart, at about 1 week part, at about 1 to 2 weeks apart, or more than 2 weeks apart. In certain embodiments, administration of the same agent may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same agent may be repeated and the administration may be separated by at least at least 1 day, 2 days, 3 days, 5 days, 10 days, 15 days, 30 days, 45 days, 2 months, 75 days, 3 months, or 6 months. In certain embodiments, a pharmaceutical composition and a second agent are administered to a subject in a sequence and within a time interval such that the pharmaceutical composition can act together with the other agent to provide an increased benefit than if they were administered otherwise. For example, the second active agent can be administered at the same time or sequentially in any order at different points in time; however, if not administered at the same time, they should be administered sufficiently close in time so as to provide the desired therapeutic or prophylactic effect. In one embodiment, the pharmaceutical composition and the second active agent exerts their effect at times which overlap. Each second active agent can be administered separately, in any appropriate form and by any suitable route. In other embodiments, the pharmaceutical composition is administered before, concurrently or after administration of the second active agent. The term "about" refers to ±10% of the referenced value. In other embodiments, courses of treatment are administered concurrently to a patient, i.e., individual doses of the second agent are administered separately yet within a time interval such that the pharmaceutical composition can work together with the second active agent. For example, one component can be administered once per week in combination with the other components that can be administered once every two weeks or once every three weeks. In other words, the dosing regimens are carried out concurrently even if the therapeutics are not administered simultaneously or during the same day. The second agent can act additively or synergistically with the pharmaceutical composition. In one embodiment, the prucalopride and/or PF 04995274 is/are administered concurrently with one or more second agents in the same pharmaceutical composition. In another embodiment, a pharmaceutical composition provided herein is administered concurrently with one or more second agents in separate pharmaceutical compositions. In still another embodiment, a pharmaceutical composition provided herein is administered prior to or subsequent to administration of a second agent. Also contemplated are administration of a pharmaceutical composition provided herein and a second agent by the same or different routes of administration, e.g., oral and parenteral. In certain embodiments, when the pharmaceutical composition provided herein is administered concurrently with a second agent that potentially produces adverse side effects including, but not limited to, toxicity, the second active agent can advantageously be administered at a dose that falls below the threshold that the adverse side effect is elicited.

### Anxiety Disorders

Treatment with the pharmaceutical composition may prevent an anxiety disorder such as stress-induced fear. The five major types of anxiety disorders are: panic disorder, obsessive-compulsive disorder, post-traumatic stress disorder, generalized anxiety disorder and phobias (including social phobia, also called social anxiety disorder). Each anxiety disorder has its own distinct features, but they are all bound together by the common theme of excessive, irrational fear and dread. It is common for an anxiety disorder to accompany depression, eating disorders, substance abuse, or another anxiety disorder.

Panic disorder is characterized by repeated episodes of intense fear that strike often and without warning. Physical symptoms include chest pain, heart palpitations, shortness of breath, dizziness, abdominal distress, feelings of unreality, and fear of dying. Obsessive-compulsive disorder is characterized by repeated, unwanted thoughts or compulsive behaviors that seem impossible to stop or control. Generalized Anxiety Disorder is characterized by exaggerated worrisome thoughts and tension about everyday routine life events and activities, lasting at least six months. Almost always anticipating the worst even though there is little reason to expect it; accompanied by physical symptoms, such as fatigue, trembling, muscle tension, headache, or nausea. Phobias are characterized into two major types of phobias, social phobia and specific phobia. People with social phobia have an overwhelming and disabling fear of scrutiny, embarrassment, or humiliation in social situations, which leads to avoidance of many potentially pleasurable and meaningful activities. People with specific phobia experience extreme, disabling, and irrational fear of something that poses little or no actual danger; the fear leads to avoidance of objects or situations and can cause people to limit their lives unnecessarily.

### Posttraumatic Stress Disorder (PTSD)

Typically, a subject suffering from PTSD was exposed to a traumatic event in which the person experienced, witnessed, or was confronted with an event or events that involved actual or threatened death or serious injury, or a threat to the physical integrity of self or others and the person's response involved intense fear, helplessness, or horror.

Having repeated intrusive memories of the trauma exposure is one of the core symptoms of PTSD. Patients with PTSD are known to display impairments in learning and memory during neuropsychological testing. Other core symptoms of PTSD include heightened stress sensitivity (startle), tension and anxiety, memory disturbances, and dissociation.

Typically, the traumatic event is persistently re-experienced in one or more of the following ways: recurrent and intrusive distressing recollections of the event, including images, thoughts, or perceptions, recurrent distressing dreams of the event, acting or feeling as if the traumatic event were recurring (includes a sense of reliving the experience, illusions, hallucinations, and dissociative flashback episodes, including those that occur on awakening or when intoxicated), intense psychological distress at exposure to internal or external cues that symbolize or resemble an aspect of the traumatic event, physiological reactivity on exposure to internal or external cues that symbolize or resemble an aspect of the traumatic event. An individual suffering from PTSD also has persistent avoidance of stimuli associated with the trauma and numbing of general responsiveness (not present before the trauma), as indicated by 3 or more of the following: efforts to avoid thoughts, feelings, or conversations associated with the trauma, efforts to avoid activities, places, or people that arouse recollections of the trauma, inability to recall an important aspect of the trauma, significantly diminished interest or participation in significant activities, feeling of detachment or estrangement from others, restricted range of affect (e.g., unable to have loving feelings), sense of a foreshortened future (e.g., does not expect to have a career, marriage, children, or a normal life span), persistent symptoms of increased arousal (not present before the trauma), as indicated by 2 or more of the following: difficulty falling or staying asleep, irritability or outbursts of anger, difficulty concentrating, hypervigilance, exaggerated startle response. The disturbance, which has lasted for at least a month, causes clinically significant distress or impairment in social, occupational, or other important areas of functioning.

Whatever the source of the problem, some people with PTSD repeatedly relive the trauma in the form of nightmares and disturbing recollections during the day. They may also experience other sleep problems, feel detached or numb, or be easily startled. They may lose interest in things they used to enjoy and have trouble feeling affectionate. They may feel irritable, more aggressive than before, or even violent. Things that remind them of the trauma may be very distressing, which could lead them to avoid certain places or situations that bring back those memories.

The disorder may be accompanied by depression, substance abuse, or one or more other anxiety disorders. In severe cases, the person may have trouble working or socializing.

### Major Depressive Disorder

Major depressive disorder refers to a class of syndromes characterized by negative affect and repeated episodes of depression without any history of independent episodes of mood elevation and over-activity that fulfill the criteria of mania. Multiple subtypes of major depressive disorders are recognized, including these with atypical characteristics, psychotic components, etc. The age of onset and the severity, duration and frequency of the episodes of depression are all highly variable. The disorder may begin at any age. The symptoms of major depressive disorder typically develop over days to weeks. Prodromal symptoms include generalized anxiety, panic attacks, phobias or depressive symptoms and may occur during several months preceding the episode. Individual episodes also last between 3 and 12 months but recur less frequently. Most patients are asymptomatic between episodes, but a minority of patients may develop a persistent depression, mainly in old age. Individual episodes of any severity are often precipitated by stressful life events. Common symptoms of a depressive episode include reduced concentration and attention; reduced self-esteem and self-confidence; ideas of guilt and unworthiness, ideas or acts of self-harm or suicide; disturbed sleep; and diminished appetite. A major depressive episode may follow a psychosocial stressor, e.g., death of a loved one, marital separation, childbirth or the end of an important relationship.

The lowered mood varies little from day to day and is often unresponsive to circumstances, yet may show a characteristic diurnal variation as the day goes on. As with manic episodes, the clinical presentation shows marked individual variations, and atypical presentations are particularly common in adolescence. In some cases, anxiety, distress, and motor agitation may be more prominent at times that the depression, and the mood change may also be masked by added features such as irritability, excessive consumption of alcohol, histrionic behavior, and exacerbation of pre-existing phobic or obsessional symptoms, or by hypochondria.

### Psychiatric evaluations

The effects or efficacy of treatment with the pharmaceutical composition may be evaluated by the subject and/or a medical professional, e.g., the subject's physician. The evaluation may be conducted within about 10 minutes, within about 15 minutes, within about 20 minutes, within about 25 minutes, within about 0.5 hours, within about 1 hour, within about 2 hours, within about 2.5 hours, within about 3 hours, within about 3.5 hours, within about 4 hours, within about 4.5 hours, within about 5 hours, within about 5.5 hours, within about 6 hours, within about 6.5 hours, within about 7 hours, within about 7.5 hours, within about 8 hours, within about 8.5 hours, within about 9 hours, within about 9.5 hours, within about 10 hours, within about 10.5 hours, within about 11 hours, within about 11.5 hours, within about 12 hours, within about 18 hours, within about 1 day, within about 2 days, within about 3 days, within about 4 days, within about 5 days, within about 6 days, within about 1 week, within about 2 weeks, within about 3 weeks, within about 4 weeks, within about 1 month, within about 2 months, within about 3 months, within about 4 months, within about 5 months, within about 6 months, within about 1 year, within about 2 years, or longer, following a stressor and administration of the pharmaceutical composition.

Psychiatric evaluations of a patient being treated with the pharmaceutical composition can be conducted to determine whether the pharmaceutical composition is effective. The psychiatric evaluation may be carried out before treatment, at the time of treatment, during treatment, and/or after treatment. When the psychiatric evaluation is carried out both before treatment and after (and/or during) treatment with the pharmaceutical composition, the results of the evaluation before treatment can provide a baseline for comparison to the results of the evaluation during and/or after treatment. Psychiatric evaluation may be conducted only after treatment.

Psychophysiological stress tests can be performed to measure the amount of stress-induced anxiety present in the various systems of the body (i.e. muscular, cardiovascular, digestive, respiratory and neurological systems). These stress tests are routinely used in the art. Test results are compared to both local and national norms, to determine if the individual is exhibiting an excessive amount of physiological anxiety and whether or not they are able to recover from a standardized stressful stimuli in an appropriate length of time.

Psychiatric testing can be used to monitor a subject to determine the emotional and/or social etiology of the stress disorder. These tests are known in the art and include health-related assessments, mental health assessments, personality tests, and personality type assessment.

Clinician-administered evaluation and/or self-report instruments may be used, with the aim of measuring baseline symptomatology as well as drug actions on (1) the overall severity of the disorder, (2) the core symptoms, and (3) depressed mood.

Non-limiting examples of psychiatric evaluation tools and questionnaires include the following measures.

The Diagnostic and Statistical Manual of Mental Disorders (DSM-5) includes the revised diagnostic criteria for PTSD. See, American Psychiatric Association: Diagnostic and Statistical Manual of Mental Disorders, Fifth Edition. Arlington, Va., American Psychiatric Association, 2013. See also ptsd.va.gov/professional/PTSD-verview/dsmS criteria ptsd.asp.

The Structured Clinical Interview for DSM-IV Axis I Disorders, Patient Edition (SCID-P) is a semi-structured interview that provides probe questions as well as follow-up questions to be asked by the clinician to assist in diagnosis. First et al., Structured Clinical Interview for DSM-IV TR Axis I Disorders, Research Version, Patient Edition (SCID-I/P). New York: New York State Psychiatric Institute, Biometrics Research; 2001. It includes an overview to obtain information about demographics, work, chief complaint, history of present illness, past history, treatment history, and current functioning. The main body of SCID-P includes 9 modules that are designed to diagnose 51 mental illnesses in all.

The SCID-P for DSM-5 is the SCID--Patient version, and is the next edition of the SCID modified to incorporate the new DSM-5 criteria.

The Clinician-Administered PTSD Scale (CAPS) is a structured clinical interview designed to assess the essential features of PTSD as defined by the DSM-IV. Weathers et al., Clinician-administered PTSD scale: a review of the first ten years of research. Depress Anxiety. 2001; 13(3):132-156. The CAPS can be used to provide categorical ratings of diagnostic status as well as a quantitative index of symptom severity. Both frequency and intensity scores are derived for each individual symptom. The CAPS total score is based on an individual's response to the 17 items that assess the frequency and intensity of current PTSD symptoms. Subscales of the CAPS are utilized to assess specific symptom clusters. The total score can range from 0 to 136.

The Clinician-Administered PTSD Scale for DSM-5 (CAPS-5) is a 30-item structured interview that can be used to make current (past month) diagnosis of PTSD, make lifetime diagnosis of PTSD, and to assess PTSD symptoms over the past week. CAPS-5 is a 30-item questionnaire, corresponding to the DSM-5 diagnosis for PTSD. The language of the CAPS-5 reflects both changes to existing symptoms and the addition of new symptoms in DSM-5. Weathers, F. W., et al (2013). The Clinician-Administered PTSD Scale for DSM-5 (CAPS-5).

The Treatment Outcome PTSD Scale (TOP-8) is a brief interviewer-administered scale designed specifically for the assessment of commonly occurring signs and symptoms of PTSD that are subject to change in response to treatment (Davidson, J. R., & Colket, J. T. (1997). The eight-item treatment-outcome post-traumatic stress disorder scale: A brief measure to assess treatment outcome in post-traumatic stress disorder. International Clinical Psychopharmacology, 12(1), 41-45). The TOP-8 is comprised of eight items, each measured on a scale of 0-4, with defined anchors given for each item. The items are representative of the three core features of PTSD with a maximum possible score of 32.

The Hamilton Psychiatric Rating Scale for Anxiety (HAM-A) is a widely used observational rating measure of anxiety severity. The scale consists of 14 items. Each item is rated on a scale of 0 to 4. This scale is administered to assess the severity of anxiety and its improvement during the course of treatment. The HAM-A total score is the sum of the 14 items and the score ranges from 0 to 56. Hamilton M. The Assessment of Anxiety-States by Rating. Br J Med Psychol. 1959; 32(1):50-55.

The Montgomery-Asberg Depression Rating Scale (MADRS) is a 10-item instrument used for the evaluation of depressive symptoms in adults and for the assessment of any changes to those symptoms. Montgomery S. A., et al., A new depression scale designed to be sensitive to change. Br J Psychiatry. 1979 April; 134:382-389. Each of the 10 items is rated on a scale of 0 to 6, with differing descriptors for each item. These individual item scores are added together to form a total score, which can range between 0 and 60 points.

The Young Mania Rating Scale, item 1 (YMRS-1) used to assess mood elevation on the infusion days. Young R C, et al. Rating-Scale for Mania--Reliability, Validity and Sensitivity. Br J Psychiatry. 1978; 133(NOV):429-435.

The Brief Psychiatric Rating Scale (BPRS) is used to assess acute behavioral changes during the infusions. Overall J E et al., The Brief Psychiatric Rating-Scale. Psychol. Rep. 1962; 10(3):799-812 Four key BPRS items for the positive (+) symptoms of psychosis are used: conceptual disorganization, hallucinatory behavior, suspiciousness, and unusual thought content. Three items representing the negative (-) symptoms of psychosis will also be used: blunted affect, emotional withdrawal, and motor retardation.

The Clinician-Administered Dissociative States Scale (CADSS) is used to measure dissociative effects during the infusions. Bremner J D, et al., Measurement of Dissociative States with the Clinician-Administered Dissociative States Scale (CADSS). J Trauma Stress. 1998; 11(1): 125-136 The scale includes 19 questions and 8 observer ratings scored from 0 (not at all) to 4 (extremely). The CADSS measures impairment in body perception, environmental perception, time perception, memory impairment, and feelings of unreality.

The Patient Rating Inventory of Side Effects (PRISE) is a patient self-report used to qualify side effects by identifying and evaluating the tolerability of each symptom. Levine J, Schooler N R. SAFTEE: A technique for the systematic assessment of side effects in clinical trials. Psychopharmacol Bull. 1986; 22(2):343-381.

The Clinical Global Impression (CGI) scale assesses treatment response in psychiatric patients. The administration time is 2 minutes. This scale consists of three items: Severity of Illness (item 1); Global Improvement (item 2); and Efficacy Index (item 3). Item 1 is rated on a seven-point scale (1=normal, 7=among the most extremely ill patients) as is item 2 (1=very much improved, 7=very much worse). Each includes an additional response of "not assessed." Item 3 is rated on a four-point scale (from "none" to "outweighs therapeutic effect").

The Impact of Events Scale (IES) is one of the most widely used self-report measures of stress reactions to traumatic events. Horowitz et al., Impact of Event Scale: a measure of subjective stress. Psychosom Med. 1979 May; 41(3):209-218. See also, Weiss et al., The Impact of Event Scale--Revised In: Wilson J, Keane T M, eds. Assessing psychological trauma and PTSD. New York: Guilford; 1996:399-411. It measures both intrusion and avoidance. Sundin et al., Impact of Event Scale: psychometric properties. Br J Psychiatry. 2002 March; 180:205-209. Joseph S. Psychometric evaluation of Horowitz's Impact of Event Scale: a review. J Trauma Stress. 2000 January; 13(1):101-113. The total score can range from 0 to 75.

The Posttraumatic Stress Disorder Checklist (PCL-5) is a 17-item self-report measure reflecting DSM-5 symptoms of PTSD. The PCL-5 measures symptoms in response to stressful situations (Weathers, F., et al. (1993). The PTSD checklist (PCL): Reliability, validity, and diagnostic utility. Annual Convention of the International Society for Traumatic Stress Studies, San Antonio, Tex.).

The Quick Inventory of Depressive Symptomatology, Self Report (QIDS-SR) is a 16-item self-rated instrument designed to assess the severity of depressive symptoms present in the past seven days. Rush A J, Trivedi M H, Ibrahim H M et al. The 16-Item quick inventory of depressive symptomatology (QIDS), clinician rating (QIDS-C), and self-report (QIDS-SR): a psychometric evaluation in patients with chronic major depression. Biol. Psychiatry. 2003; 54(5):573-583. The 16 items cover the nine symptom domains of major depression, and are rated on a scale of 0-3. Total score ranges from 0 to 27, with ranges of 0-5 (normal), 6-10 (mild), 11-15 (moderate), 16-20 (moderate to severe), and 21+ (severe).

The Childhood Trauma Questionnaire (CTQ) is a 28-item self-report instrument that assesses childhood trauma in the following areas: physical, sexual and emotional abuse and physical and emotional neglect. Bernstein D P, Stein J A, Newcomb M D et al. Development and validation of a brief screening version of the Childhood Trauma Questionnaire. Child Abuse Negl. 2003 February; 27(2):169-190. Each item is rated on a scale of 1 (never true) to 5 (very often true). The 5 subscales are then totaled, with scores ranging from 5-25 for each traumatic category.

Visual Analogue Scales (VAS) are used to assess subjective state changes. Bond A, Lader M. The use of analogue scales in rating subjective feelings. Br J Med Psychol. 1974; 47(3):211-218. They are 100-mm horizontal lines marked proportionately to the perceived intensity of the subjective experience (0=not at all, to 10=extremely) for the following states: anxious, depressed, drowsy, high, hungry, and nauseous.

The Sheehan Disability Scale (SDS) is a self-report disability measure. It has demonstrated sensitivity to impairment and changes as a result of treatment across a wide range of psychiatric disorders. The SDS asks only about current levels of impairment, providing no indication of whether the person has done better or worse in the past, thus making it a reasonable short-term outcome measure that is un-confounded by historical impressions. The dependent variable is the total score, which is based on the sum of three 10-point items (work, social life, and family life), with higher scores reflecting greater disability. Sheehan D. The Anxiety Disease. New York, N.Y.: Scribner; 1983.

The Wechsler Abbreviated Scale of Intelligence 2-Subtest (WASI-2) is a reliable brief measure of IQ for 6 to 89 year-olds that includes Vocabulary (an estimate of verbal fluid abilities) and Matrix Reasoning (an estimate of nonverbal fluid abilities). Wechsler D. Wechsler Abbreviated Scale of Intelligence San Antonio, Tex.: Psychological Corporation; 1999. It is extensively used in clinical, educational, and research settings. Average reliability coefficient is 0.96 and test-retest reliability is 0.88.

The Hopkins Verbal Learning Test (HVLT) is a repeatable test of memory acquisition and delayed recall of words. Subjects are presented with the same 12-item list for 3 learning trials and asked each time to repeat the items on each list. Delayed recall and recognition conditions are administered later. Dependent variables used in this study include total learning over the 3 trials (for the acquisition variable) and total delayed recall score (for the recall component). Brandt J, Benedict R. Hopkins Verbal Learning Test, Revised. Odessa, Fla.: Psychological Assessment Resources; 1997.

The Profile of Mood States-Bipolar (POMS-Bi) scale measures moods and feelings primarily in clinical rather than nonclinical settings. It can help to determine an individual's psychiatric status for therapy, or be used to compare mood profiles associated with various personality disorders. It is also a useful instrument in identifying the effects of drug treatments.

The Post-Traumatic Cognitions Inventory (PTCI) is a 33-item scale, which is rated on a Likert-type scale ranging from 1 (totally disagree) to 7 (totally agree). Scale scores are formed for the three subscales, which show a high degree of intercorrelation (rs=0.57-0.75).

The New Cognitions scale is a 6-item pilot scale, which is rated on a Likert-type scale ranging from 1 (not at all) to 4 (a lot). The scale is based on the Post Traumatic Growth Inventory (PTGI) from which items have been directly selected (new items were added to the scale as well), and on the Brief-COPE (see Carver, C. S. (1997) "You want to measure coping but your protocol's too long: Consider the brief COPE." International Journal of Behavioral Medicine 4; 92-100).

The Medical Outcomes Study (MOS) Social Support Survey is a 19-item self-report measure designed to assess levels of functional social support. The MOS-SS has two subscales (emotional and instrumental social support) to identify potential social support deficits (Sherbourne, C. D. & Stewart, A. L. (1991). "The MOS Social Support Survey." Soc Sci Med 32(6): 705-714).

The Purpose in Life test-Short Form (PIL-SF) is a brief, 4-item form of the 20-item Purpose in Life test. This scale asks respondents to report to what extent they have achieved their goals in life, and to what extent they perceive their life to be meaningful or purposeful. (Schulenberg et al 2010; Psychotherapy (Chic). 2008 December; 45(4):447-63).

Posttraumatic Growth Inventory (PTGI)-Short Version is a 10-item shortened version of the PTGI self-report questionnaire (ref). It asks respondents to rate the extent to which they have changed as the result of experiencing a highly stressful life event. Items span positive changes in five domains: relating to others, new possibilities, personal strength, spiritual change, and appreciation of life (Cann, A., et al. (2010). A short form of the Posttraumatic Growth Inventory. Anxiety, Stress & Coping, 23, 127-137).

The Quality of Life Enjoyment and Satisfaction Questionnaire (Q-LES-Q) is a self-report scale measuring the degree of enjoyment and satisfaction experienced by subjects in various areas of daily functioning. The summary scores are reliable and valid measures of these dimensions in a group of depressed subjects (Endicott J, et al. Quality of Life Enjoyment and Satisfaction Questionnaire: A New Measure. Psychopharmacology Bulletin; 1993; 29:321-326).

Self-evaluation of the subject being treated may be conducted.

### Pharmaceutical Compositions

The pharmaceutical composition, which comprises the prucalopride and/or PF-04995274 or salts or solvates thereof, may comprise one or more pharmaceutically acceptable carriers, diluents, or excipients. The carrier(s), diluent(s) or excipient(s) must be acceptable in the sense of being compatible with the other ingredients of the formulation and not deleterious to the recipient thereof. A process for the preparation of a pharmaceutical composition may include admixing the prucalopride and/or PF-04995274, or salts or solvates thereof, with one or more pharmaceutically acceptable carriers, diluents or excipients.

The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions may encompass any composition made by admixing prucalopride and/or PF-04995274, and pharmaceutically acceptable excipients.

Acceptable excipients, diluents, and carriers for therapeutic use are well known in the pharmaceutical art, and are described, for example, in Remington: The Science and Practice of Pharmacy. Lippincott Williams & Wilkins (A. R. Gennaro edit. 2005). The choice of pharmaceutical excipient, diluent, and carrier can be selected with regard to the intended route of administration and standard pharmaceutical practice.

As used herein, the phrase "pharmaceutically acceptable" refers to molecular entities and compositions that are "generally regarded as safe", e.g., that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopoeia or other generally recognized pharmacopeias for use in animals, and more particularly in humans.

Pharmaceutical compositions may be presented in unit dose forms containing a predetermined amount of active ingredient per unit dose. Such a unit may contain, for example, 5 µg to 1 g, preferably 1 mg to 700 mg, more preferably 5 mg to 100 mg of prucalopride and/or PF-04995274, depending on the condition being treated, the route of administration and the age, weight and condition of the patient. Such unit doses may therefore be administered more than once a day. Preferred unit dosage compositions are those containing a daily dose or sub-dose (for administration more than once a day), as herein above recited, or an appropriate fraction thereof, of an active ingredient. Furthermore, such pharmaceutical compositions may be prepared by any of the methods well known in the pharmacy art.

Pharmaceutical compositions may be adapted for administration by any appropriate route, for example by the oral (including buccal or sublingual), inhaled, nasal, ocular, or parenteral (including intravenous and intramuscular) route. The pharmaceutical composition may be injected. Such pharmaceutical compositions may be prepared by any method known in the art of pharmacy, for example by bringing into association the active ingredient with the carrier(s) or excipient(s).

Pharmaceutical compositions which are adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. Prucalopride and/or PF-04995274 can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

It should be understood that, in addition to the ingredients particularly mentioned above, the pharmaceutical compositions may include other agents conventional in the art having regard to the type of formulation in question, for example those suitable for oral administration may include flavoring agents.

A therapeutically effective amount of prucalopride and/or PF-04995274 will depend upon a number of factors including, for example, the age and weight of the subject, the precise condition requiring treatment and its severity, the nature of the formulation, and the route of administration, and will ultimately be at the discretion of the attendant physician or veterinarian.

### Kits

Also provided are kits for use in prophylactically treating the stress-induced affective disorders or psychopathologies.

The kits can include the pharmaceutical composition, and instructions providing information to a health care provider regarding usage. The kit may optionally contain a second agent or composition. Instructions may be provided in printed form or in the form of an electronic medium such as a floppy disc, CD, or DVD, or in the form of a website address where such instructions may be obtained. A unit dose of the pharmaceutical composition, or a second agent or composition, can include a dosage such that when administered to a subject, a therapeutically or prophylactically effective plasma level of the compound or composition can be maintained in the subject for at least 1 days. A compound or composition can be included as a sterile aqueous pharmaceutical composition or dry powder (e.g., lyophilized) composition. Suitable packaging may be provided. As used herein, "packaging" includes a solid matrix or material customarily used in a system and capable of holding within fixed limits the pharmaceutical composition and/or a second agent suitable for administration to a subject. Such materials include glass and plastic (e.g., polyethylene, polypropylene, and polycarbonate) bottles, vials, paper, plastic, and plastic-foil laminated envelopes and the like.

The kits described herein contain one or more containers, which contain compounds, signaling entities, biomolecules and/or particles as described. The kits also contain instructions for mixing, diluting, and/or administrating the compounds. The kits also include other containers with one or more solvents, surfactants, preservative and/or diluents (e.g., saline (0.9% NaCl), or 5% dextrose) as well as containers for mixing, diluting or administering the components to the sample or to the patient in need of such treatment.

The compositions of the kit may be provided as any suitable form, for example, as liquid solutions or as dried powders. When the composition provided is a dry powder, the powder may be reconstituted by the addition of a suitable solvent, which may also be provided. Where liquid forms of the pharmaceutical composition are used, the liquid form may be concentrated or ready to use. The solvent will depend on the compound and the mode of use or administration. Suitable solvents for drug compositions are well known and are available in the literature. The solvent will depend on the compound and the mode of use or administration.

The kits comprise a carrier being compartmentalized to receive in close confinement one or more container such as vials, tubes, and the like, each of the container comprising one of the separate elements to be used in the method. For example, one of the container may comprise a positive control in an assay. Additionally, the kit may include containers for other components, for example, buffers useful in the assay.

This invention will be better understood from the Examples, which follow. However, one skilled in the art will readily appreciate that the specific methods and results discussed are merely illustrative.

### EXAMPLES

### EXAMPLE 1: Prophylactic efficacy of 5-HT4R agonists against stress

Enhancing stress resilience could protect against stress-induced psychiatric disorders in at-risk populations. We have previously reported that (*R*,*S*)-ketamine acts as a prophylactic against stress when administered 1 week before stress. While we have shown that the selective 5-hydroxytryptamine (5-HT) (serotonin) reuptake inhibitor (SSRI) fluoxetine (Flx) is ineffective as a prophylactic, we hypothesized that other serotonergic compounds such as serotonin 4 receptor (5-HT₄R) agonists could act as prophylactics. We tested if three 5-HT₄R agonists with varying affinity could protect against stress in two mouse strains by utilizing chronic corticosterone (CORT) administration or contextual fear conditioning (CFC). Mice were administered saline, (*R,S*)-ketamine, Flx, RS-67,333, prucalopride, or PF-04995274 at varying doses and then 1 week later were subjected to chronic CORT or CFC. In C57BL/6N mice, chronic Flx administration attenuated CORT-induced weight changes and increased open arm entries in the elevated plus maze (EPM). Chronic RS-67,333 administration attenuated CORT-mediated weight changes and protected against depressive- and anxiety-like behavior. In 129S6/SvEv mice, RS-67,333 attenuated learned fear in male, but not female mice. RS-67,333 was ineffective against stress-induced depressive-like behavior in the forced swim test (FST) but prevented anxiety-like behavior in both sexes. Prucalopride and PF-04995274 attenuated learned fear and decreased stress-induced depressive-like behavior. Electrophysiological recordings following (*R,S*)-ketamine or prucalopride administration revealed that both drugs alter AMPA receptor-mediated synaptic transmission in CA3. These data show that in addition to (*R,S*)-ketamine, 5-HT₄R agonists are also effective prophylactics against stress, suggesting that the 5-HT₄R may be a novel target for prophylactic drug development.

Here, we hypothesized that since 5-HT₄Rs have been heavily implicated in depression and anxiety, they may have a role in stress resilience. We focused our studies on three 5-HT₄R agonists with varying affinity. First, RS-67,333 (1-(4-amino-5-chloro-2-methoxyphenyl)-3-[1(n-butyl)-4-piperidinyl]-1-propanone HCl) is a high-affinity 5-HT₄R partial agonist [22]. This drug is effective in improving behavioral deficits, decreasing the number of amyloid plaques as well as level of amyloid beta (Aβ) species, and decreasing hippocampal astrogliosis and microgliosis in the 5xFAD mouse model of Alzheimer's disease (AD) [23]. Second, prucalopride (4-amino-5-chloro-2,3-dihydro-*N*-[1-3-methoxypropyl)-4-piperidinyl]-7-benzofuran carboxamide monohydrochloride) is a selective, high affinity 5-HT₄R agonist [24]. In 2018, it was approved by the FDA for chronic constipation and is currently being tested for chronic intestinal pseudo-obstruction. Prucalopride has also been tested in two separate clinical trials to investigate its effects on emotional processing in health volunteers after an acute (e.g., single dose) or chronic (e.g., 1 week) administration [25,26]. Third, PF-04995274 (4-[4-[4-Tetrahydrofuran-3-yloxy)-benzo[d]isoxazol-3-yloxymethyl]-piperidin-1-ylmethyl]-tetrahydropyran-4-ol) is a potent, partial 5-HT₄R agonist [27]. A clinical trial was conducted to evaluate PF-04995274, alone or in combination with donepezil, on scopolamine-induced deficits in psychomotor and cognitive function in healthy adults; however, this trial was terminated, but not due to safety concerns [28]. Currently, a clinical trial is underway to test whether adjunctive administration of PF-04995247 has positive effects on emotional processing and neural activity in mediated, treatment-resistant (TRD) depressed patients compared to placebo [29].

To determine if 5-HT₄R agonists may be potential prophylactics against stress, we utilized two different stress models (acute and chronic) in two different strains of mice (C57BL/6NTac and 129S6/SvEv). We found that RS-67,333, prucalopride, and PF-04995274 attenuate learned fear. RS-67,333 prevents depressive-like behavior when administered chronically and stress-induced anxiety-like behavior in both sexes when administered acutely. Prucalopride and PF-04995274 decrease stress-induced depressive-like behavior in the FST. To investigate shared or distinct mechanisms of prophylactic (*R,S*)-ketamine and 5-HT₄R agonists, we utilized slice electrophysiology to investigate spontaneous glutamatergic transmission in CA3. We found that (*R,S*)-ketamine and prucalopride attenuate bursts of large amplitude AMPA receptor-mediated synaptic currents. These data suggest that in addition to (*R,S*)-ketamine, 5-HT₄R agonists are also effective prophylactics against stress and may alter AMPA-related glutamatergic transmission to enhance stress resilience.

### MATERIALS AND METHODS

**Mice:** All mice were housed in a 12-h (06:00-18:00) light-dark colony room at 22°C. Food and water were provided *ad libitum.* Behavioral testing was performed during the light phase. **C57BL/6NTac mice:** Male C57BL/6NTac mice were purchased from Taconic Farms (Lille Skensved, Denmark) at 8 weeks of age and were housed 5 per cage before the start of CORT treatment. All testing was conducted in compliance with the laboratory animal care guidelines and with protocols approved by the Institutional Animal Care and Use Committee (IACUC) (European Directive, 2010/63/EU for the protection of laboratory animals, permissions # 92-256B, authorization ethical committee CEEA n°26 2012_098). **129S6/SvEv mice:** Male and female 129S6/SvEvTac mice were purchased from Taconic (Hudson, NY) at 7-8 weeks of age. The procedures described herein were conducted in accordance with the National Institutes of Health (NIH) regulations and approved by the IACUC of the New York State Psychiatric Institute (NYSPI).

### Stress models:

**Corticosterone (CORT) model:** In this model, glucocorticoid levels are exogenously increased in C57BL/6NTac mice. This chronic CORT elevation dysregulates the hypothalamic-pituitary-adrenal axis (HPA) in a manner similar to that observed in clinical depression. The dose and duration of CORT treatment was selected based on previous studies [20,30]. CORT (35 µg/ml, equivalent to about 5 mg/kg/day) dissolved in 0.45% hydroxypropyl-β-cyclodextrin (β-CD) or vehicle (VEH) (0.45% β-CD) was available *ad libitum* in the drinking water in opaque bottles to protect it from light. VEH- and CORT-treated water was changed every 3 days to prevent possible degradation.

**Contextual Fear Conditioning (CFC):** A 3-shock CFC procedure was administered as previously published [31,32]. Briefly, mice were placed into context A and administered 3 2-s shocks (0.75 mA) at 180 s, 240 s or 300 s following placement into context A. Mice were removed from the context 15 s following the termination of shock (at 317 s). For context retrieval, mice were placed back into context A for 300 s.

**Electrophysiology:** Electrophysiology was conducted as previously described [33].

**Statistical Analysis:** Results from data analyses are expressed as means ± SEM. Alpha was set to 0.05 for all analyses. Data were analyzed using GraphPad Prism v7.0 or v8.0. For all experiments, unless otherwise noted, one- or two-way ANOVAs with repeated-measures were applied to the data as appropriate. Significant main effects and/or interactions were followed by Fisher's PLSD post hoc analysis or unpaired t-tests. All main effects, interactions, and p values are listed in **Table 2.**

**Drugs:** All drugs were prepared in physiological saline and all injections were administered intraperitoneally (i.p.) in volumes of 0.1 cc per 10 mg body weight unless otherwise noted. **Fluoxetine hydrochloride (Flx):** Flx (BioTrend Chemicals AG, BG197, Zurich, Germany) was administered in the drinking water (18 mg/kg/day) for 3 weeks before the start of CORT. **RS-67,333 (RS):** RS-67,333 (Tocris Bioscience, 0989, Bristol, United Kingdom) was administered chronically or in a single injection. For the chronic experiment, RS-67,333 (1.5 mg/kg/day) was administered via ALZET osmotic minipumps (ALZET, Model 2004, Cupertino, CA) [30]. For the acute experiment, RS-67,333 was administered in a single dose of 1.5, 10, or 30 mg/kg of body weight 1 week before the start of CFC. RS-67,333 was dissolved in saline using an ultrasonic homogenizer (BioLogics, Model 3000, Manassas, VA). **(R,S)-ketamine (K):** (*R*,*S*)-ketamine (Ketaset III, Ketamine HCl injection, Fort Dodge Animal Health, Fort Dodge, IA) was administered in a single dose at 30 mg/kg of body weight 1 week before the start of CFC. A dose of 30 mg/kg of body weight was chosen in the 129S6/SvEv experiments, as previous studies indicated that is the effective dose for prophylactic efficacy [S1].

**Prucalopride:** Prucalopride (Sigma, 179474-81-8, St. Louis, MO) was administered a single dose at 3 or 10 mg/kg of body weight 1 week before the start of CFC. Prucalopride was dissolved in saline using an ultrasonic homogenizer (BioLogics, Model 3000, Manassas, VA). **PF-04995274:** PF-04995274 (Sigma, Catalog No. 1331782-27-4, St. Louis, MO) was administered a single dose at 3 or 10 mg/kg of body weight 1 week before the start of CFC. PF-04995274 was dissolved in saline using an ultrasonic homogenizer (BioLogics, Model 3000, Manassas, VA).

**Osmotic minipump implantation:** ALZET osmotic minipumps (Model 2004, 0.25 µl/hr, 28 days) were implanted subcutaneously under isoflurane anesthesia as previously described [S2]. Osmotic minipumps were rotated under the skin two to three times per week.

**Behavioral Assays:** All experiments were approved by the Institutional Animal Care and Use Committee (IACUC) at the New York Psychiatric Institute (NYSPI).

**Elevated Plus Maze (EPM):** Testing was performed as previously described [S3]. Briefly, the maze is a plus-cross-shaped apparatus consisting of four arms, two open and two enclosed by walls, linked by a central platform at a height of 50 cm from the floor. Mice were individually placed in the center of the maze facing an open arm and were allowed to explore the maze for 5 min. The time spent in and the number of entries into the open arms was used as an anxiety index. Videos were scored using ANY-maze behavior tracking software (Stoelting, Wood Dale, IL).

**Novelty-Suppressed Feeding:** The NSF is a conflict test that elicits competing motivations: the drive to eat and the fear of venturing into the center of a brightly lit arena. The latency to feed is used as an index of anxiety -like behavior, because classical anxiolytic drugs decrease this measure. The NSF test was carried out during an 8 min period as previously described [S3]. Briefly, the testing apparatus consisted of a plastic box (50x50x20 cm), the floor of which was covered with approximately 2 cm of bedding. For 129S6/SvEv experiments, mice were food restricted for 12 h. For the C57BL/6N experiments, mice were food restricted for 24 h. At the time of testing, a single pellet of food (regular chow) was placed on a paper platform positioned in the center of the box. Each animal was placed in a corner of the box, and a stopwatch was immediately started. The latency to feed (defined as the mouse biting the pellet) was timed. Immediately afterwards, the animal was transferred to its home cage, and the amount of food consumed by the mouse in the subsequent 5 min was measured, serving as a control for change in appetite as a possible confounding factor.

**Splash Test:** This test consisted of squirting 200 µl of a 10% sucrose solution on the mouse's snout. The grooming duration was quantified using Stopwatch+ (Center for Behavioral Neuroscience, Georgia State University).

**Forced Swim Test (FST):** The FST is typically used in rodents to screen for potential human antidepressants [S4,S5]. In fact, many papers examining ketamine in mouse models only observe effects in the FST [S6-S8]. In the FST, time spent immobile, as opposed to swimming, is used as a measure of depressive behavior. The FST was administered as previously described [1]. Briefly, mice were placed into clear plastic buckets 20 cm in diameter and 23 cm deep filled 2/3 of the way with 22°C water. Mice were videotaped from the side for 6 min and were exposed to the swim test on 2 consecutive days. Immobility time was scored by an experimenter blind to the experimental groups.

**Open Field (OF):** The OF assay was administered as previously described [3]. Briefly, motor activity was quantified in four Plexiglas open field boxes 43x43 cm² (MED Associates, Georgia, VT). Two sets of 16 pulse-modulated infrared photobeams on opposite walls 2.5-cm apart recorded x-y ambulatory movements. Activity chambers were computer interfaced for data sampling at 100-ms resolution. The computer defined grid lines that dividing center and surround regions, with the center square consisting of four lines 11 cm from the wall.

**Electrophysiology:** One week after saline, (*R,S*)-ketamine (30 mg/kg), or prucalopride (3 mg/kg) injection, mice were anesthetized by isoflurane inhalation, decapitated, and brains rapidly removed. CA3 slices (350 µm) were cut on a vibratome (Leica VT1000S) in ice cold partial sucrose artificial cerebrospinal fluid (ACSF) solution (in mM): 80 NaCl, 3.5 KCl, 4.5 MgSO₄, 0.5 CaCl₂, 1.25 H₂PO₄, 25 NaHCO₃, 10 glucose, and 90 sucrose equilibrated with 95% O₂ / 5% CO₂ and stored in the same solution at 37°C for 30 minutes, then at room temperature until use. Recordings were made at 30-32°C (TC324-B; Warner Instrument Corp) in ACSF (in mM: 124 NaCl, 8.5 KCl, 1 NaH₂PO₄, 25 NaHCO₃, 20 glucose, 1 MgCl₂, 2 CaCl₂). Whole-cell voltage clamp recordings (-70 mV) were obtained using a patch pipette (4-6 M MΩ) containing (in mM): 135 K-Gluconate, 5 KCl, 0.1 EGTA-Na, 10 HEPES, 2 NaCl, 5 ATP, 0.4 GTP, 10 phosphocreatine (pH 7.2; 280-290 mOsm). Bicuculline (5 µM) was also included in the bath solution to inhibit GABA_{A}Rs. NBQX (20 µM) was added later in recordings to inhibit AMPAR synaptic currents. Patch pipettes were made from borosilicate glass (A-M Systems, Sequium, WA) using a micropipette puller (Model P-1000; Sutter Instruments). Recordings were made without correction for junction potentials. Pyramidal cells were visualized and targeted via infrared-differential interference contrast (IR-DIC; 40x objective) optics on an Axioskop-2 FS (Zeiss).

### RESULTS

### Chronic administration of RS-67,333 is prophylactic against stress in male mice

We have previously reported that chronic Flx administration (3 weeks of administration) is not prophylactic in 129S6/SvEv mice [3]. However, it remained to be determined if other serotonergic drugs could act as prophylactics. Here, we administered Flx (18 mg/kg/day) in the drinking water or RS-67,333 (1.5 mg/kg/day) in osmotic minipumps for 3 weeks prior to CORT administration in C57Bl/6NTac male mice followed by a series of behavioral assays, including the EPM, novelty-suppressed feeding (NSF), and sucrose splash test (ST) **(****Fig. 1A-1B****).** CORT increased body weight over the 6-week behavioral protocol, as previously observed [34], **(****Fig. 1C-1F****),** but this was attenuated by Flx and RS-67,333 administration.

In the EPM, CORT + Veh, CORT + Flx, and CORT + RS-67,333 administration did not alter the time spent in the open arms when compared with VEH + Veh administration **(****Fig. 1G****).** However, CORT + Veh mice exhibited a significantly decreased number of entries into the open arms of the EPM when compared with VEH + Veh mice **(****Fig. 1H****).** CORT + Flx and CORT + RS-67,333 mice had significantly more entries into the open arms of the EPM when compared with CORT + Veh mice. The total distance traveled in the EPM did not differ between any of the groups **(****Fig. 1I****)**.

Next, the NSF task was administered to assay anxiety-like behavior **(****Fig. 1J-1K****).** CORT + Veh mice exhibited an increased latency to approach the food pellet when compared with VEH + Veh mice. CORT + RS-67,333, but not CORT + Flx mice exhibited a significantly decreased latency to approach the pellet when compared with CORT + Veh mice.

Finally, in the ST, CORT + Veh mice exhibited decreased grooming duration when compared with VEH + Veh mice **(****Fig. 1L****).** CORT + RS-67,333, but not CORT + Flx mice exhibited increased grooming duration when compared with CORT + Veh mice. These data suggest that chronic RS-67,333, but not chronic Flx administration is prophylactic against a wide range of CORT-induced behavioral abnormalities.

### A single injection of RS-67,333 attenuates learned fear and protects against stress-induced hypophagia in male mice

Previously, we have shown that a single injection of (R,S)-ketamine is prophylactic against stress-induced depressive-like behavior and attenuates learned fear in 129S6/SvEv mice [3]. Here, we sought to determine if a single injection of RS-67,333 could also prevent a variety of maladaptive behaviors following a single, acute stressor. Male 129S6/SvEv mice were injected with saline or RS-67,333 (1.5, 10, or 30 mg/kg) **(****Fig. 2A****).** One week later, mice were administered 3-shock CFC. Mice administered 30, but not 1.5 or 10 mg/kg, of RS-67,333 exhibited significantly less freezing during CFC training when compared with mice administered saline **(****Fig. 2B****).** Five days later, mice were re-exposed to the training context. Mice administered 1.5 or 10, but not 30 mg/kg of RS-67,333 exhibited significantly less freezing when compared with mice administered saline **(****Fig. 2C-2D****).**

Following CFC, mice were administered the FST. On Day 1, mice administered 10, but not 1.5 or 30 mg/kg, of RS-67,333 were significantly less immobile when compared with saline mice **(****Fig. 2E****).** However, on Day 2, immobility time was comparable between all groups **(****Fig. 2F-2G****).**

Next, mice administered saline or RS-67,333 (10 mg/kg) were tested in the OF. Both groups of mice travelled a comparable distance **(****Fig. 2H****)** and spent a comparable amount of time in the center of the arena **(****Fig. 2I****)**. Subsequently, mice were tested in the EPM, and neither in the open arms nor entries into the open arms of the maze was significantly different between saline or RS-67,333 mice **(****Fig. 2J-2K****).**

Finally, mice were administered the NSF. Mice given prophylactic RS-67,333 (10 mg/kg) exhibited a significantly reduced latency to approach the pellet **(****Fig. 2L-2M****).** However, neither food eaten in the home cage nor weight loss following food deprivation differed between the groups **(****Fig. 2N-2O****)**. Together, these data indicate that a single injection of RS-67,333 is effective as a prophylactic in attenuating learned fear and preventing stress-induced hypophagia, but not depressive-like behavior, as measured by the FST, in male 129S6/SvEv mice.

### A single prophylactic injection of RS-67,333 protects against stress-induced anxiety-like behavior in female mice

We next sought to determine if a single injection of RS-67,333 could also be prophylactic in female mice. Female 129S6/SvEv mice were injected with saline or RS-67,333 (1.5 or 10 mg/kg) **(****Fig. 3A****).** One week later, mice were administered 3-shock CFC. All groups of mice exhibited comparable levels of freezing during CFC training **(****Fig. 3B****).** Five days later, mice were re-exposed to the training context. Again, all groups of mice exhibited comparable levels of freezing **(****Fig. 3C-3D****).** Following CFC, mice were administered the FST. During days 1 **(****Fig. 3E****)** and 2 **(****Fig. 3F-3G****)** of the FST, all groups of mice had comparable levels of immobility.

Next, mice were tested in the OF and the EPM. Mice in all groups travelled comparable distances in the OF and spent a comparable amount of time in the center of the arena **(****Fig. 3H-****3I).** Similarly, in the EPM, mice spent a comparable amount of time in the open arms of the maze **(****Fig. 3J****)** and had a comparable number of entries into the open arms **(****Fig. 3K****).**

Finally, mice were assayed in the NSF paradigm. Prophylactic RS-67,333 (10 mg/kg), but not RS-67,333 (1.5 mg/kg), significantly reduced latency to feed **(****Fig. 3L-3M****).** Neither food eaten in the home cage nor weight loss following food deprivation differed between the groups **(****Fig. 3N-3O****)**. Together, these data indicate that RS-67,333 does not attenuate learned fear or protect against stress-induced depressive-like behavior, but may prevent stress-induced hypophagia in the NSF in female 129S6/SvEv mice.

### A single prophylactic injection of prucalopride or PF-04995274 is prophylactic against stress in male mice

We next sought to determine if other 5-HT₄R agonists could also be prophylactic in male 129S6/SvEv mice. Male 129S6/SvEv mice were injected with saline, (*R,S*)-ketamine (30 mg/kg), prucalopride (3 or 10 mg/kg), or PF-04995274 (3 or 10 mg/kg) **(****Fig. 4A****).** One week later, mice were administered 3-shock CFC. All groups of mice exhibited comparable levels of freezing during CFC training **(****Fig. 4B****).** Five days later, mice were re-exposed to the training context. As we have previously published, (*R,S*)-ketamine attenuated learned fear **(****Fig. 4C-****4D).** Interestingly, prucalopride at 3 mg/kg, but not 10 mg/kg, and PF04995274 at 10 mg/kg, but not 3 mg/kg, attenuated learned fear when compared with saline administration.

Following CFC, mice were administered the FST. During day 1, all groups of mice had comparable levels of immobility **(****Fig. 4E****).** During day 2, (*R,S*)-ketamine administration decreased immobility time when compared with saline administration **(****Fig. 4F-4G****).** Moreover, prucalopride at 3 mg/kg, but not 10 mg/kg, and PF04995274 at 10 mg/kg, but not 3 mg/kg, decreased immobility time when compared with saline administration.

Stress-induced anxiety-like behavior was next quantified. In the OF, all groups of mice traveled a comparable distance **(****Fig. 4H****).** In the EPM, all groups of mice spent comparable time in the open arms **(****Fig. 4I****)** and entered into the open arms a comparable number of times **(****Fig. 4J****).** In the NSF paradigm, all groups of mice approached the pellet in a comparable amount of time **(****Fig. 4K-4L****).** Finally, all mice lost a comparable amount of weight during the NSF paradigm **(****Fig. 4M****).** In summary, these data indicate that a single injection of prucalopride or PF0499574 results in prophylactic efficacy by attenuating learned fear and decreasing stress-induced depressive-like behavior. However, these drugs are not prophylactic against stress-induced anxiety-like behavior.

### (R,S)-ketamine and prucalopride exhibit a common mechanism by reducing bursts of large AMPA receptor-driven synaptic currents in CA3

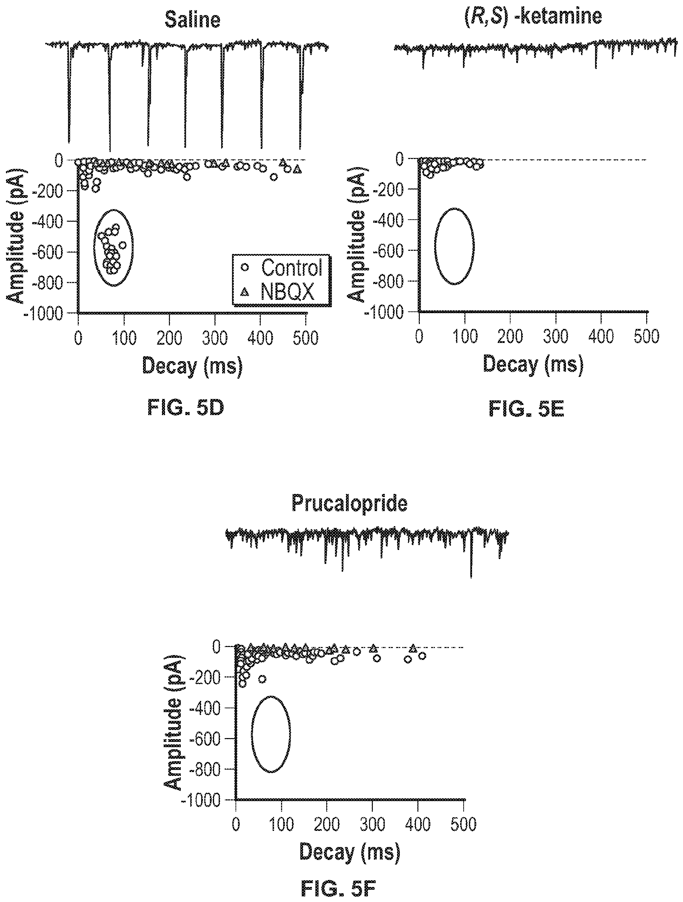

We next sought to elucidate potential common mechanisms between (R,S)-ketamine and a 5HT₄R agonist such as prucalopride. Specifically, we hypothesized that there may be similarities between the effects of (*R*,*S*)-ketamine and prucalopride on glutamatergic transmission in CA3 since we previously reported that prophylactic (*R*,*S*)-ketamine alters activity in ventral CA3 (vCA3), but not in the DG [6]. To test this, mice were injected with saline, (*R,S*)-ketamine (30 mg/kg), or prucalopride (3 mg/kg) and were euthanized 1 week later **(****Fig. 5A****).** We performed whole-cell voltage clamp recordings of spontaneous excitatory postsynaptic currents (EPSCs) in CA3 pyramidal cells. We found there were no differences in the average EPSC amplitude **(****Fig. 5B****)** or the number of EPSCs **(****Fig. 5C****)** between the groups. However, we did find that saline-treated mice typically displayed large bursts of EPSCs (-590.8±13.85 pA), which were completely blocked by the AMPA receptor blocker NBQX **(****Fig. 5D****)**. These large AMPA receptor-mediated signals were not present in either (*R*,*S*)-ketamine- **(****Fig. 5E****)** or prucalopride-treated mice **(****Fig. 5F****),** suggesting that although these drugs target different receptors, they both alter AMPA-mediated synaptic transmission in a similar manner.

### DISCUSSION

Here, we hypothesized that 5-HT₄R agonists could be prophylactic against fear, depressive-like, and/or anxiety-like behavior. We tested if three 5-HT₄R agonists with varying affinity could protect against stress. Chronic administration of RS-67,333 was prophylactic against CORT stress. A single injection of RS-67,333 attenuated learned fear in male, but not female, 129S6/SvEv mice, and prevented stress-induced hypophagia in the NSF in both sexes. Acute administration of RS-67,333 was ineffective against stress-induced depressive-like behavior. A single injection of either prucalopride or PF-04995274 attenuated learned fear and decreased depressive-like behavior but had no effect on anxiety-like behavior. Moreover, a single injection of (*R*,*S*)-ketamine or prucalopride reduced large, spontaneous AMPA receptor-driven bursts in CA3, indicating a common mechanism by which either drug may protect against stress-induced maladaptive behavior.

The 5-HT₄R is widely distributed throughout the brain and heavily expressed in areas related to emotional regulation and cognitive function. The 5-HT₄R is also heavily expressed throughout the periphery and plays a crucial role in regulating ENS activity and function.

The three 5-HT₄R agonists chosen in this study have differential affinity to the 5-HT₄R (Table 1). RS-67,333, Prucalopride, and PF-04995274 have varying selectivity and affinity for the 5-HT₄R. These differences may contribute to the drugs' prophylactic efficacy in preventing fear, depressive-like, or anxiety-like behavior following stress. RS-67,333 and PF-04995274 are high-affinity 5-HT₄R partial agonists, whereas prucalopride is a selective, high-affinity 5-HT₄R agonist. RS-67,333 attenuated learned fear and protected against novelty-induced hypophagia, but did not decrease stress-induced depressive-like behavior. Prucalopride and PF-04995274 attenuated learned fear and decreased depressive-like behavior but had no effect on various measures of anxiety-like behavior. These data suggest that the unique combination of high pKᵢ and partial selectivity for the 5-HT₄R exhibited by RS-67,333 is sufficient to prevent against anxiety-like behavior whereas the differential activity of prucalopride and PF-04995274 at the 5-HT₄R protect against stress-induced depressive-like behavior. Further study is necessary to determine if and how the 5-HT₄R may contribute to the neurobiological mechanisms underlying stress resilience.

**Table 1. Summary of behavioral results in male mice**

| **Activity of 5HT₄R agonists** | | | | | | | |
|---|---|---|---|---|---|---|---|
| Drug | Selectivity | Affinity (pKi) | Behavioral efficacy | | | | |
| | | | Fear | Depressive-like | Anxiety-like | Dose-specific? | Activity at other receptors? |
| RS-67,333 | partial | 8.7 | + | - | + | Y | 5HT_{1a, 1d}, _{2a, 2c,} D1, D2 and M1-3 |
| Prucalopride | selective | 8.1-8.6 | + | + | - | Y | >290-fold selectivity for 5HT₄R |
| PF-04995274 | partial | 0.34-0.82 | + | + | - | Y | (data not available) |

The expression and activity of 5-HT₄Rs within the central nervous system (CNS) and periphery may provide insight into these mechanisms. In the brain, 5-HT₄Rs are expressed in areas of the brain involved in processing emotion, including the HPC, AMG, and PFC [11,16,21,35,36]. In addition to a multitude of other functions, such as modulating dopamine and acetylcholine release [36], as well as facilitating synaptic plasticity [36], 5-HT₄Rs are known to interact with the calcium effector protein p11 [37]. 5-HT₄Rs are highly co-expressed with p11, which increases surface expression of the receptor in the HPC and AMG, facilitates its downstream signaling pathways, and is necessary for the antidepressant effects of 5-HT₄R stimulation [37,38]. Levels of p11 are correlated with measures of suicidality and PTSD, indicating its potential as a biomarker for suicidal ideation and PTSD [39-41]. Additionally, 5-HT₄R expression and activity in the PFC is regulated by casein kinase 2 (CK2), which may be an important modulator of depressive- and anxiety-like behaviors [42]. Further studies examining 5-HT₄R agonists and their effects on these cellular regulators of 5-HT₄R expression and activity could yield further insight into prophylactic efficacy.

Because all three 5-HT₄R agonists exhibited prophylactic properties similar to (*R,S*)-ketamine, we investigated whether these compounds had comparable effects on neural activity in CA3. We found that a single injection of (*R,S*)-ketamine or prucalopride eliminated large bursts of AMPA receptor-mediated synaptic currents typically seen in saline controls without significantly altering the overall amplitude or number of EPSCs. Therefore, although these compounds target distinct receptors, they may achieve similar behavioral effects by altering AMPA receptor-dependent glutamatergic transmission in a convergent manner. Although (*R*,*S*)-ketamine is known to inhibit NMDA receptors [43-45], emerging evidence indicates that (*R*,*S*)-ketamine may also act on AMPA receptors to exert its *antidepressant* effects [46,47]. Our results are congruent with these data and suggest that (*R,S*)-ketamine's actions on AMPA receptor-mediated glutamatergic activity may contribute to the compound's *prophylactic* effects. Additionally, previous studies show that pharmacological activation of 5-HT₄Rs results in the long-term potentiation (LTP) of CA3-CA1 synapses along the Schaffer collaterals [48]. In combination with these data, our results suggest that (*R,S*)-ketamine and 5-HT₄R agonists, by attenuating large, spontaneous AMPA receptor-driven synaptic events in the CA3 autoassociative network, may reduce overall noise in the hippocampal circuit which may allow for a greater signal-to-noise ratio of relevant stimuli [49]. However, further research is necessary to confirm this hypothesis and to examine whether this potential mechanism directly contributes to enhanced resilience.

In addition to the actions of 5-HT₄R agonists within the brain, it is likely that these compounds exert additional changes within the periphery. 5-HT₄Rs are expressed in the periphery, such as the enteric nervous system (ENS), adrenal glands, and heart [17]. Importantly, 5-HT₄Rs play a major role in maintaining communication along the gut-brain axis. Recent data indicate that microbiota in the ENS communicate with the CNS by stimulating 5-HT₄Rs present throughout the gut to stimulate serotonin release in the brain [50]. Concurrently, numerous previous studies have shown that activation of 5-HT₄Rs is neuroprotective against oxidative stress, reduces inflammation, and stimulates neurogenesis in the brain and ENS [50-52]. Our manipulations may have stimulated gut-brain communication to promote neuroprotection and neurogenesis and thereby, enhance resilience against stress. We hypothesize that this action may have had an additive effect on the numerous, well-characterized consequences of 5-HT₄R stimulation in the brain, such as increasing neuronal firing in the medial PFC (mPFC) and enhancing mitogenesis in the HPC [19,35], although this remains to be determined.

To develop safe and efficacious pharmacological methods of enhancing stress resilience it will be necessary to determine the toxicity of 5-HT₄R agonists. Because 5-HT₄Rs are so widely expressed throughout the periphery, chronic exposure to these drugs could result in negative outcomes [17]. We found that chronic administration of RS-67,333 did not result in adverse side effects. However, because we did not conduct additional assays, such as assessing changes in cardiovascular activity or liver toxicity, it is impossible to know if chronic 5-HT₄R administration would negatively impact peripheral organs. Nonetheless, the drugs that we tested were efficacious in enhancing stress resilience even after a single dose, obviating chronic administration.

Here, we utilized two strains - C57BL/6NTac and 129S6/SvEv - in order to validate our effects of RS,67-333 in both a neuroendocrine model of stress and a fear-based stressor. In the C57BL/6NTac mice, we found prophylactic RS-67,333 was effective at decreasing depressive- and anxiety-like behavior, whereas in the 129S6/SvEv mice, we found prophylactic RS-67,333 was effective at attenuating learned fear and preventing anxiety-like behavior in the NSF, but not decreasing depressive-like behavior.

Previous research examining 5-HT₄R agonists as rapid-acting *antidepressants* have exclusively used male subjects [19,20,54]. However, previous studies indicate that *antidepressant* (*R*,*S*)-ketamine exhibits sex-specific behavioral and neurobiological effects. Across the estrous cycle, the efficacy of *antidepressant* (*R,S*)-ketamine varies in female mice, and this variability may be attributed to changing levels of neurotrophic factors (e.g. BDNF) or changes in NMDA receptor activity across the estrous cycle [55,56]. Additionally, acute (*R*,*S*)-ketamine administration may lead to a sustained increase in GluR1 and GluR2 AMPA receptor subunits in the mPFC and HPC of male, but not female mice [45,46,57,58]. Despite numerous studies showing *prophylactic* efficacy in male rodents, only one study to date has examined female rodents [59]. Maier and colleagues showed that *prophylactic* (*R*,*S*)-ketamine reduced stress-induced activation of the dorsal raphe nucleus (DRN) and eliminated DRN-dependent social exploration deficits in female rats. However, this study did not measure fear, depressive-like, and anxiety-like behavior as done here. Nonetheless, we show that RS-67,333 does not attenuate learned fear or prevent depressive-like behavior but does protect against stress-induced anxiety-like behavior in female 129S6/SvEv mice. Thus, our data indicate that 5-HT₄R agonists may exclusively target the neural circuits underlying anxiety-like, but not depressive- or fear-related, behaviors in female mice. We did not utilize female C57BL/6NTac mice, as a previous study of our own has shown that female C57BL/6NTac mice are insensitive to chronic CORT [60].

Overall, the present study has identified three novel compounds to be effective prophylactics against two types of stress and in both sexes. These data suggest that the 5-HT₄R can be a novel target for prophylactic development and future studies can be directed to how 5-HT₄R agonists administered prior to a stressor result in stress resiliency.

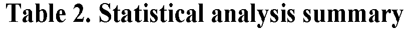

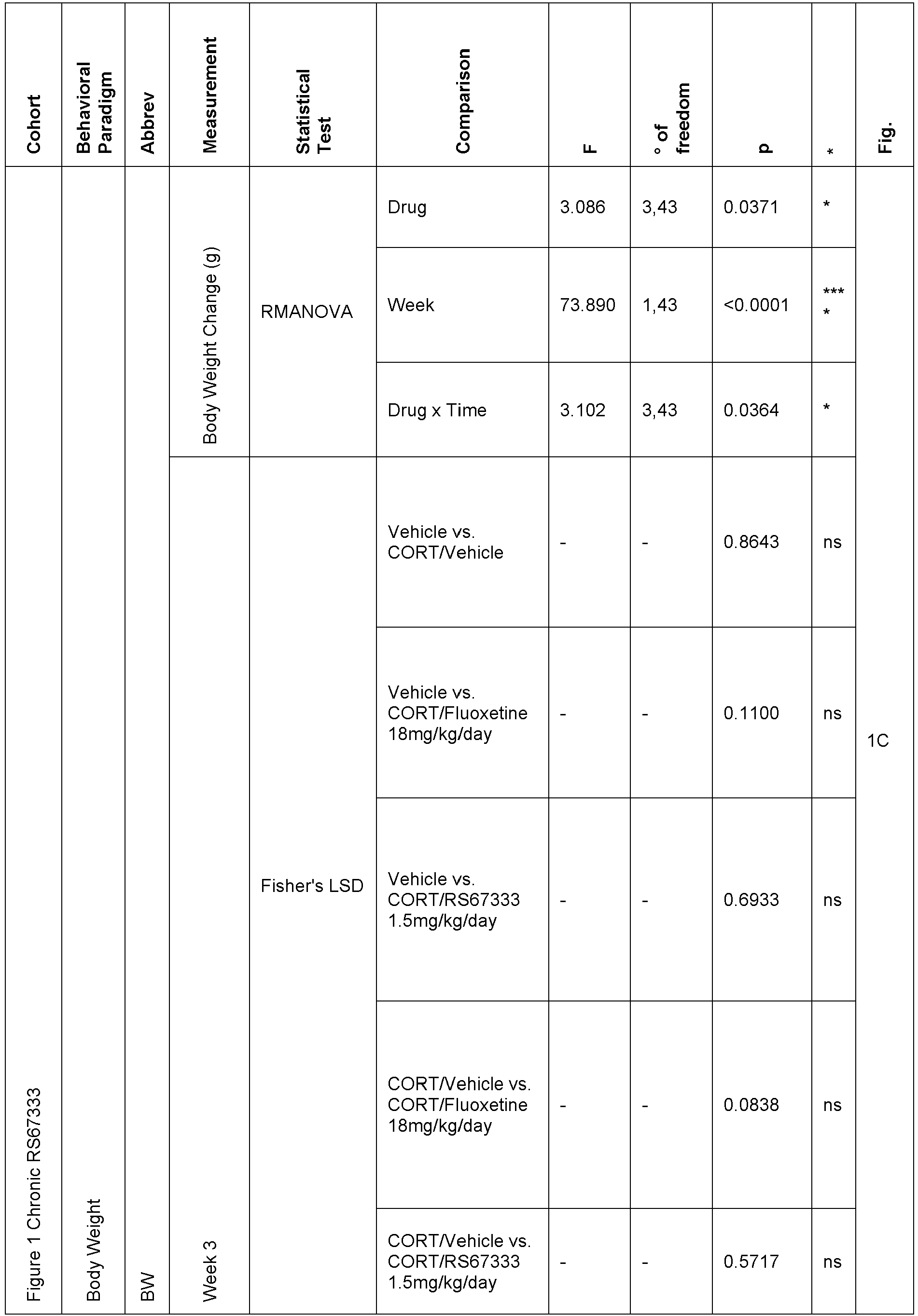

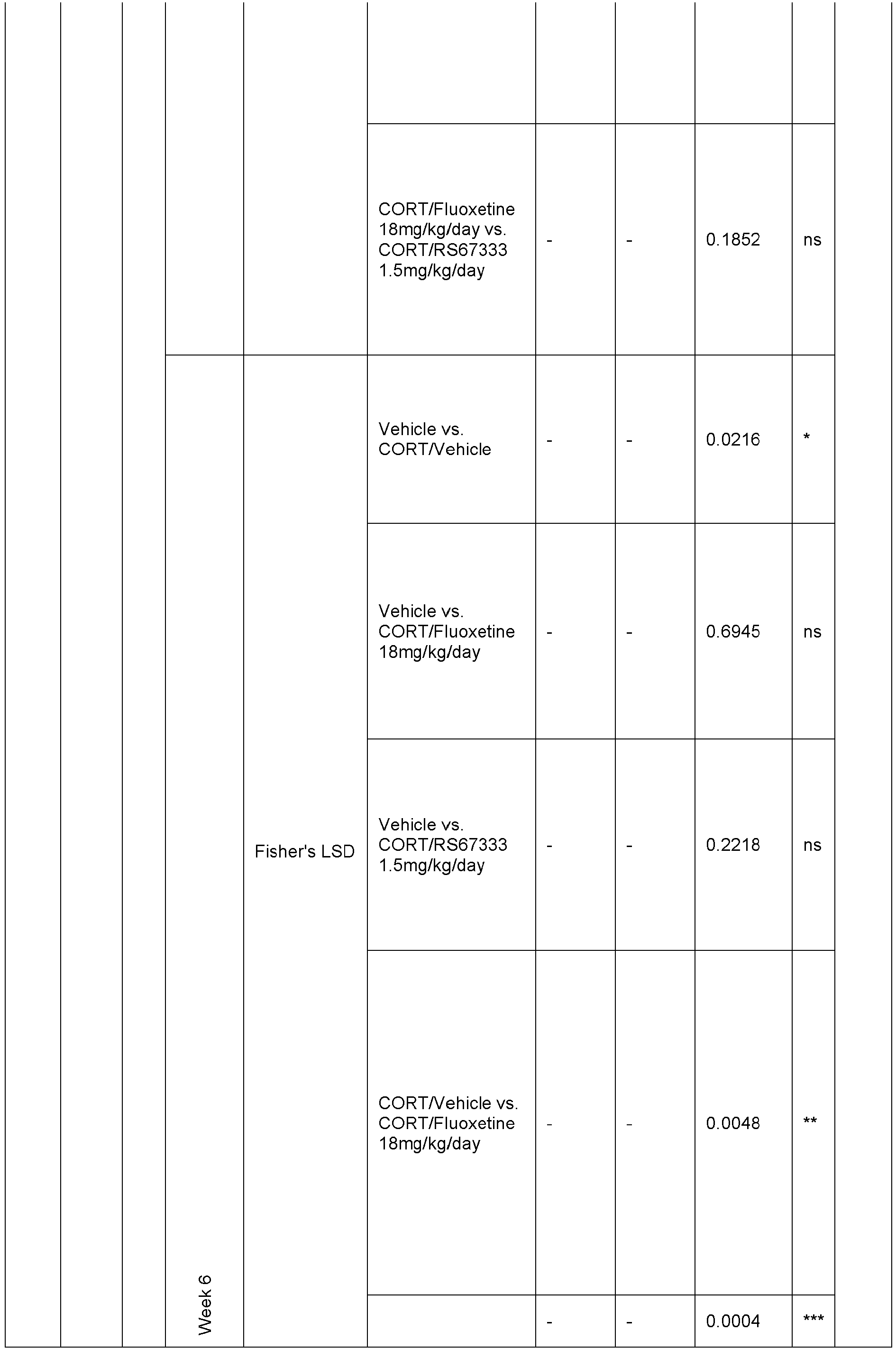

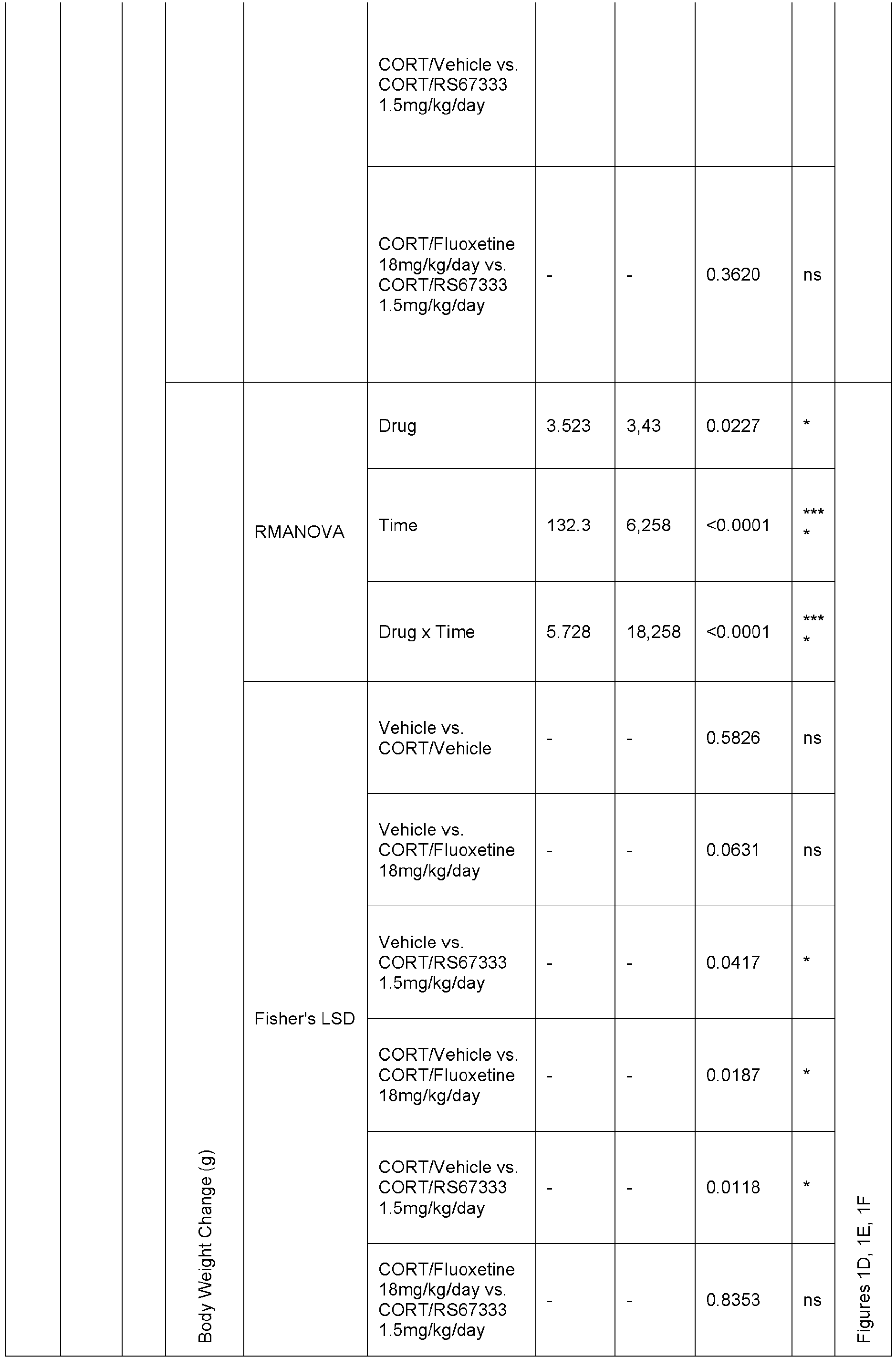

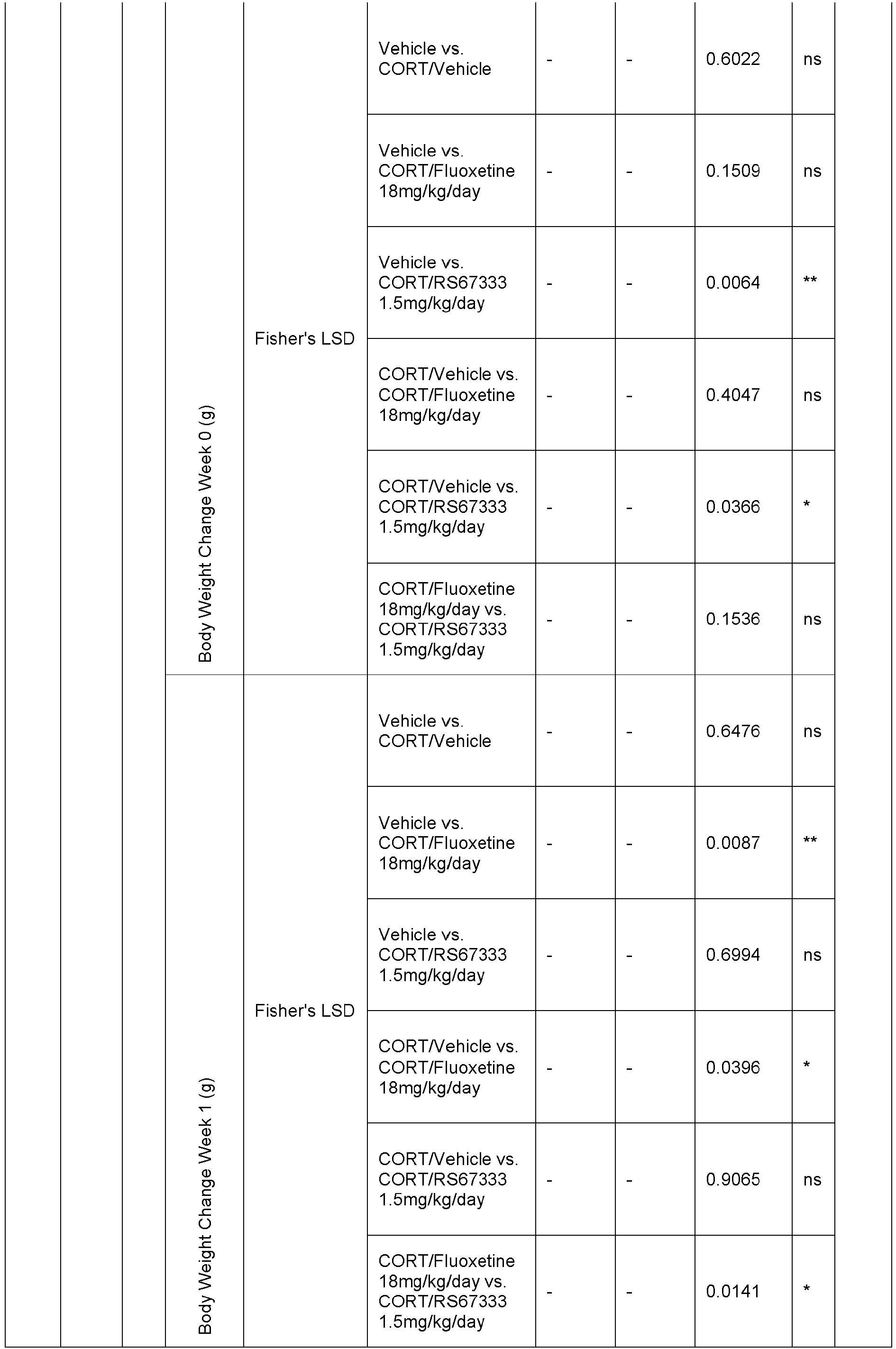

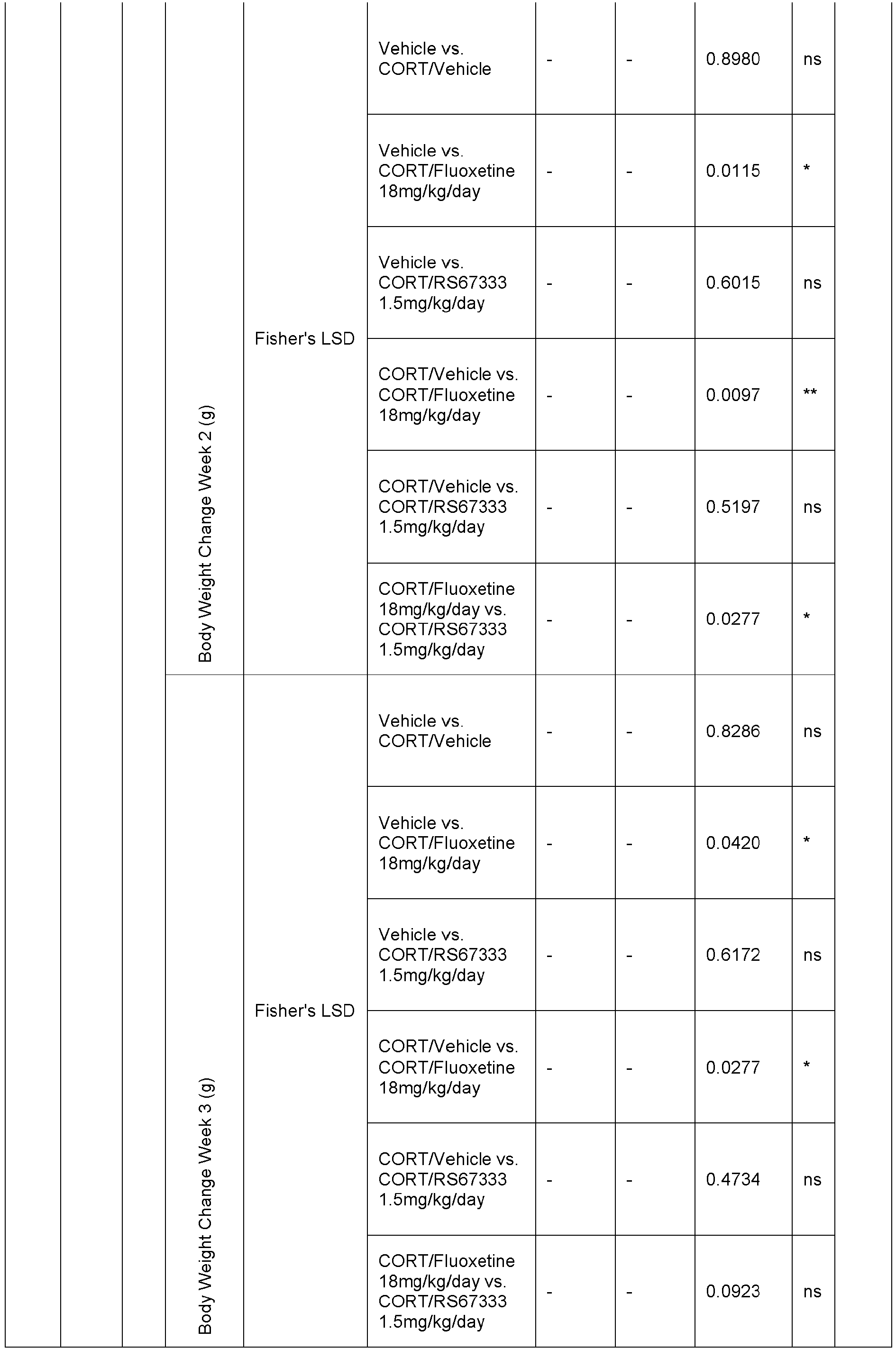

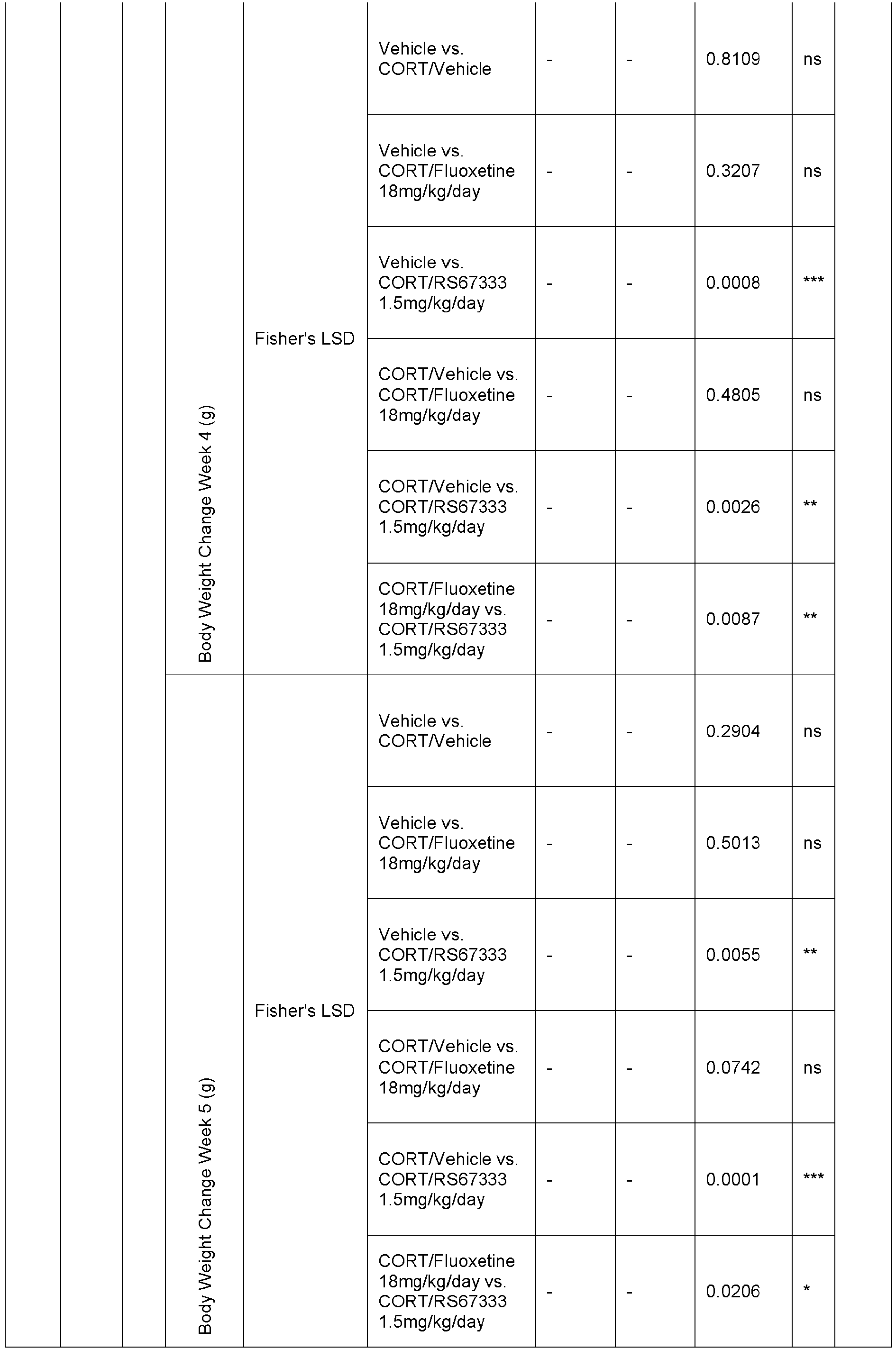

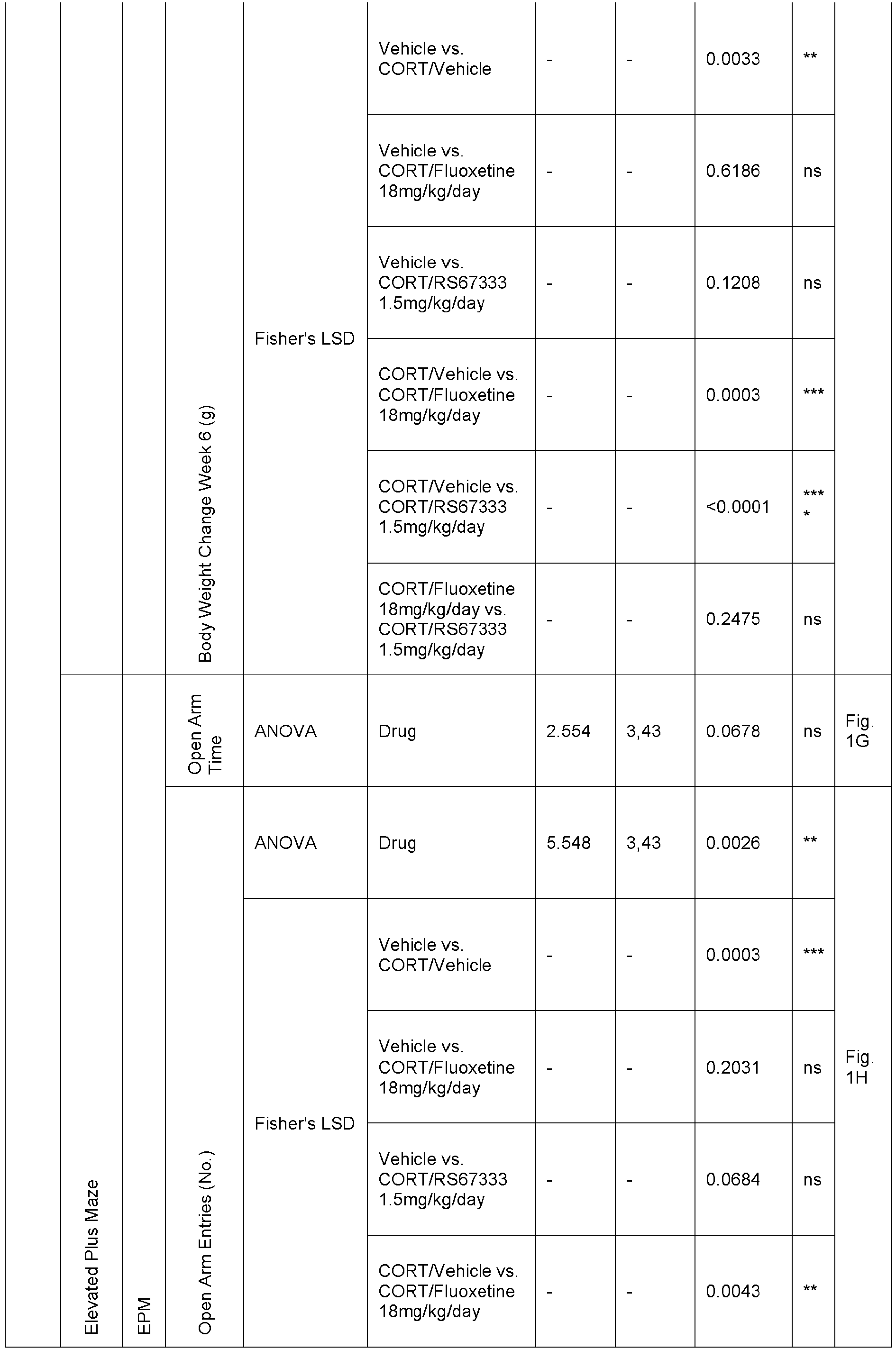

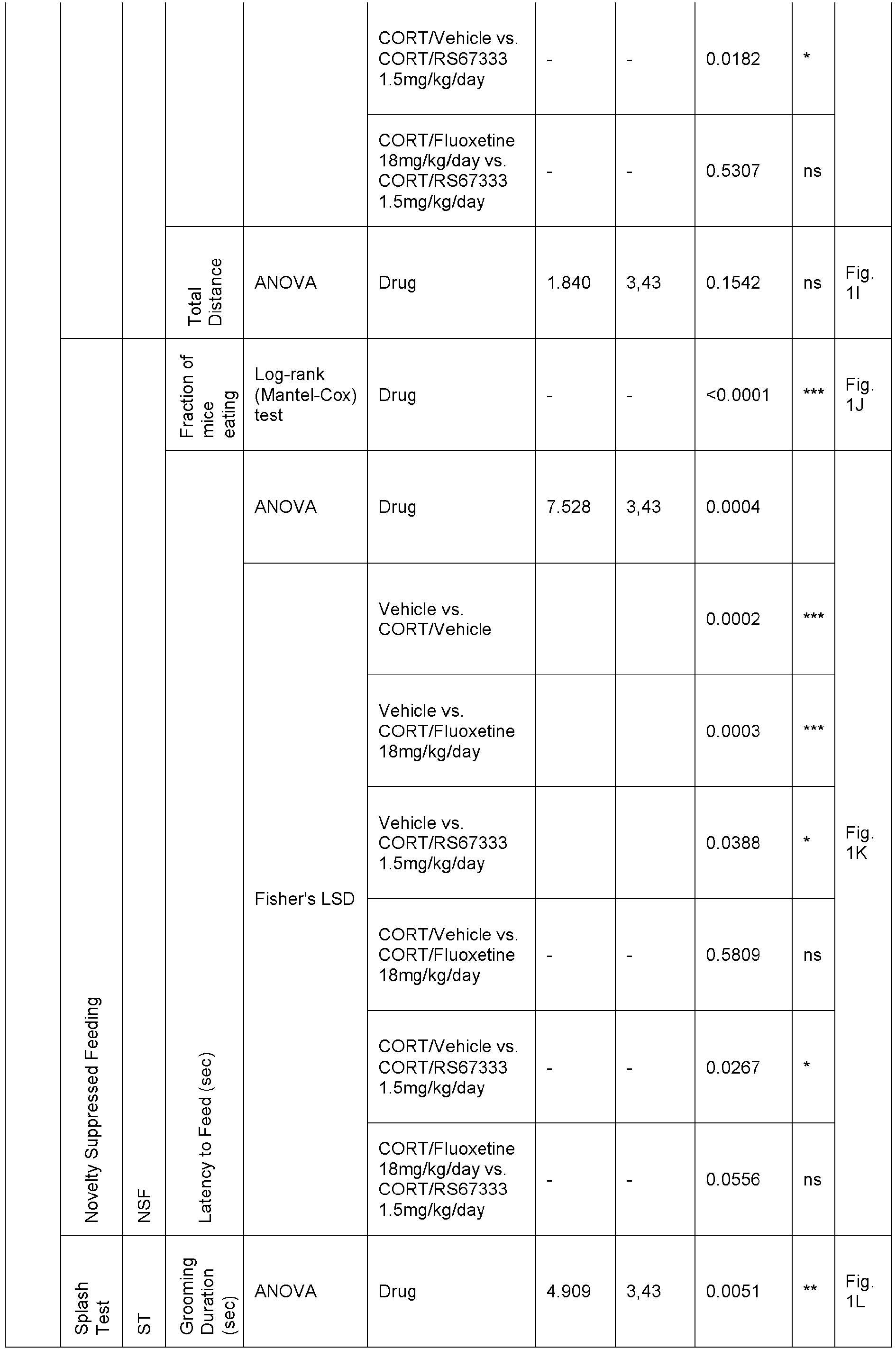

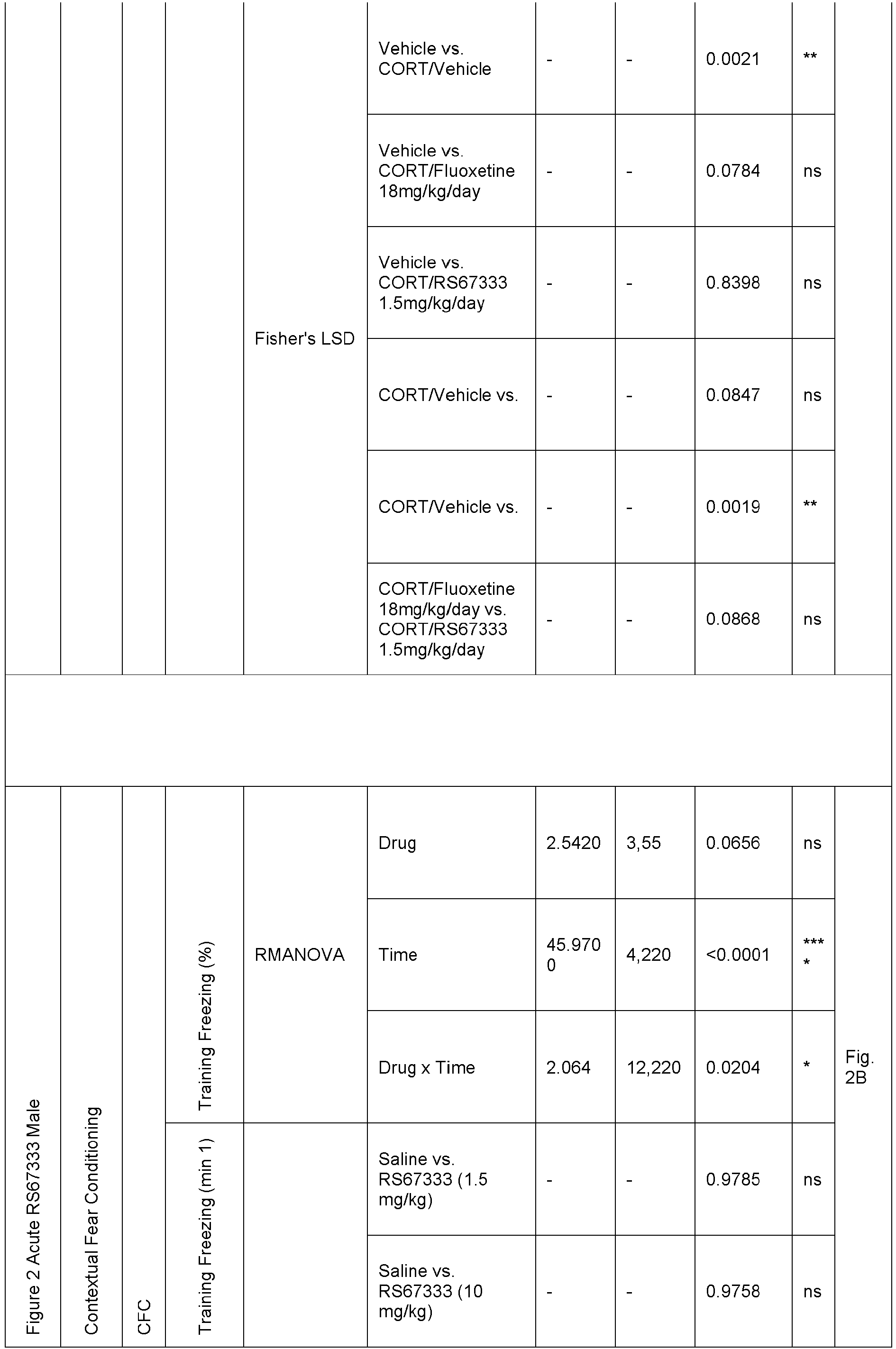

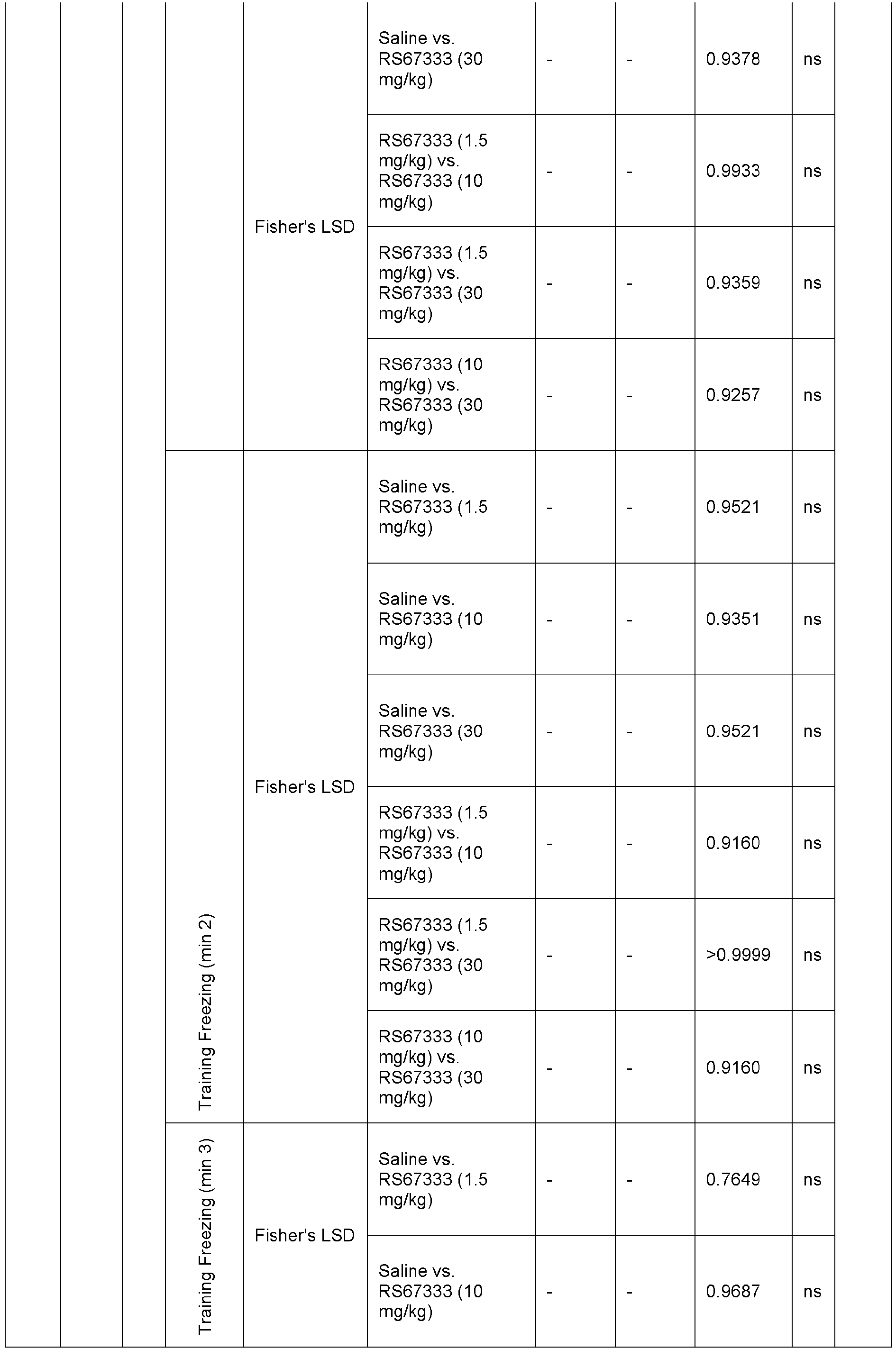

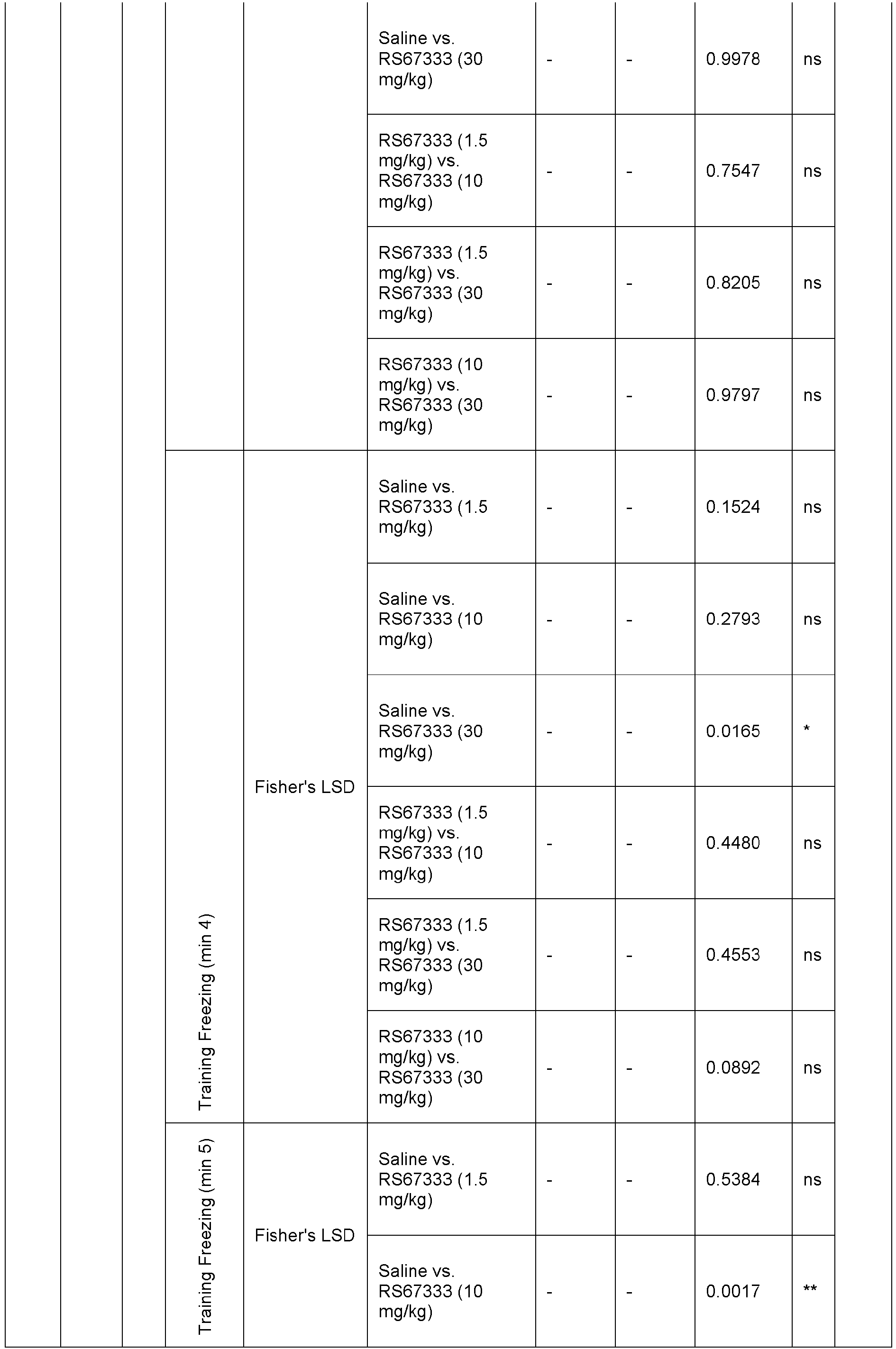

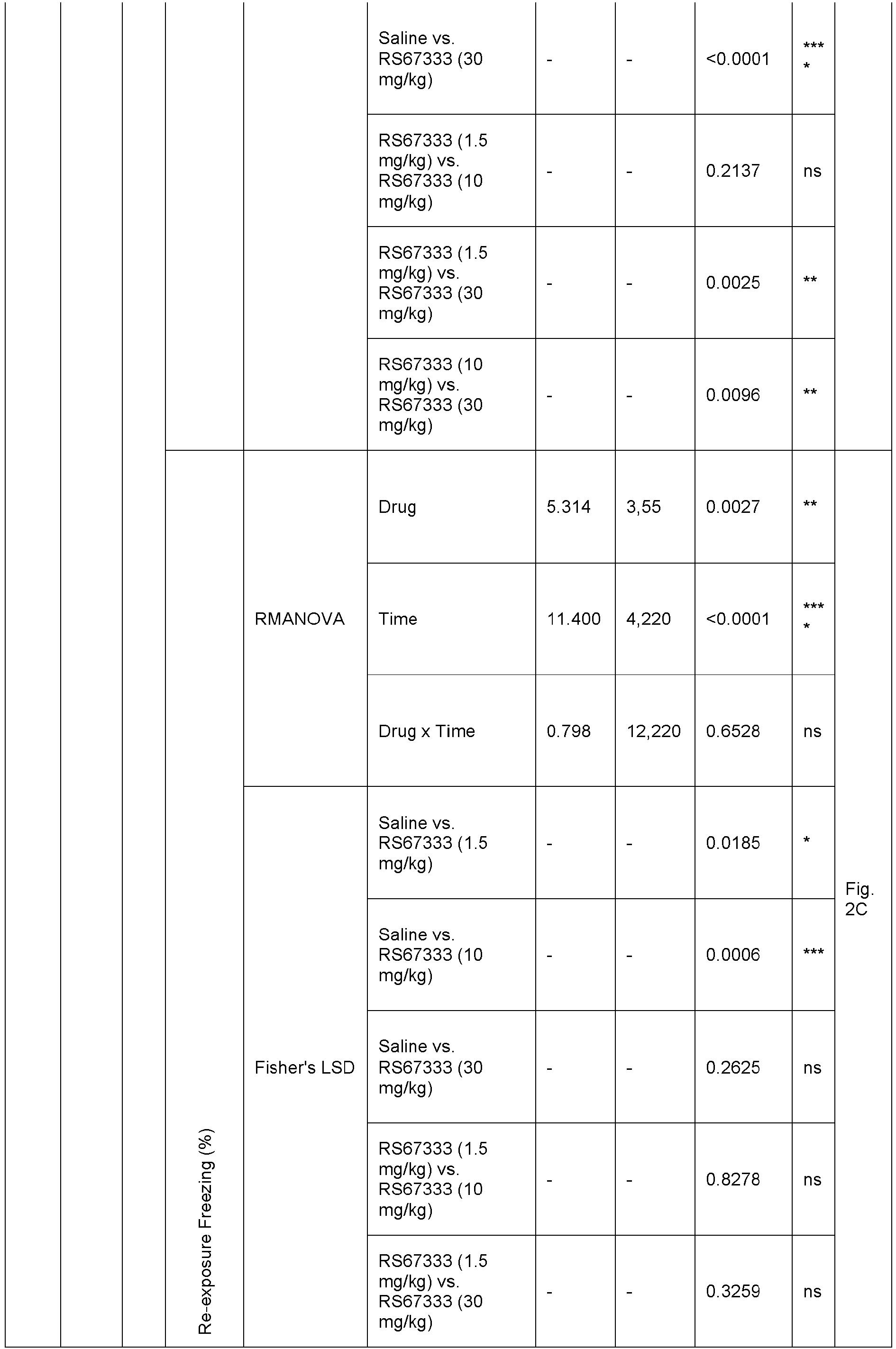

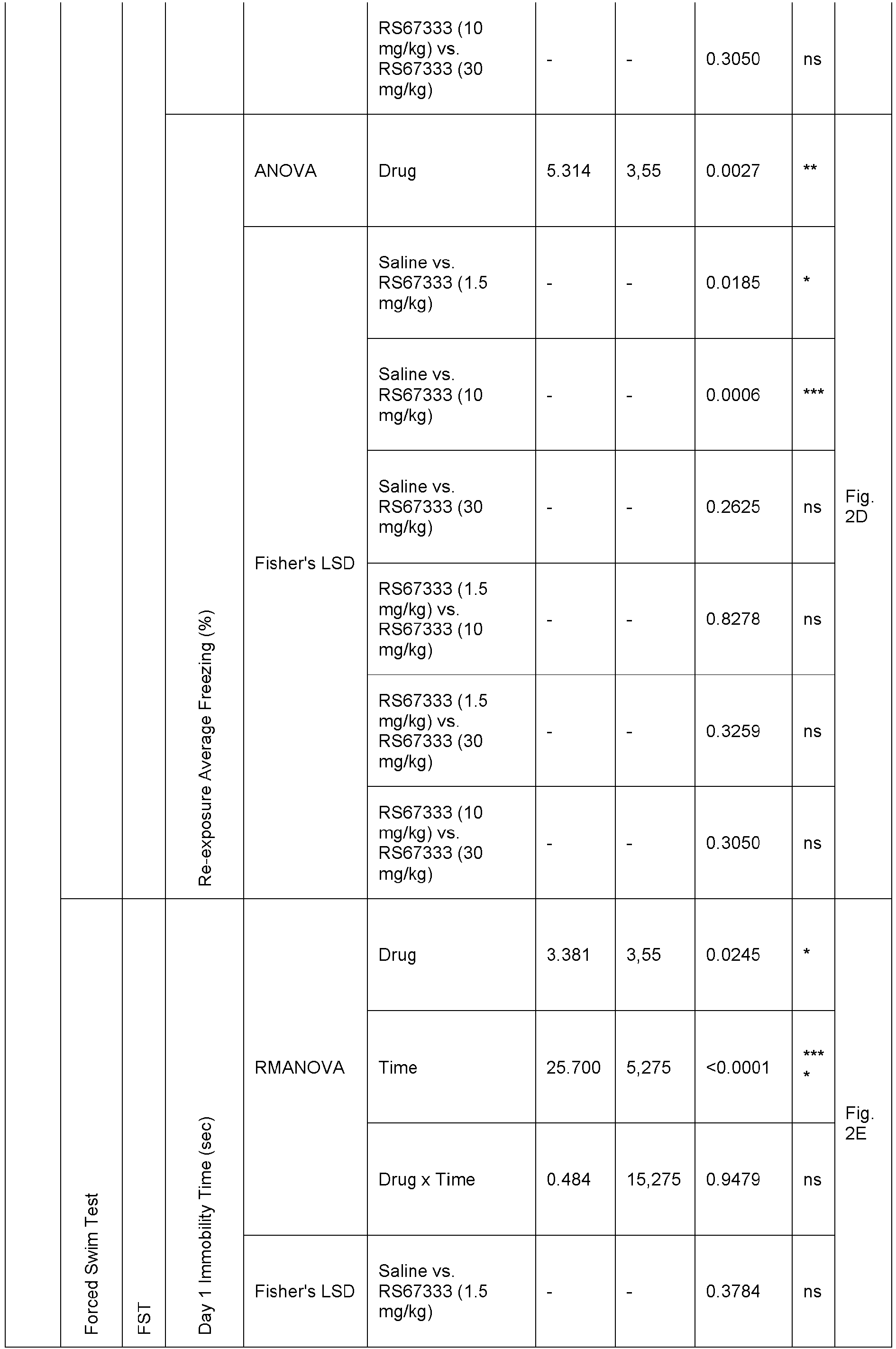

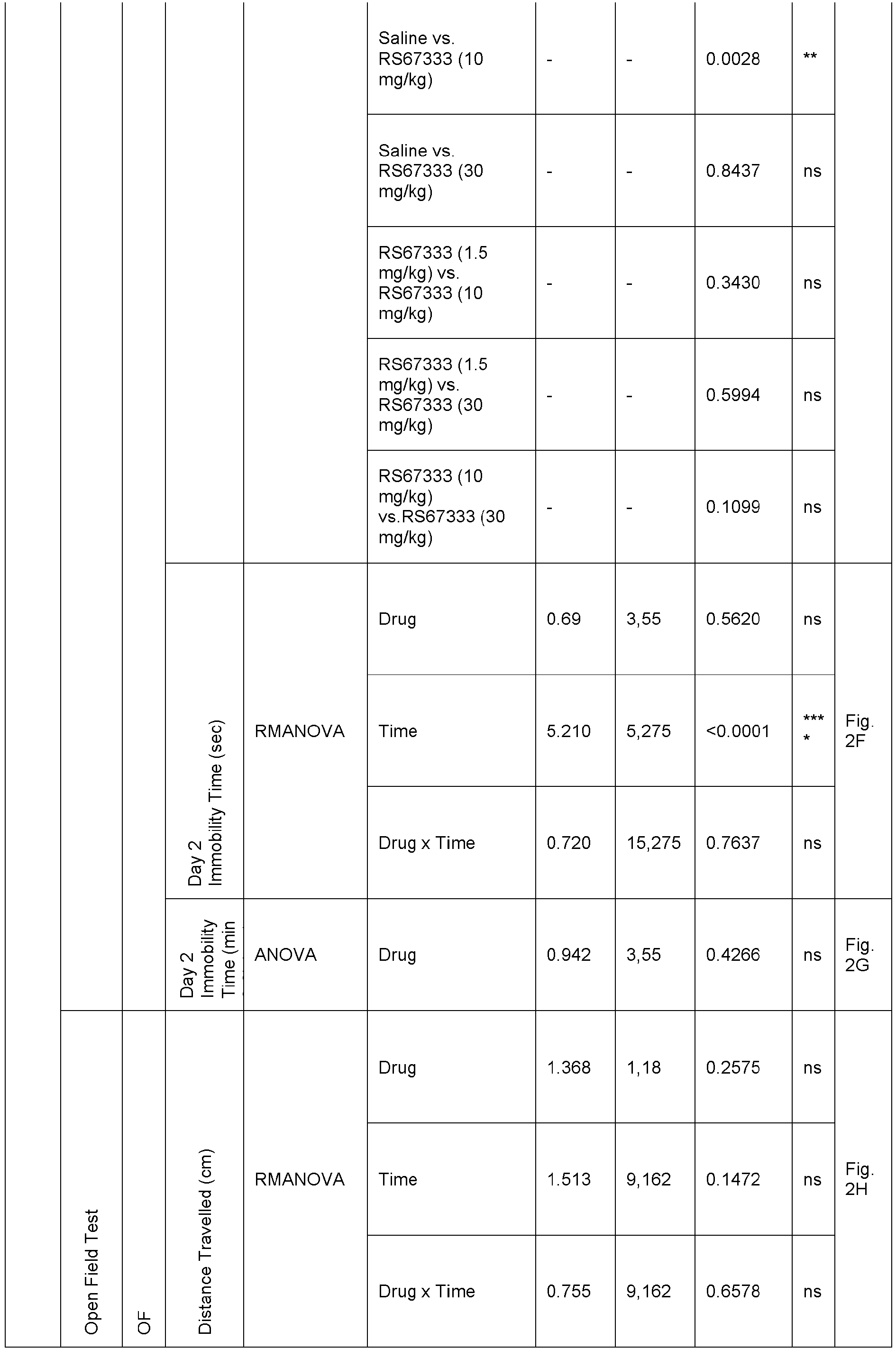

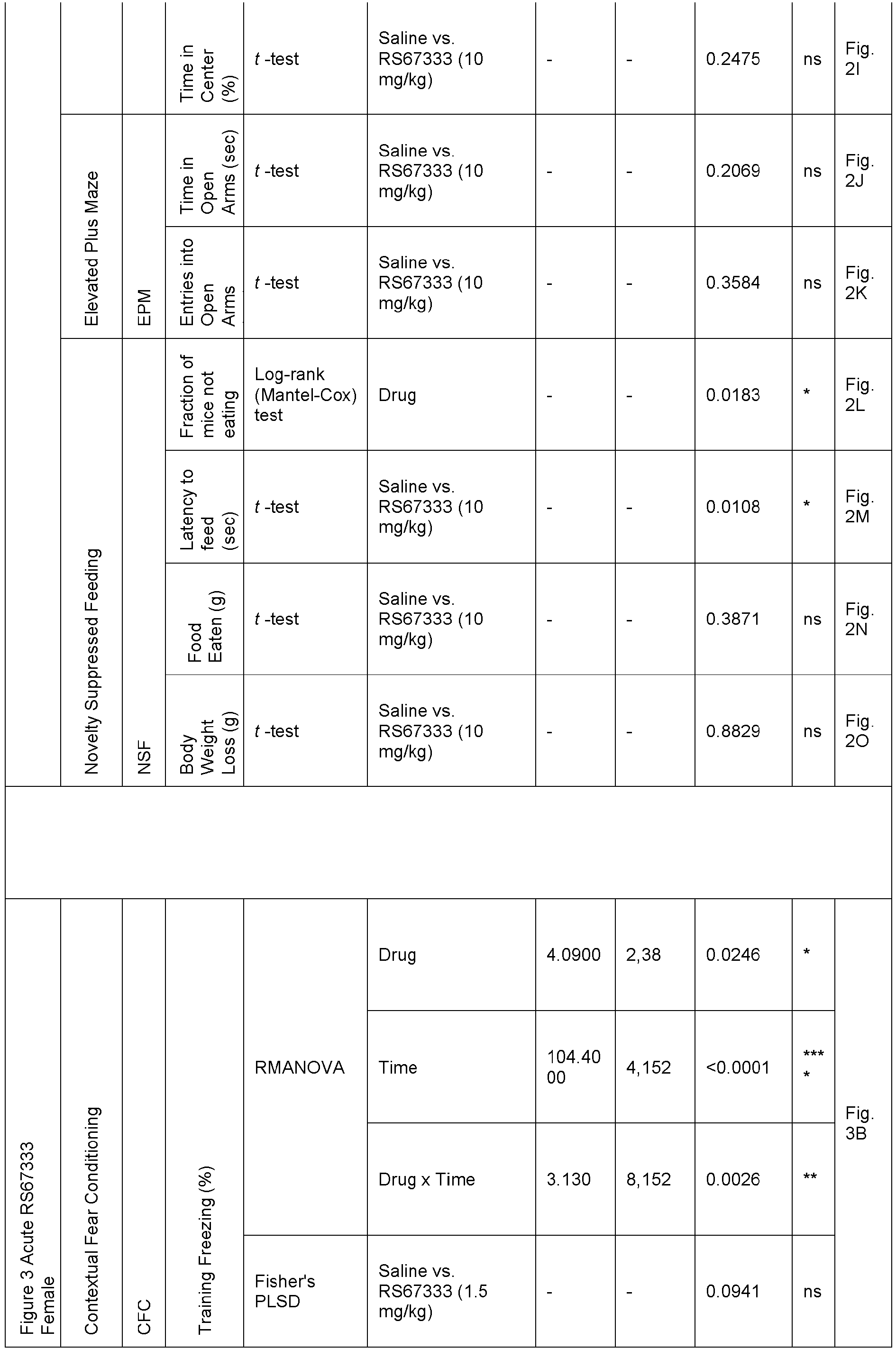

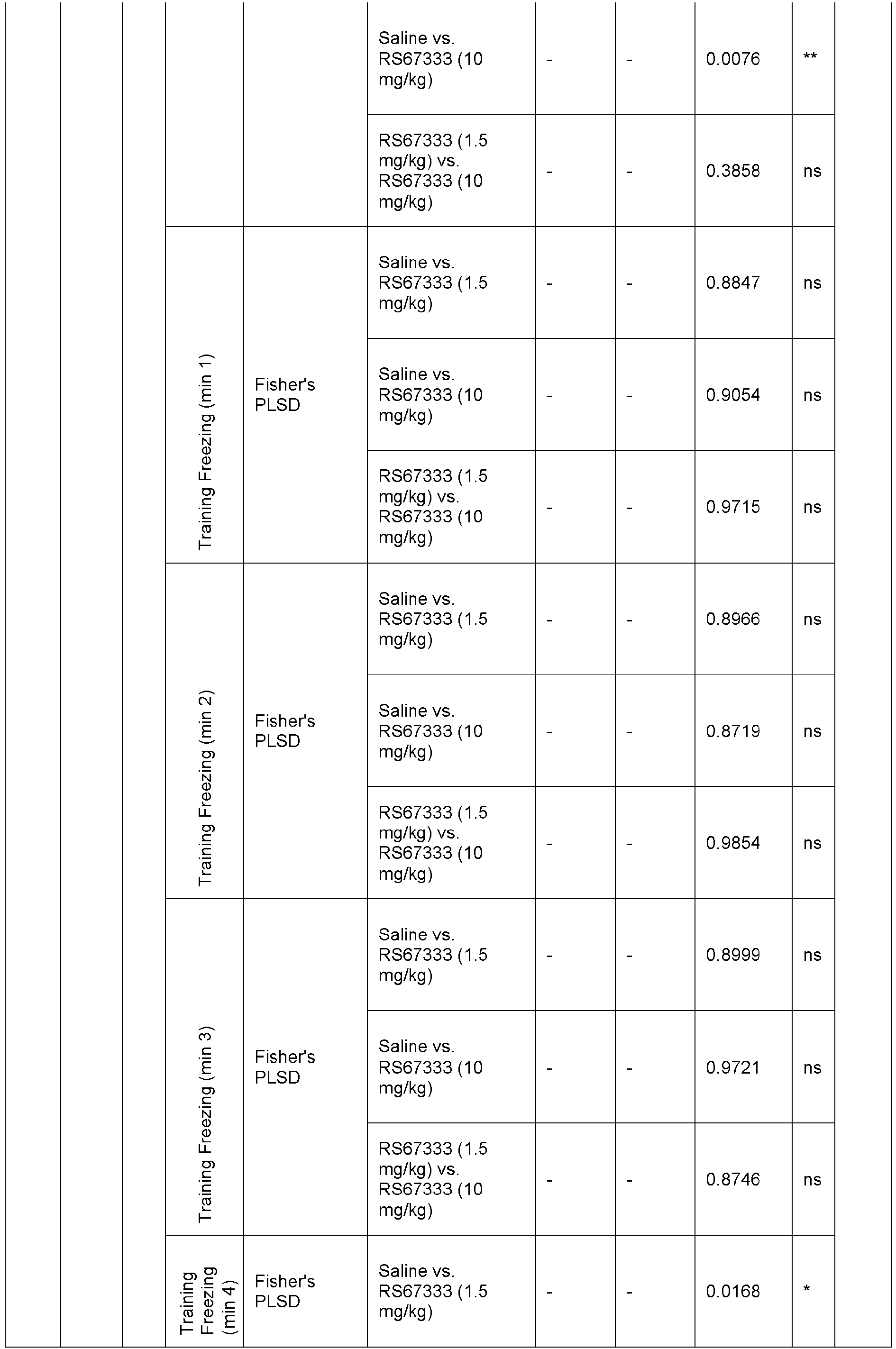

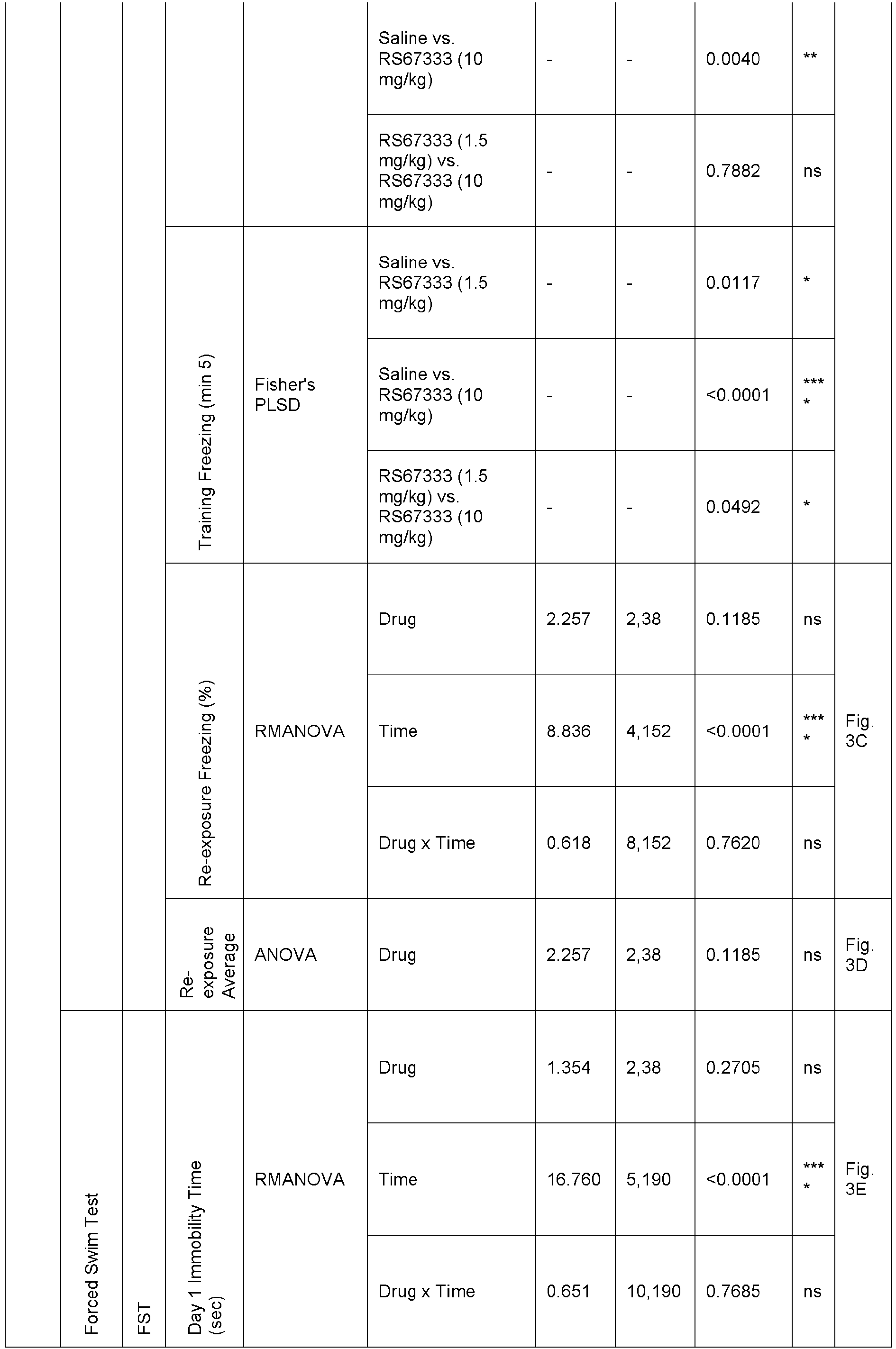

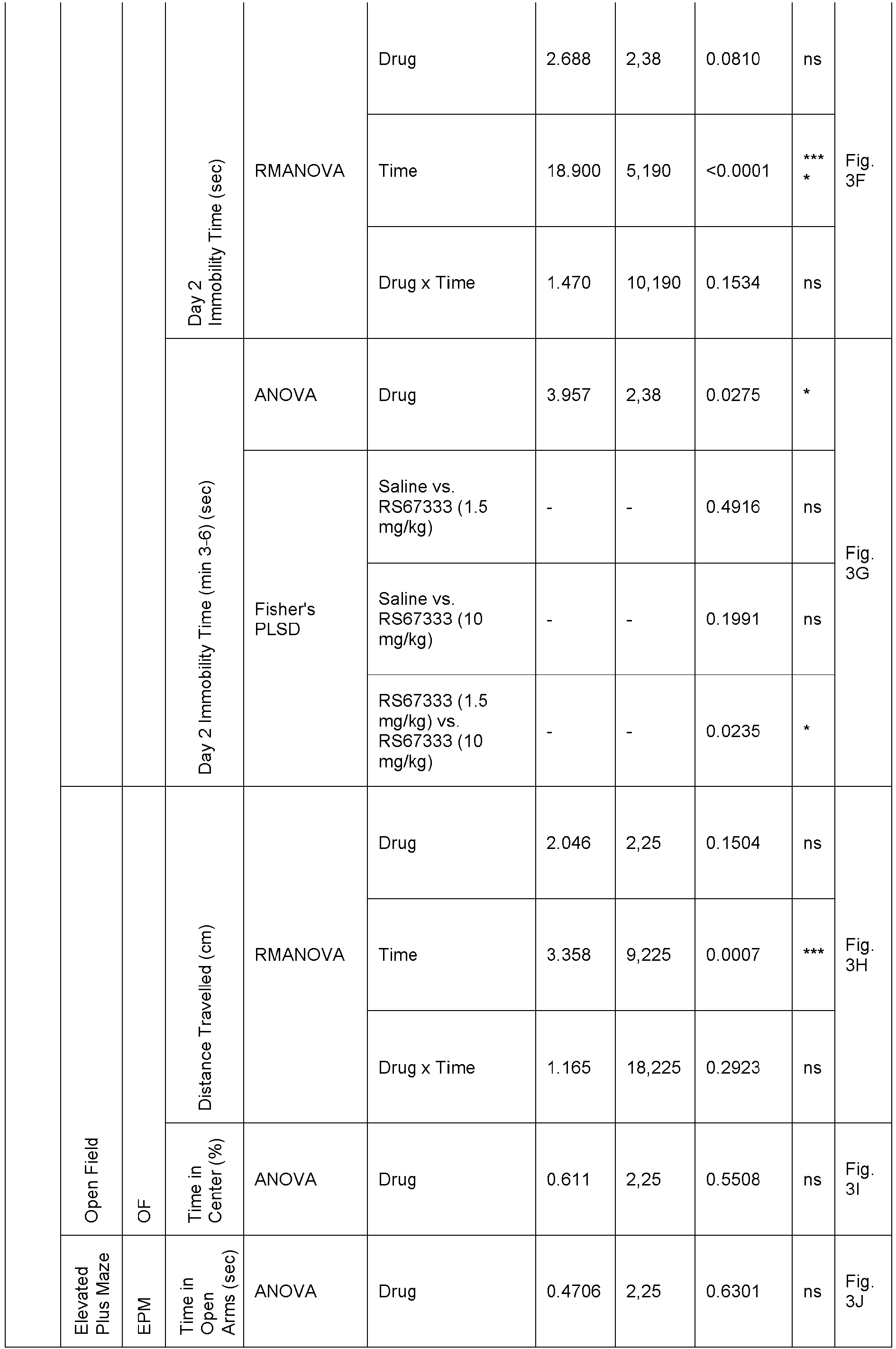

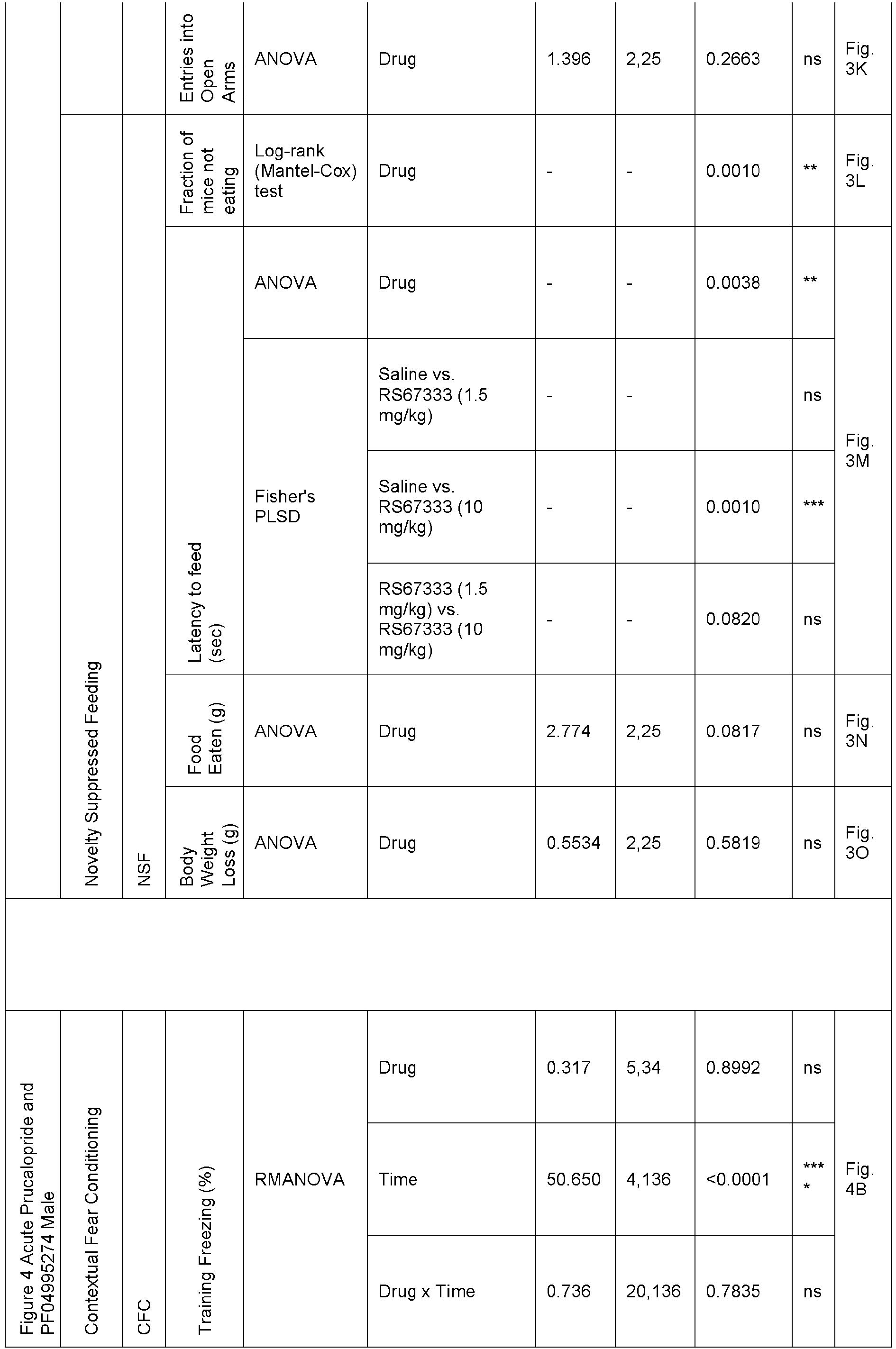

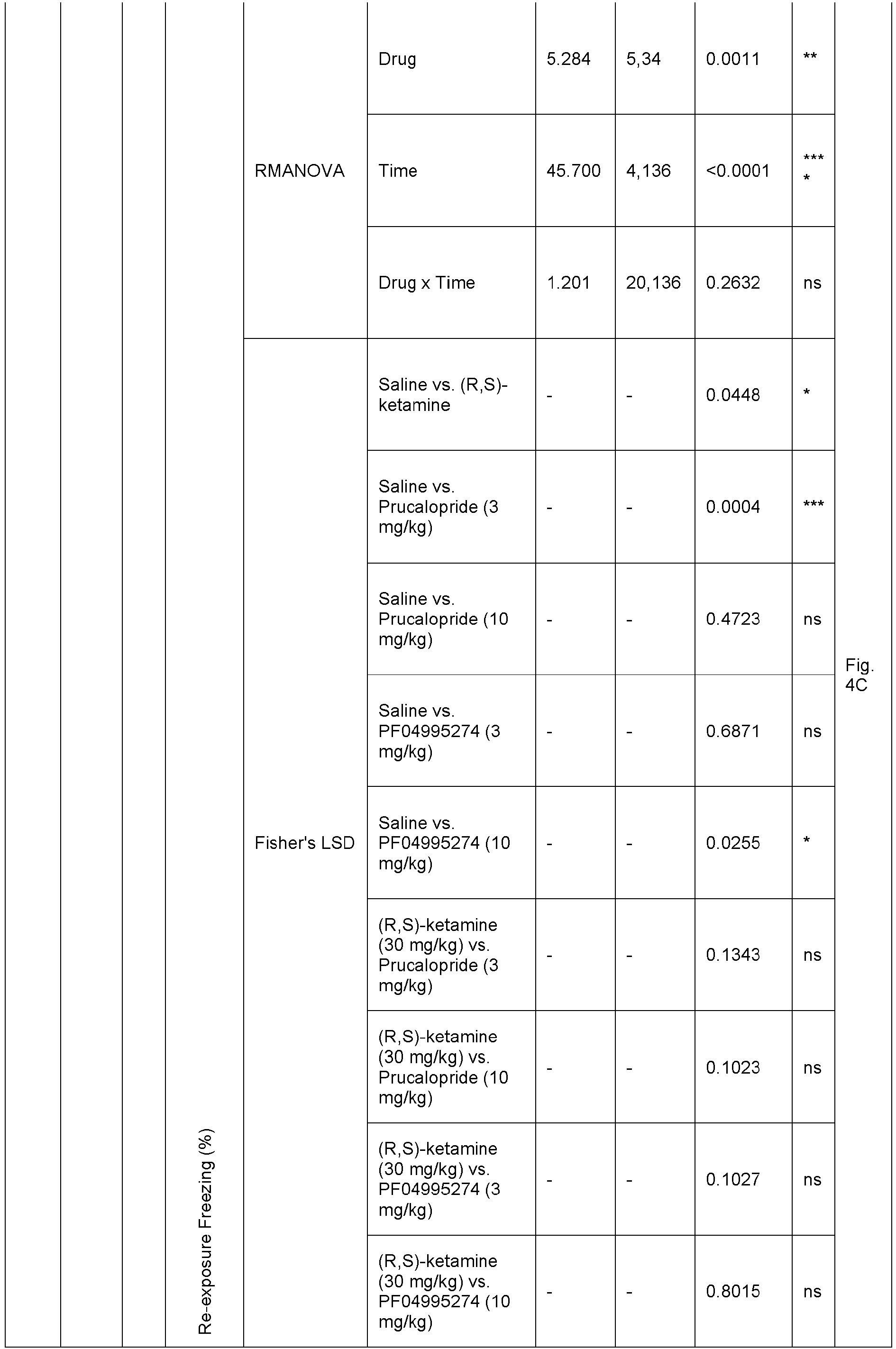

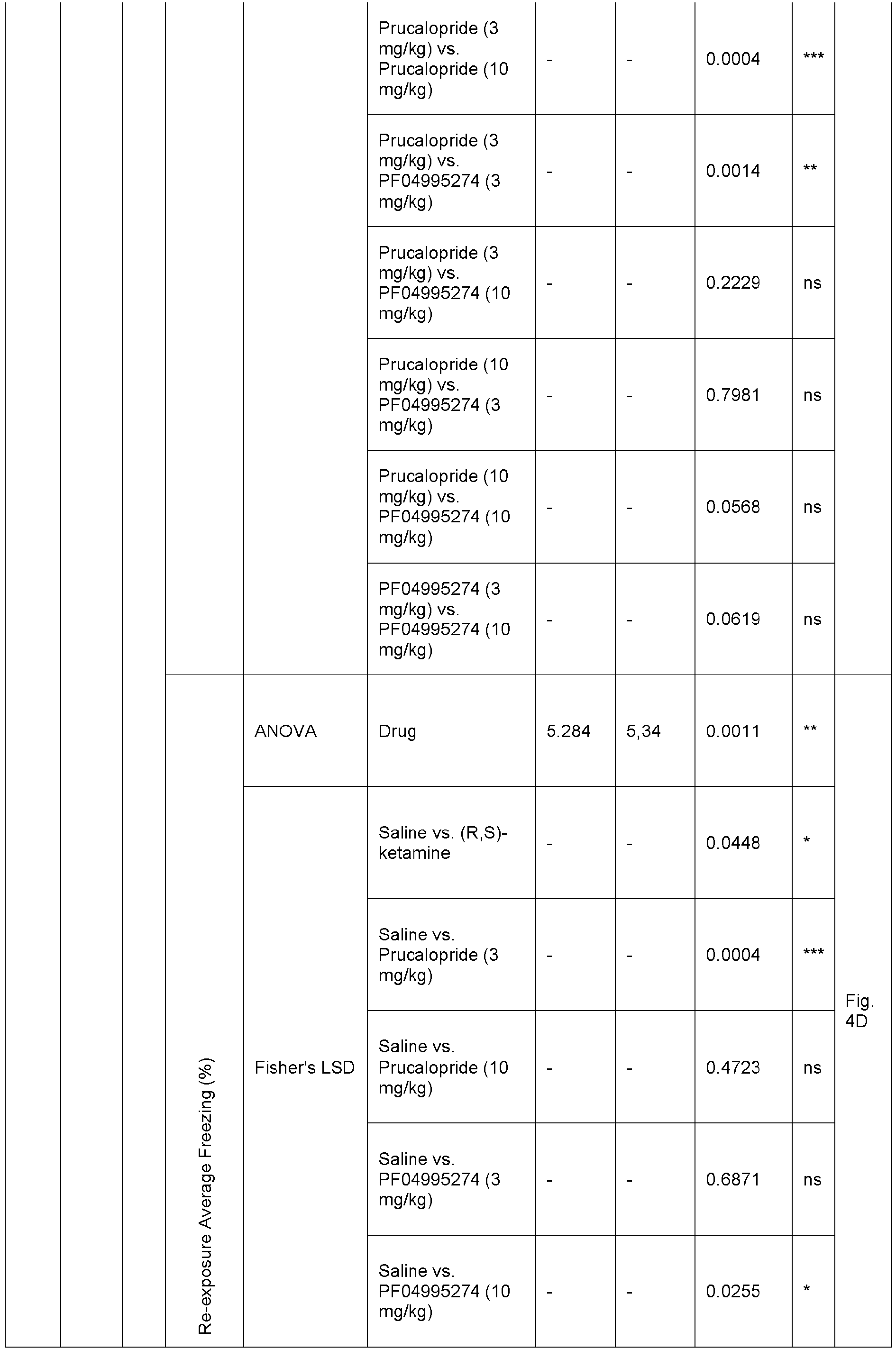

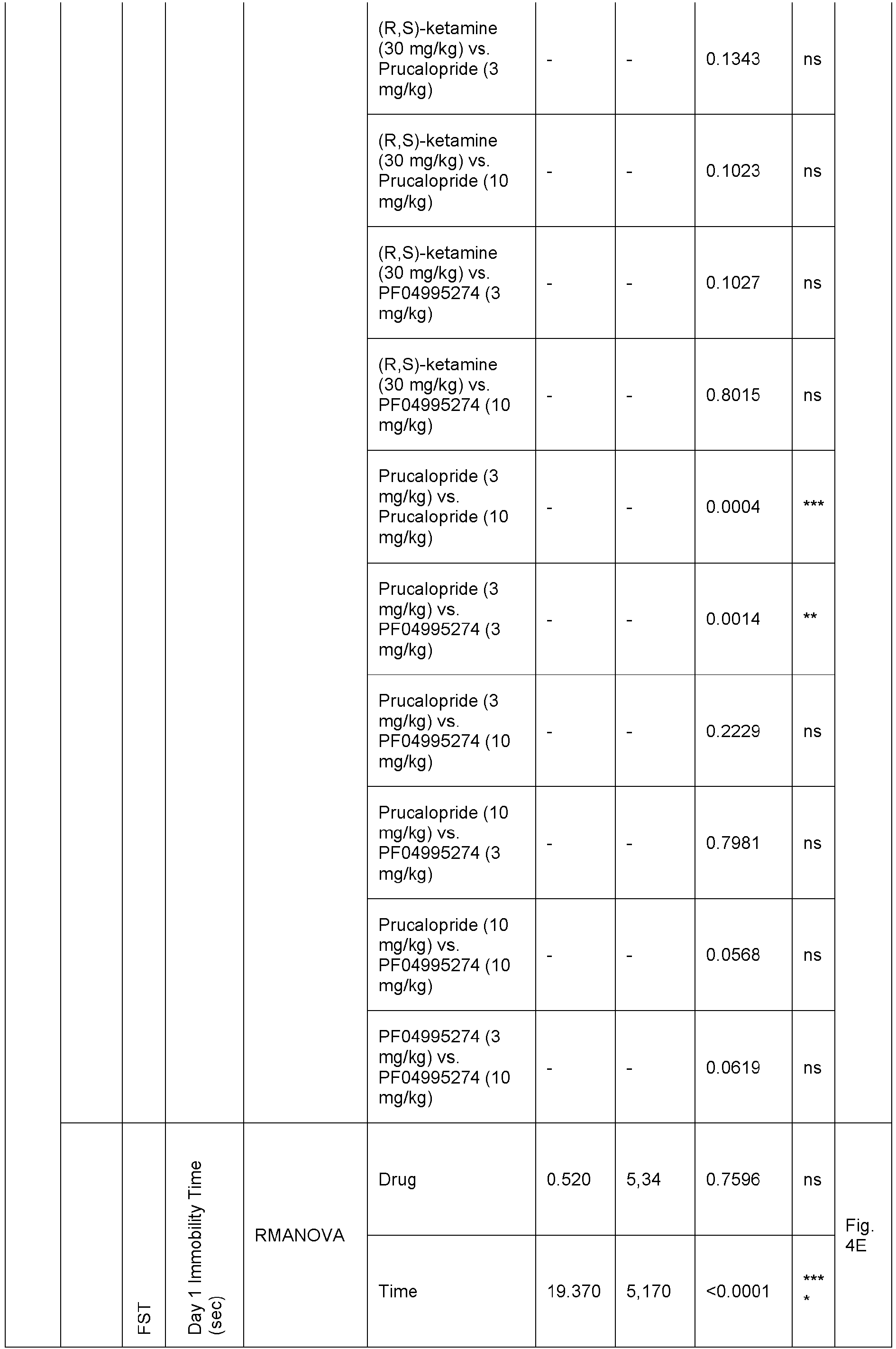

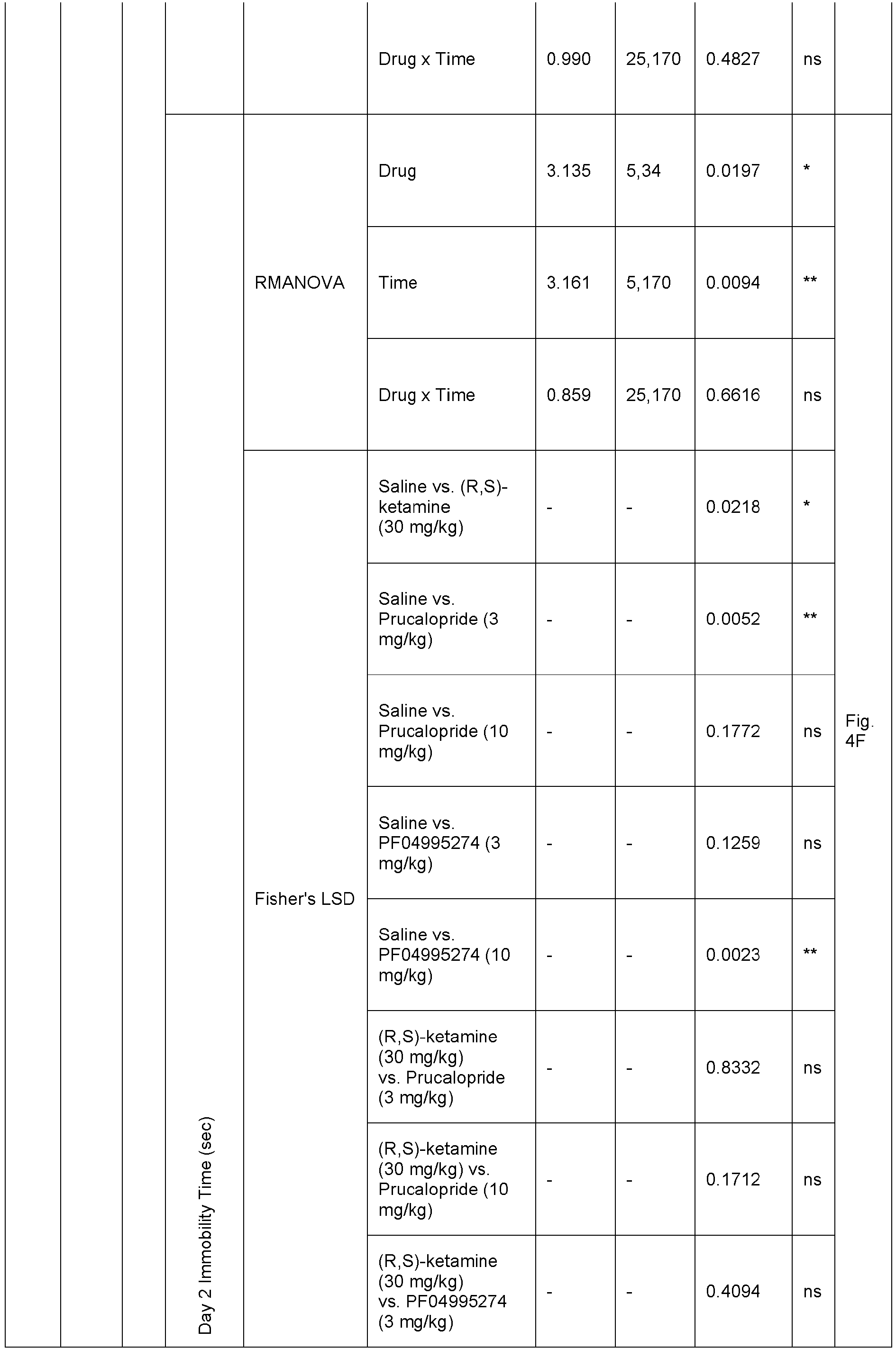

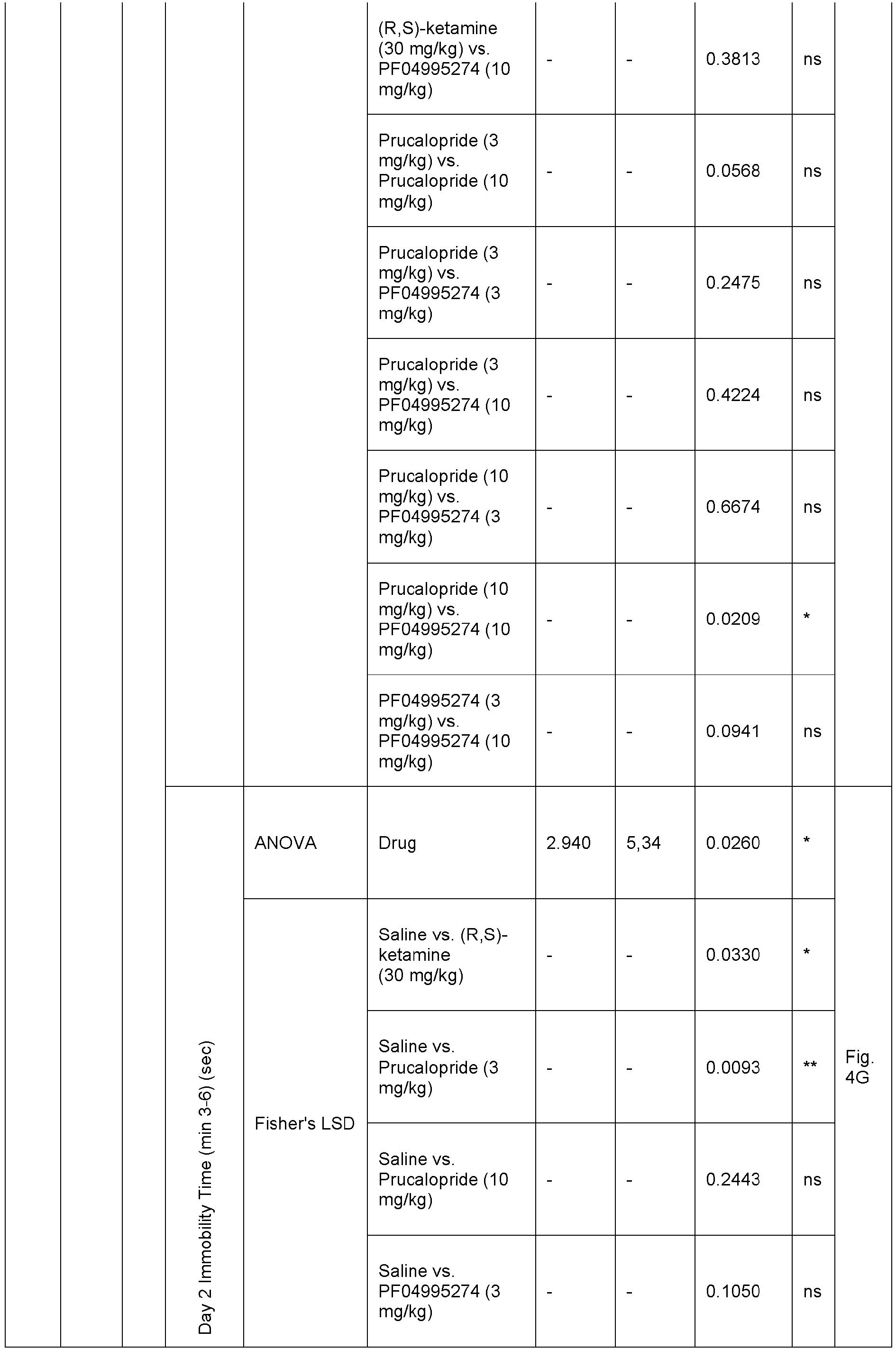

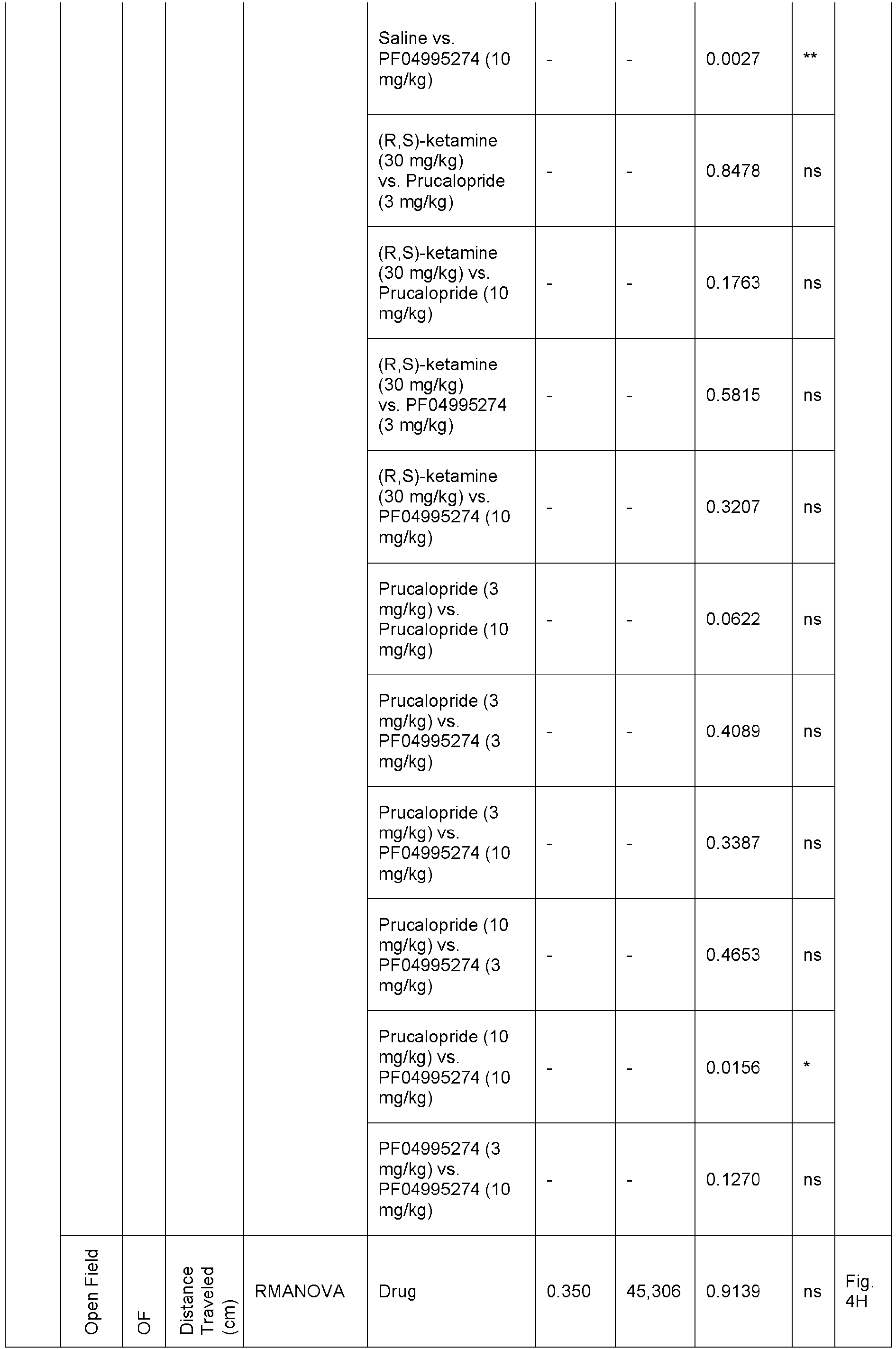

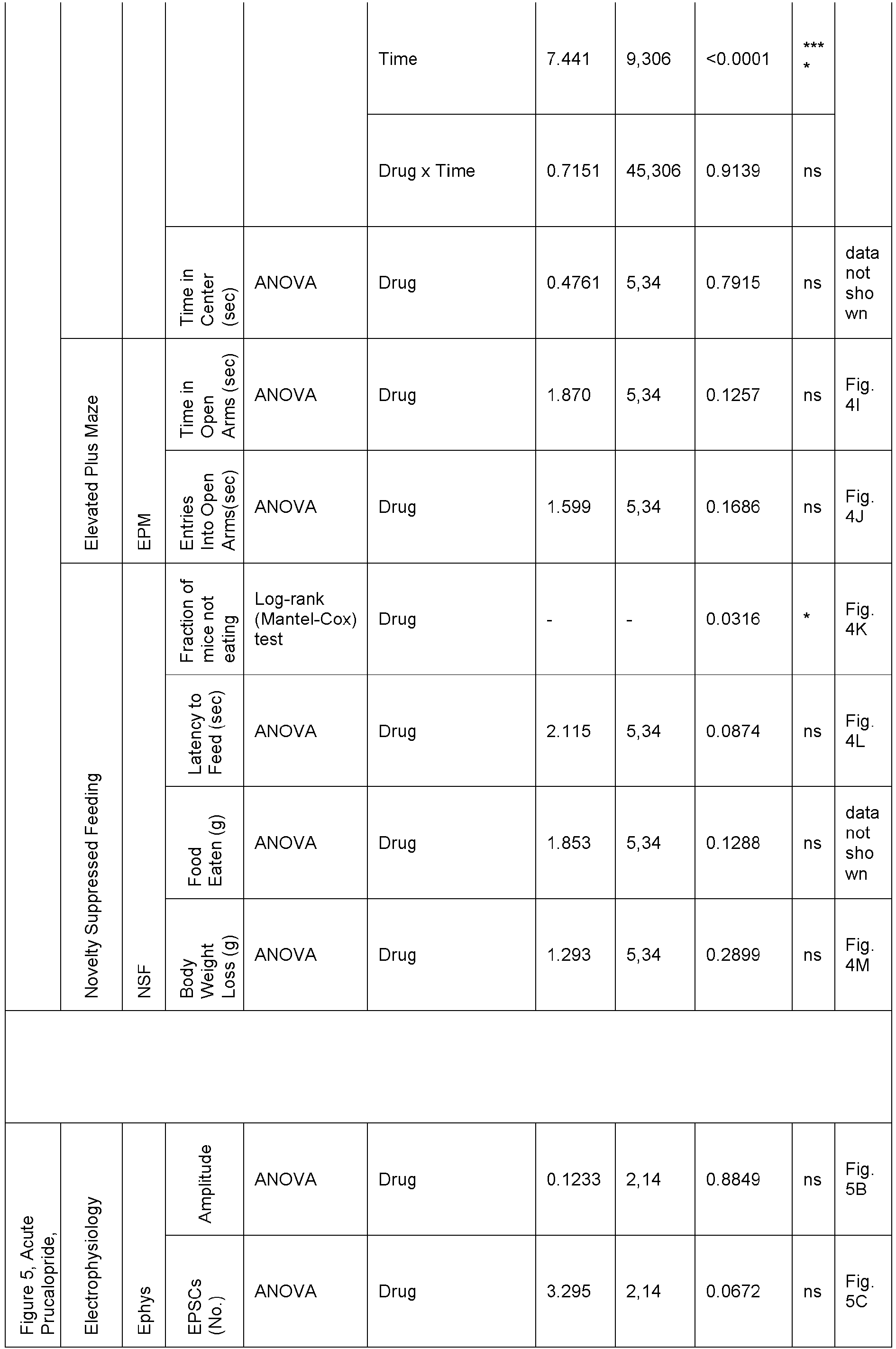

### REFERENCES

- 1: Kessler RC, Sonnega A, Bromet E, Hughes M, Nelson CB. Posttraumatic stress disorder in the National Comorbidity Survey. Arch Gen Psychiatry. 1995;52(12):1048-60.
- 2: Amat J, Dolzani SD, Tilden S, Christianson JP, Kubala KH, Bartholomay K, et al. Previous Ketamine Produces an Enduring Blockade of Neurochemical and Behavioral Effects of Uncontrollable Stress. J Neurosci. 2016;36(1):153-61.
- 3: Brachman RA, McGowan JC, Perusini JN, Lim SC, Pham TH, Faye C, et al. Ketamine as a Prophylactic Against Stress-Induced Depressive-like Behavior. Biol Psychiatry. 2016;79(9):776-86.
- 4: McGowan JC, LaGamma CT, Lim SC, Tsitsiklis M, Neria Y, Brachman RA, et al. Prophylactic Ketamine Attenuates Learned Fear. Neuropsychopharmacology. 2017.
- 5: McGowan JC, Hill C, Mastrodonato A, LaGamma CT, Kitayev A, Brachman RA, et al. Prophylactic ketamine alters nucleotide and neurotransmitter metabolism in brain and plasma following stress. Neuropsychopharmacology. 2018;43(9):1813-21.
- 6: Mastrodonato A, Martinez R, Pavlova IP, LaGamma CT, Brachman RA, Robison AJ, et al. Ventral CA3 Activation Mediates Prophylactic Ketamine Efficacy Against Stress-Induced Depressive-like Behavior. Biol Psychiatry. 2018;84(11):846-56.
- 7: McGhee LL, Maani CV, Garza TH, Slater TM, Petz LN, Fowler M. The intraoperative administration of ketamine to burned U.S. service members does not increase the incidence of post-traumatic stress disorder. Mil Med. 2014;179(8 Suppl):41-6.
- 8: Ma J-H, Wang S-Y, Yu H-Y, Li D-Y, Luo S-C, Zheng S-S, et al. Prophylactic use of ketamine reduces postpartum depression in Chinese women undergoing cesarean section. Psychiatry Research. 2019*.*
- 9: Xu Y, Li Y, Huang X, Chen D, She B, Ma D. Single bolus low-dose of ketamine does not prevent postpartum depression: a randomized, double-blind, placebo-controlled, prospective clinical trial. Arch Gynecol Obstet. 2017;295(5):1167-74.
- 10: Highland JN, Zanos P, Georgiou P, Gould TD. Group II metabotropic glutamate receptor blockade promotes stress resilience in mice. Neuropsychopharmacology. 2019.
- 11: Bockaert J, Claeysen S, Compan V, Dumuis A. 5-HT4 receptors. Curr Drug Targets CNS Neurol Disord. 2004;3(1):39-51.
- 12: Bockaert J, Claeysen S, Compan V, Dumuis A. 5-HT(4) receptors: history, molecular pharmacology and brain functions. Neuropharmacology. 2008;55(6):922-31.
- 13: Dumuis A, Sebben M, Bockaert J. The gastrointestinal prokinetic benzamide derivatives are agonists at the non-classical 5-HT receptor (5-HT4) positively coupled to adenylate cyclase in neurons. Naunyn Schmiedebergs Arch Pharmacol. 1989;340(4):403-10.
- 14: Dumuis A, Sebben M, Bockaert J. BRL 24924: a potent agonist at a non-classical 5-HT receptor positively coupled with adenylate cyclase in colliculi neurons. Eur J Pharmacol. 1989;162(2):381-4.
- 15: Faye C, Hen R, Guiard BP, Denny CA, Gardier AM, Mendez-David I, et al. Rapid anxiolytic effects of RS67333, a serotonin type 4 receptor agonist, and diazepam, a benzodiazepine, are mediated by projections from the prefrontal cortex to the dorsal raphe nucleus. Biological Psychiatry*.*
- 16: Bonaventure P, Hall H, Gommeren W, Cras P, Langlois X, Jurzak M, et al. Mapping of serotonin 5-HT(4) receptor mRNA and ligand binding sites in the post-mortem human brain. Synapse. 2000;36(1):35-46.
- 17: Hegde SS, Eglen RM. Peripheral 5-HT4 receptors. Faseb j. 1996;10(12):1398-407.
- 18: Amigo J, Diaz A, Pilar-Cuellar F, Vidal R, Martin A, Compan V, et al. The absence of 5-HT4 receptors modulates depression- and anxiety-like responses and influences the response of fluoxetine in olfactory bulbectomised mice: Adaptive changes in hippocampal neuroplasticity markers and 5-HT1A autoreceptor. Neuropharmacology. 2016;111:47-58.
- 19: Lucas G, Rymar VV, Du J, Mnie-Filali O, Bisgaard C, Manta S, et al. Serotonin(4) (5-HT(4)) receptor agonists are putative antidepressants with a rapid onset of action. Neuron. 2007;55(5):712-25.
- 20: Mendez-David I, David DJ, Darcet F, Wu MV, Kerdine-Romer S, Gardier AM, et al. Rapid anxiolytic effects of a 5-HT(4) receptor agonist are mediated by a neurogenesis-independent mechanism. Neuropsychopharmacology. 2014;39(6):1366-78.
- 21: Samuels BA, Mendez-David I, Faye C, David SA, Pierz KA, Gardier AM, et al. Serotonin 1A and Serotonin 4 Receptors: Essential Mediators of the Neurogenic and Behavioral Actions of Antidepressants. Neuroscientist. 2016;22(1):26-45.
- 22: Eglen RM, Bonhaus DW, Johnson LG, Leung E, Clark RD. Pharmacological characterization of two novel and potent 5-HT4 receptor agonists, RS 67333 and RS 67506, in vitro and in vivo. Br J Pharmacol. 1995;115(8):1387-92.
- 23: Giannoni P, Gaven F, de Bundel D, Baranger K, Marchetti-Gauthier E, Roman FS, et al. Early administration of RS 67333, a specific 5-HT4 receptor agonist, prevents amyloidogenesis and behavioral deficits in the 5XFAD mouse model of Alzheimer's disease. Front Aging Neurosci. 2013;5:96*.*
- 24: Prins NH, Van Haselen JF, Lefebvre RA, Briejer MR, Akkermans LM, Schuurkes JA. Pharmacological characterization of 5-HT4 receptors mediating relaxation of canine isolated rectum circular smooth muscle. Br J Pharmacol. 1999;127(6):1431-7.
- 25: Morris PJ, Moaddel R, Zanos P, Moore CE, Gould TD, Zarate CA, Jr., et al. Synthesis and N-Methyl-d-aspartate (NMDA) Receptor Activity of Ketamine Metabolites. Org Lett. 2017;19(17):4572-75.
- 26: Zanos P, Gould TD. Intracellular Signaling Pathways Involved in (S)- and (R)-Ketamine Antidepressant Actions. Biol Psychiatry. 2018;83(1):2-4.
- 27: Grimwood S, Drummond E, Zasadny K, Skaddan M, Brodney M, Coffman K, et al. Translational receptor occupancy for the 5-HT4 partial agonist PF-04995274 in rats, non-human primates and healthy volunteers. Alzheimer's & Dementia: The Journal of the Alzheimer's Association. 2011;7(4):S653.
- 28: Zanos P, Highland JN, Liu X, Troppoli TA, Georgiou P, Lovett J, et al. (R)-Ketamine exerts antidepressant actions partly via conversion to (2R,6R)-hydroxynorketamine, while causing adverse effects at sub-anaesthetic doses. Br J Pharmacol. 2019;176(14):2573-92.
- 29: Morris PJ, Moaddel R, Zanos P, Moore CE, Gould TD, Zarate CA, Jr., et al. Correction to "Synthesis and N-Methyl-d-aspartate (NMDA) Receptor Activity of Ketamine Metabolites". Org Lett. 2017;19(19):5494.
- 30: David DJ, Samuels BA, Rainer Q, Wang J-W, Marsteller D, Mendez I, et al. Neurogenesis-Dependent and -Independent Effects of Fluoxetine in an Animal Model of Anxiety/Depression. Neuron. 2009;62(4):479-93.
- 31: Denny CA, Burghardt NS, Schachter DM, Hen R, Drew MR. 4- to 6-week-old adult-born hippocampal neurons influence novelty-evoked exploration and contextual fear conditioning. Hippocampus. 2012;22(5):1188-201.
- 32: Drew MR, Denny CA, Hen R. Arrest of adult hippocampal neurogenesis in mice impairs single- but not multiple-trial contextual fear conditioning. Behav Neurosci. 2010;124(4):446-54.
- 33: Luna VM, Anacker C, Burghardt NS, Khandaker H, Andreu V, Millette A, et al. Adult-born hippocampal neurons bidirectionally modulate entorhinal inputs into the dentate gyrus. Science. 2019;364(6440):578.
- 34: David DJ, Samuels BA, Rainer Q, Wang JW, Marsteller D, Mendez I, et al. Neurogenesis-dependent and -independent effects of fluoxetine in an animal model of anxiety/depression. Neuron. 2009;62(4):479-93.
- 35: Lucas G, Compan V, Charnay Y, Neve RL, Nestler EJ, Bockaert J, et al. Frontocortical 5-HT4 receptors exert positive feedback on serotonergic activity: viral transfections, subacute and chronic treatments with 5-HT4 agonists. Biol Psychiatry. 2005;57(8):918-25.
- 36: Eglen RM, Wong EH, Dumuis A, Bockaert J. Central 5-HT4 receptors. Trends Pharmacol Sci. 1995;16(11):391-8.
- 37: Warner-Schmidt JL, Flajolet M, Maller A, Chen EY, Qi H, Svenningsson P, et al. Role of p11 in Cellular and Behavioral Effects of 5-HT4 Receptor Stimulation. The Journal of Neuroscience. 2009;29(6):1937.
- 38: Egeland M, Warner-Schmidt J, Greengard P, Svenningsson P. Co-expression of serotonin 5-HT1B and 5-HT4 receptors in p11 containing cells in cerebral cortex, hippocampus, caudate-putamen and cerebellum. Neuropharmacology. 2011;61(3):442-50.
- 39: Su T-P, Zhang L, Chung M-Y, Chen Y-S, Bi Y-M, Chou Y-H, et al. Levels of the potential biomarker p11 in peripheral blood cells distinguish patients with PTSD from those with other major psychiatric disorders. Journal of Psychiatric Research. 2009;43(13):1078-85.
- 40: Zhang L, Su T-P, Choi K, Maree W, Li C-T, Chung M-Y, et al. P11 (S100A10) as a potential biomarker of psychiatric patients at risk of suicide. Journal of Psychiatric Research. 2011;45(4):435-41.
- 41: Zhang L, Ursano RJ, Li H. P11: a potential biomarker for posttraumatic stress disorder. Methods Mol Biol. 2012;829:453-68.
- 42: Castello J, LeFrancois B, Flajolet M, Greengard P, Friedman E, Rebholz H. CK2 regulates 5-HT4 receptor signaling and modulates depressive-like behavior. Mol Psychiatry. 2018;23(4):872-82.
- 43: Autry AE, Adachi M, Nosyreva E, Na ES, Los MF, Cheng PF, et al. NMDA receptor blockade at rest triggers rapid behavioural antidepressant responses. Nature. 2011;475(7354):91-5.
- 44: Nosyreva E, Szabla K, Autry AE, Ryazanov AG, Monteggia LM, Kavalali ET. Acute suppression of spontaneous neurotransmission drives synaptic potentiation. J Neurosci. 2013;33(16):6990-7002.
- 45: Zanos P, Gould TD. Mechanisms of ketamine action as an antidepressant. Mol Psychiatry. 2018;23(4):801-11.
- 46: Zanos P, Moaddel R, Morris PJ, Georgiou P, Fischell J, Elmer GI, et al. NMDAR inhibition-independent antidepressant actions of ketamine metabolites. Nature. 2016;533(7604):481-6.
- 47: Suzuki K, Nosyreva E, Hunt KW, Kavalali ET, Monteggia LM. Effects of a ketamine metabolite on synaptic NMDAR function. Nature. 2017;546(7659):E1-e3.
- 48: Teixeira CM, Rosen ZB, Suri D, Sun Q, Hersh M, Sargin D, et al. Hippocampal 5-HT Input Regulates Memory Formation and Schaffer Collateral Excitation. Neuron. 2018;98(5):992-1004.e4.
- 49: Rebola N, Carta M, Mulle C. Operation and plasticity of hippocampal CA3 circuits: implications for memory encoding. Nat Rev Neurosci. 2017;18(4):208-20.
- 50: De Vadder F, Grasset E, Manneras Holm L, Karsenty G, Macpherson AJ, Olofsson LE, et al. Gut microbiota regulates maturation of the adult enteric nervous system via enteric serotonin networks. Proc Natl Acad Sci U S A. 2018;115(25):6458-63.
- 51: Bianco F, Bonora E, Natarajan D, Vargiolu M, Thapar N, Torresan F, et al. Prucalopride exerts neuroprotection in human enteric neurons. Am J Physiol Gastrointest Liver Physiol. 2016;310(10):G768-75.
- 52: Liu MT, Kuan YH, Wang J, Hen R, Gershon MD. 5-HT4 receptor-mediated neuroprotection and neurogenesis in the enteric nervous system of adult mice. J Neurosci. 2009;29(31):9683-99.
- 53: Sittig LJ, Carbonetto P, Engel KA, Krauss KS, Barrios-Camacho CM, Palmer AA. Genetic Background Limits Generalizability of Genotype-Phenotype Relationships. Neuron. 2016;91(6):1253-59.
- 54: Darcet F, Gardier AM, David DJ, Guilloux JP. Chronic 5-HT4 receptor agonist treatment restores learning and memory deficits in a neuroendocrine mouse model of anxiety/depression. Neurosci Lett. 2016;616:197-203.
- 55: Dossat AM, Wright KN, Strong CE, Kabbaj M. Behavioral and biochemical sensitivity to low doses of ketamine: Influence of estrous cycle in C57BL/6 mice. Neuropharmacology. 2018;130:30-41.
- 56: Picard N, Takesian AE, Fagiolini M, Hensch TK. NMDA 2A receptors in parvalbumin cells mediate sex-specific rapid ketamine response on cortical activity. Mol Psychiatry. 2019;24(6):828-38.
- 57: Thelen C, Flaherty E, Saurine J, Sens J, Mohamed S, Pitychoutis PM. Sex Differences in the Temporal Neuromolecular and Synaptogenic Effects of the Rapid-acting Antidepressant Drug Ketamine in the Mouse Brain. Neuroscience. 2019;398:182-92.
- 58: Li N, Lee B, Liu RJ, Banasr M, Dwyer JM, Iwata M, et al. mTOR-dependent synapse formation underlies the rapid antidepressant effects of NMDA antagonists. Science. 2010;329(5994):959-64.
- 59: Dolzani SD, Baratta MV, Moss JM, Leslie NL, Tilden SG, Sorensen AT, et al. Inhibition of a Descending Prefrontal Circuit Prevents Ketamine-Induced Stress Resilience in Females. eNeuro. 2018;5(1)*.*
- 60: Mekiri M, Gardier AM, David DJ, Guilloux JP. Chronic corticosterone administration effects on behavioral emotionality in female c57bl6 mice. Exp Clin Psychopharmacol. 2017;25(2):94-104.
- 61: Kessler RC, McGonagle KA, Zhao S, Nelson CB, Hughes M, Eshleman S, et al. Lifetime and 12-month prevalence of DSM-III-R psychiatric disorders in the United States. Results from the National Comorbidity Survey. Arch Gen Psychiatry. 1994;51(1):8-19.
- S1: Brachman RA, McGowan JC, Perusini JN, Lim SC, Pham TH, Faye C, et al. Ketamine as a Prophylactic Against Stress-Induced Depressive-like Behavior. Biol Psychiatry. 2016;79(9):776-86.
- S2: Denny CA, Burghardt NS, Schachter DM, Hen R, Drew MR. 4- to 6-week-old adult-born hippocampal neurons influence novelty-evoked exploration and contextual fear conditioning. Hippocampus. 2012;22(5):1188-201.
- S3: David DJ, Samuels BA, Rainer Q, Wang J-W, Marsteller D, Mendez I, et al. Neurogenesis-Dependent and -Independent Effects of Fluoxetine in an Animal Model of Anxiety/Depression. Neuron. 2009;62(4):479-93.
- S4: Holmes PV. Rodent models of depression: reexamining validity without anthropomorphic inference. Crit Rev Neurobiol. 2003;15(2):143-74.
- S5: Petit-Demouliere B, Chenu F, Bourin M. Forced swimming test in mice: a review of antidepressant activity. Psychopharmacology (Berl). 2005;177(3):245-55.
- S6: Lindholm JS, Autio H, Vesa L, Antila H, Lindemann L, Hoener MC, et al. The antidepressant-like effects of glutamatergic drugs ketamine and AMPA receptor potentiator LY 451646 are preserved in bdnf(+)/(-) heterozygous null mice. Neuropharmacology. 2012;62(1):391-7.
- S7: Ghasemi M, Raza M, Dehpour AR. NMDA receptor antagonists augment antidepressant-like effects of lithium in the mouse forced swimming test. J Psychopharmacol. 2010;24(4):585-94.
- S8: Liu RJ, Lee FS, Li XY, Bambico F, Duman RS, Aghajanian GK. Brain-derived neurotrophic factor Val66Met allele impairs basal and ketamine-stimulated synaptogenesis in prefrontal cortex. Biol Psychiatry. 2012;71(11):996-1005.

### EXAMPLE 2 Rapid anxiolytic effects of RS67333, a serotonin type 4 receptor agonist, and diazepam, a benzodiazepine, are mediated by projections from the prefrontal cortex to the dorsal raphe nucleus

### Summary

Background: Activating the serotonin (5-HT) 4 receptors (5-HT₄Rs) has been shown to have anxiolytic effects in a variety of animal models. Characterizing the circuits responsible for these effects should offer insights into new approaches to treat anxiety.

Methods: We evaluated whether acute 5-HT₄R activation in glutamatergic axon terminals arising from the medial prefrontal cortex (mPFC) to the dorsal raphe nucleus (DRN) induced fast anxiolytic effects. Anxiolytic effects of an acute systemic administration (1.5 mg/kg, intraperitoneally, i.p.) or intra-mPFC infusion with the 5-HT₄R agonist, RS67333 (0.5 mg/side), were examined in mice. To provide evidences that anxiolytic effects of RS67333 recruited an mPFC-DRN neural circuit, *in vivo* recordings of firing rate of DRN serotonin (5-HT) neurons, cerebral 5-HT depletion, and optogenetic activation/silencing were performed. Results: Acute systemic administration and intra-mPFC infusion of RS67333 produced fast anxiolytic effects and increased DRN 5-HT cell firing. Serotonin depletion prevented anxiolytic effects induced by mPFC infusion of RS67333. Surprisingly the anxiolytic effects of mPFC infusion diazepam (1.5 mg/side) were also blocked by 5HT depletion. Optogenetically activating mPFC terminals targeting the DRN reduced anxiety whereas silencing this circuit blocked RS67333 and diazepam mPFC infusion -induced anxiolytic effects. Finally, anxiolytic effects induced by an acute systemic RS67333 or diazepam administration were partially blocked after optogenetically inhibiting cortical glutamatergic terminals in the DRN.

Conclusions: Our findings suggest that activating 5-HT₄R acutely in the mPFC or targeting mPFC pyramidal cell terminals in the DRN, may constitute a strategy to produce a fast-anxiolytic response.

Unlike SSRIs, treatment with RS67333, a 5-HT₄R agonist (A13), induced fast anxiolytic/antidepressant-like effects through a neurogenesis-independent mechanism (A5). Although a number of studies have assessed the anxiolytic/antidepressant-like activity of 5-HT₄R modulation after subchronic or chronic treatment, few have evaluated their anxiolytic-like profile acutely. Conflicting evidence suggests that 5-HT₄R antagonists have acute anxiolytic-like effects (A14, A15). Two reports showed an anxiolytic effect of 5-HT₄R antagonists SB 204070, GR 113808 (A15) and SB 207266A (A14, A15) in rats in the elevated plus maze (EPM). However, another study did not detect an effect of the antagonists SB 204070 and GR 113808 on the number of open arm entries in the EPM (A15). Similarly, a direct effect of the 5-HT₄R antagonists on anxiety-like behavior in the light/dark choice test was not detected (A16). The reasons for these discrepancies are unclear. In contrast, acute 5-HT₄R activation has been shown to be an encouraging pharmacological strategy to obtain a fast anxiolytic-like response. Recently, acute administration of RS67333, induced anxiolytic-like effects in mice (A17) and reversed the anxiogenic effects of chronic exposure to cannabinoids during adolescence (A18).

Interestingly, approximately 60% of pyramidal neurons recorded in the medial prefrontal cortex (mPFC) contain both the 5-HT₄R transcript and protein. Activation of these somatodendritic 5-HT₄R in the mPFC results in glutamate release in the DRN to stimulate the firing of 5-HT neurons (A19). A large body of evidence also suggests that mPFC projections to the DRN modulate anxiety and depression-related behaviors (A20-A22). Indeed, chronic optical stimulation of layer V pyramidal cells in the PFC induced a long-lasting anxiolytic-like effect in a mouse model of anxiety/depression (A23), and inhibition of mPFC terminals targeting to the DRN induces a long-lasting suppression of anxiety-like behavior in socially stressed mice (A24). Lastly, a recent study revealed a key role for DRN circuits in environment-specific adaptive behaviors (A25). As a result, it is possible that projection from the mPFC to DRN may mediate the anxiolytic effects of 5-HT₄R activation.

Here, using behavioral paradigms predictive of anxiolytic-like activity, we first evaluated the consequences of an acute systemic or intra-mPFC administration with RS67333 or the GABA_{A} modulator diazepam in male BALB/cJRj anxious mice (A26). Then, using optogenetic techniques, we assessed the contribution of glutamatergic axon terminals arising from the mPFC to the DRN on fast anxiolytic-like effects.

### MATERIALS AND METHODS

### Subjects

Male BALB/cJRj mice (Janvier Labs, Le Genest-St-Isle, France) were 7-8 weeks old, weighed 25-30 g, and were maintained on a 12h light: 12h dark schedule (lights on at 06:00 hours). Food and water were provided *ad libitum* except during behavioral observations. The protocols were conducted in conformity with the institutional guidelines that are in compliance with national and international laws and policies (Council directive #87-848, October 19, 1987, Ministère de l'Agriculture et de la Forêt, Service Vétérinaire de la Santé et de la Protection Animale, permissions # 92-256B to DJD, Institutional Animal Care and Use Committee 26 authorization #4074).

### Drugs

1-(4-amino-5-chloro-2-methoxyphenyl)-3-(1-butyl-4piperidinyl)-1-propanone hydrochloride [RS67333, a serotonin 4 receptor (5-HT₄R) agonist] administered intraperitoneally (i.p.) at 1.5 mg/kg (A-S1), or locally in the medial Prefrontal Cortex (mPFC) (A-S2, A-S3) at 0.5 µg/side and 5-Fluoro-2-methoxy-[1-[2-[(methylsulfonyl)amino]ethyl]-4-piperidinyl]-1H-indole-3-methylcarboxylate sulfamate (GR125487, a 5-HT₄R antagonist) administered i.p. at 1 mg/kg (1) were dissolved in saline (0.9 % NaCl) solution and purchased from Tocris Bioscience (Bristol, United Kingdom). RS67333 shows high binding affinity for the 5-HT₄R with a pKi of 8.7 (A-S4, A-S5). Except for the sigma receptors, which are bound at affinities comparable to 5-HT₄R (sigma 1: pKi = 8.9; and sigma 2: pKi = 8.0), RS67333 has a pKi of less than 6.7 for other neurotransmitter receptors. Diazepam hydrochloride (dissolved in 0.5 % Tween^{®}20 solution, Sigma-Aldrich, Saint-Quentin Fallavier, France) was administered i.p. at 1.5 mg/kg (A-S6) or locally in the mPFC at 1.5 µg/side (A-S7), 45 minutes before testing. Fluoxetine hydrochloride (dissolved in saline, Anawa Trading, Zurich, Switzerland) was administered at 18 mg/kg, i.p., 45 minutes before testing (A-S6). Parachlorophenylalanine methyl ester (p-CPA, dissolved in Tween 1% solution, Sigma-Aldrich, Saint-Quentin Fallavier, France) was administered i.p. twice a day for 3 consecutive days at 150 mg/kg ( A-S8, A-S9).

### Treatments

### Systemic administration with RS67333, diazepam or fluoxetine

RS67333 (1.5 mg/kg, i.p.), diazepam (1.5 mg/kg, i.p.), fluoxetine (18 mg/kg, i.p.) were injected 45 min before testing in the Elevated Plus Maze (EPM), the Novelty Suppressed Feeding (NSF) or the Open Field (OF) in three independent cohorts of male BALB/cJRj mice.

To ensure the selectivity of the anxiolytic-like effects of RS67333, in a new cohort of male BALB/cJRj mice, GR125487 (1.0 mg/kg, i.p.) dissolved in 0.9 % NaCl solution, was injected 15 minutes before RS67333 administration (1.5 mg/kg, i.p.). EPM or NSF occurred 45 min after RS67333 administration (A-S1). Behavioral consequences of the coadministration of GR125487+RS67333 were compared to RS67333 alone, diazepam (1.5 mg/kg, i.p.) fluoxetine (18 mg/kg, i.p.) and vehicle groups (0.9% saline solution, i.p.).

### mPFC local infusion of RS67333

For mPFC drug infusion, two bilateral cannulae (75 µm-diameter silica capillary tubing inserted in 27G stainless steel catheter) were implanted in the mPFC [stereotaxic coordinates in mm from bregma: A = +2.10, L = ±0.50, V = -2.60, A, anterior; L, lateral; and V, ventral, according to (A-S10)] under anesthesia (chloral hydrate, 400 mg/kg, i.p.). The following day, RS67333 (0.5 µg/side) was continuously perfused in awake freely moving male BALB/cJRj mice at a flow rate of 0.2 µL/min for 2 minutes (LEGATO^{™} 180 syringe pump, KD Scientific Inc., Holliston, MA, USA), 45 min before testing in the EPM and in the NSF. Diazepam (1.5 mg/kg) was used as a positive control.

### Serotonin depletion

In a new cohort of male BALB/cJRj mice, p-CPA was administered twice daily (at 0900 and 1700 h) for 3 consecutive days. RS67333 (0.5 µg/side) and diazepam (1.5 µg/side) were then intra-mPFC administered 24 h after the final p-CPA administration and behavioral test (EPM) occurred 45 minutes after local infusion.

For the p-CPA study, immediately after behavioral tests, animals were sacrificed and frontal cortex were dissected and reduced in cortical brain homogenates for 5-HT concentration measurements by ELISA method (Immusmol, France) to verify the 5-HT depletion of tissue content.

### Behavioral tests

### Elevated plus maze

The EPM is a widely used behavioral assay for rodents and it has been validated to assess the anti-anxiety effects of pharmacological agents (A-S11). This test was performed as described by (A-S1). The maze is a plus-cross-shaped apparatus, with two open arms and two arms closed by walls linked by a central platform 50 cm above the floor. Mice were individually put in the center of the maze facing an open arm and were allowed to explore the maze during 5 min for the behavioral consequences of an acute systemic administration or mPFC infusion and during 6 min for the optogenetic experiments. The time spent in and the numbers of entries into the open arms were used as an anxiety index. All parameters were measured using a videotracker (EPM3C, Bioseb, Vitrolles, France).

### Novelty suppressed feeding

The NSF is a conflict test that elicits competing motivations: the drive to eat and the fear of venturing into the center of a brightly lit arena. The latency to begin eating is used as an index of anxiety/depression-like behavior, because classical anxiolytic drugs as well as chronic antidepressants decrease this measure. The NSF test was carried out during a 10 min period as previously described (A-S12). Briefly, the testing apparatus consisted of a plastic box (50x50x20 cm), the floor of which was covered with approximately 2 cm of wooden bedding. Twenty-four hours prior to behavioral testing, all food was removed from the home cage. At the time of testing, a single pellet of food (regular chow) was placed on a white paper platform positioned in the center of the box. Each animal was placed in a corner of the box, and a stopwatch was immediately started. The latency to eat (defined as the mouse sitting on its haunches and biting the pellet with the use of forepaws) was timed. Immediately afterwards, the animal was transferred to its home cage, and the amount of food consumed by the mouse in the subsequent 5 min was measured, serving as a control for change in appetite as a possible confounding factor.

### Open field paradigm (OF)

Motor activity was quantified in four 39 × 39 cm perpex plastic open field boxes (Vivo-tech/Ugo Basile, Salon de Provence, France). The apparatus was illuminated from the ground with special designed 40 x 40 cm Infra-red backlights (monochromatic wavelength 850 nm high homogeneity, Vivo-tech, Salon de Provence, France). Activity chambers were monitored by four black and white cameras with varifocal optics and polarizing filters (Vivo-tech, Salon de Provence, France). For optogenetic experiments, optical bandpass filters were specifically selected to improve tracking detection. The whole set-up was controlled using ANYMAZE version 6 video tracking software (Stoelting Co/Vivo-tech, Salon de Provence, France). Dependent measures were time in the center over a 10 min for systemic administration or 6 min test period for optogenetic experiments, total ambulatory distance and ambulatory distance traveled in the center divided by total distance.

### In vivo electrophysiological recordings

Dorsal Raphe Nucleus (DRN) 5-HT neurons were identified according to the following criteria: a slow (0.5-2.5 Hz) and regular firing rate and a long duration, positive action potential as previously reported (A-S13).

### Optogenetic manipulations

### Virus injection

To target opsin expression selectively to cortical glutamatergic terminals in the DRN, AAV5-CaMKII*α*-ChR2-enhanced yellow fluorescent protein (eYFP), AAV5-CaMKII-ArchT-green fluorescent protein (GFP) or AAV5-CaMKII-eYFP, obtained from Karl Deisseroth and Ed Boyden (UNC Vector Core, NC, USA) were bilaterally injected into the mPFC (in mm from bregma, A = +2.10, L = ±0.50, V = -2.60). Mice injected with AAV5-CamKII-eYFP were used as control.

### Optical fibers construction

As we previously described, for all experiments, a 200 µm core, 0.37 numerical aperture (NA) multimode fiber (ThorLabs, Maison Laffitte, France) was used for optical stimulation through a patch cable connected to a 100 mw 473 nm blue and 532 nm green laser diode (OEM laser systems, USA) (A-S14).

### Fiber optics implantation and optogenetic procedure

BALB/cJRj mice were surgically implanted with fiber optics targeted to the DRN (in mm from bregma, A = -4.50, L = +1.20, V = -4.0, angle 15°). A 200 mm core, 0.37 NA fiber optic (ThorLabs, ~10-12 and 15-16 mW for ChR2 and Arch-T, respectively, at the tip of optic) was used for optical stimulation via a patch cable connected to either a 473 or 532 nm laser diode (OEM laser systems, USA) as previously described (A-S15). For behavioral experiments, AAV5-CaMKII*α*-ChR2-eYFP mice and their controls received a 10 Hz stimulation, 20 ms pulses, over a 3-minute period whereas a green light was delivered continuously to AAV5-CaMKII-ArchT-GFP throughout the 3 min testing period. Similar doses of RS67333 (locally in the mPFC at 0.5 µg/side or at 1.5 mg/kg, i.p.) and diazepam (locally in the mPFC at 1.5 µg/side or at 1.5 mg/kg, i.p.) were infused in the mPFC or administered i.p. The stimulation or the inhibition of mPFC projections in the DRN occurred simultaneously to the behavioral paradigms.

### Immunohistochemistry

To ensure opsin expression, mice were perfused transcardially (cold saline for 2 minutes, followed by 4% cold PFA) after anesthesia (100 mg/ml ketamine and 20 mg/ml xylazine, i.p.). Brains were removed and cryoprotected with 30% sucrose at 4°C. Thirty-five µm-thick coronal sections were cut through the entire brain and stored in 1X phosphate buffered saline (PBS) with 0.1% sodium azide. Free-floating sections were incubated in a blocking buffer (0.5% Triton X-100, 5% normal donkey serum (NDS), 1X PBS) for 2 hours at room temperature. eYFP and GFP were detected using rabbit GFP Tag polyclonal antibody (1:500, Thermo Fisher Scientific, catalog #A-11122) in the same buffer at 4°C overnight. Following washed in 1X PBS, secondary Cy3-AffiniPure donkey anti-rabbit antibody (1:250, Jackson Immunoresearch, 711-165-152) was added in 1X PBS with 10% NDS buffer for 2 hours at room temperature. After several rinses in 1X PBS, sections mounted on slide, airdried, coverslipped with fluoromont and examined under confocal microscopy (Olympus BX51) using appropriate filters.

### Statistical analysis

Results from data analyses, expressed as mean ± SEM were analyzed using Prism 8.1.2 software (Graphpad, San Diego, CA, USA). For all experiments, Student's test, one-way or two-way ANOVAs were applied to the data as appropriate. Significant main effects and/or interactions were followed by Fisher's PLSD post-hoc analysis. In the NSF, we used the Kaplan-Meier survival analysis due to the lack of normal distribution of the data and Mantel-Cox log rank test to evaluate differences between experimental groups. Statistical significance was set at p<0.05. All statistical tests and p values are listed in Tables 3-6.

### RESULTS

### Acute systemic 5-HT₄R stimulation induced fast anxiolytic-like effect.

To assess putative fast anxiolytic 5-HT₄R activation, vehicle, fluoxetine (18 mg/kg), diazepam (1.5 mg/kg), or RS67333 (1.5 mg/kg) were administered i.p., 45 minutes before behavioral testing in the EPM or NSF (Figure 6A). In the EPM, acute systemic injection of RS67333 and diazepam induced a fast anxiolytic-like effect when compared with vehicle and fluoxetine administration in BALB/cJRj mice. RS67333 and diazepam increased time and the percent time spent in the open arms (one-way ANOVA, **p<0.01 *vs.* vehicle group, Figure 1C and inset). It is unlikely that this effect was the consequence of a change in locomotor activity, since no change in this parameter was detected and the ratio of ambulatory distance in the open arms divided by total distance was significantly increased for both drugs (one-way ANOVA, **p<0.01 *vs.* vehicle group, Figure 6D and inset).

To assess the selectivity of RS67333-induced anxiolytic-like effects, we also tested whether the 5-HT₄R antagonist GR125487 (1 mg/kg, i.p.) influenced the response of RS67333 (1.5 mg/kg) on anxiety-like behavior. Here, GR125487 was administered 15 minutes before RS administration (Figure 11A). In the EPM, GR125487 administration prevented RS67333-induced increase in time and the percent time spent in the open arms, or the increase of ambulatory distance in the open arms divided by total distance without affecting locomotor activity (one-way ANOVA, *p<0.05, **p<0.01 or ##p<0.01 *vs.* vehicle group and vs. RS67333 group respectively, Figures 11B-11C and insets).

In another anxiety-related test, the NSF, we found that RS67333 and diazepam, unlike fluoxetine that induced an anxiogenic-like effect, decreased the latency to feed when compared with saline administration (Kaplan-Meier survival analysis and one-way ANOVA, **p<0.01 *vs.* vehicle group, inset, Figures 6E-6F and inset) without affecting the home-cage food consumption. Moreover, GR125487 occluded the effect of RS67333 on the latency to feed without affecting food consumption (Kaplan-Meier survival analysis and one-way ANOVA, **p<0.01 or ##p<0.01 *vs.* vehicle group and vs. RS67333 group respectively (Figures 11D-11E and inset).

To further validate these results, we next tested the effect of RS67333, fluoxetine and diazepam in another anxiety-related test, the Open Field (OF) (Figures 12A-12C). We found that, unlike fluoxetine, acute RS67333 and diazepam increase the percent time spent in Center (one-way ANOVA, *p<0.05, **p<0.01, Figures 12B) without affecting locomotor activity. Indeed, the ratio of ambulatory distance in the center divided by total distance was significantly increased for diazepam and a trend was observed for RS67333 (one-way ANOVA, *p<0.05, **p<0.01 *vs.* vehicle group, Figures 12C and inset). In summary, these data indicate that RS67333-induced fast anxiolytic-like effects through 5-HT₄R activation.

### Acute cortical 5-HT₄R activation induces fast anxiolytic-like effects.

Since 5-HT₄Rs are expressed in the mPFC (A27), a brain region involved in the physiopathology of mood disorders related to central 5-HT dysfunction (A19, A28, A29), we examined the contribution of 5-HT₄R activation in the mPFC to fast anxiolytic-like activity (Figure 6B). In the EPM, as observed with a systemic administration of diazepam (1.5 mg/kg), a local infusion of RS67333 (1 µg) significantly increased time and percent time spent in the open arms without affecting locomotion (one-way ANOVA, *p<0.05, **p<0.01 vs. vehicle group, Figure 6G and insets), as a significant increase in ratio of ambulatory distance in the open arms divided by total distance was observed (One Way ANOVA, *p<0.05 vs. vehicle group, Figures 6H). In the NSF, RS67333 and systemic administration of diazepam decreased latency to feed without affecting the home-cage food consumption (Kaplan-Meier survival analysis and one-way ANOVA, *p<0.05, **p<0.01 *vs.* vehicle group, Figures 6I-6J and inset) confirming the anxiolytic-like effects of 5-HT₄R activation in the mPFC.

### Serotonin from the dorsal raphe nucleus is involved in fast anxiolytic-like effects of acute RS67333 and diazepam administration.

Here, we set out to test whether an acute administration of RS67333 could induce persistent changes in serotonergic activity (Figure 7A). Indeed, we found that acute systemic administration of RS67333 (1,5 mg/kg) increased the discharge frequency of DRN 5-HT neurons by 63 % (Student's test, **p<0.01 *vs.* before RS6733, Figures 2B-2C).

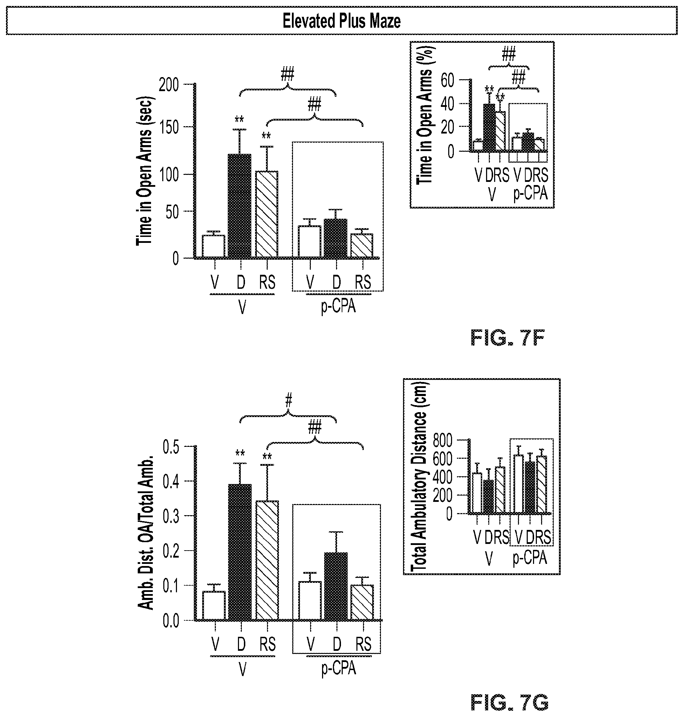

To further confirm that anxiolytic-like effects of mPFC 5-HT₄R stimulation depend on an intact 5-HT system, mice were pre-treated with p-CPA for 3 days before RS67333 (0.5 µg/side) or diazepam (1.5 µg/side) intra-mPFC infusion (Figure 7D). p-CPA induced an average decrease of 86 % in the 5-HT content in the mPFC of vehicle mice (two-way ANOVA, #p<0.05, ##p<0.01 vs. appropriate vehicle group, Figure 7E). Acute intra-mPFC infusion with RS67333 or diazepam increased time, percent time spent in the open arms of the EPM, and the ratio of ambulatory distance in the open arms / total distance were abolished in 5-HT-depleted p-CPA mice (two-way ANOVA, **p<0.01 vs. vehicle/vehicle or or #p<0.05, ##p<0.01 vs. vehicle/appropriate group, Figures 7F-7G). p-CPA -induced 5-HT depletion did not affect locomotor activity (Inset Figure 7G). These results point out the critical role of the 5-HT neurotransmission in fast anxiolytic-like effects. In summary, RS67333, as suggested previously (A29), and diazepam act on 5-HT function through a modulation of the mPFC.

### Fast anxiolytic-like effects of 5-HT₄R agonist recruit medial prefrontal cortex-brainstem neural circuit.

While emotional behaviors are mediated by mPFC pyramidal neurons projecting to the DRN (A22), no direct evidence suggests that these projections are involved in anxiolytic-like effects. Thus, using optogenetic strategies, we began an examination of the specific contribution of the incoming cortical glutamatergic terminals in the DRN to fast anxiolytic-like effects induced by acute diazepam or RS67333 administration.

First, to target opsin expression selectively to cortical glutamatergic projections to the DRN, we employed an AAV5-CaMKII*α*-virus, that specifically expresses ChR2 in mPFC pyramidal cell terminals in the DRN (Figures 8A-8B). AAV5-CaMKII*α*-ChR2-eYFP injected mice were compared to AAV5-CaMKII-eYFP-injected control. In the EPM, illumination of mPFC projections in the DRN in CamKII-ChR2-injected BALB/cJRj mice induced a significant increase in time, percent time or change in the distribution of time spent in the open arms in comparison to light OFF and also to control group (two-way ANOVA, **p<0.01 or ##<p0.01 vs. CaMKII*α*-ChR2-eYFP or CaMKII-eYFP respectively during light ON, Figures 8C and insets). It is unlikely that this effect was the consequence of a change in locomotor activity, as even if the total ambulatory distance was decrease in CamKII-ChR2-injected mice during light ON, the ratio of ambulatory distance in open arms divided by total distance was increased in comparison to light OFF (two-way ANOVA, **p<0.01 or ##<p0.01 vs. CaMKII*α*-ChR2-eYFP or CaMKII-eYFP respectively during light ON, Figures 3D and inset). To further confirm that the fast anxiolytic-like effects of the 5-HT₄R agonist recruit the mPFC-brainstem neural circuit, we evaluated the behavioral consequences of an optogenetic stimulation of mPFC terminals in the DRN in the OF paradigm (Figures 13A-13C). We found that illumination of mPFC projections in DRN in CamKII-ChR2-injected BALB/cJRj mice induced a significant increase in time spent in the center in comparison to light OFF and also to control group (two-way ANOVA, **p<0.01 *versus* during light OFF, ##p<0.01 *versus* eYFP during light ON, Figures 13B and inset). It is unlikely that this effect was the consequence of a change in locomotor activity, as the total ambulatory distance was not affected and the ratio of ambulatory distance in center divided by total distance was increased in comparison to light OFF (two-way ANOVA, **p<0.01 or ##<p0.01 vs. CaMKII*α*-ChR2-eYFP or CaMKII-eYFP during light ON, Figure 13C and inset).

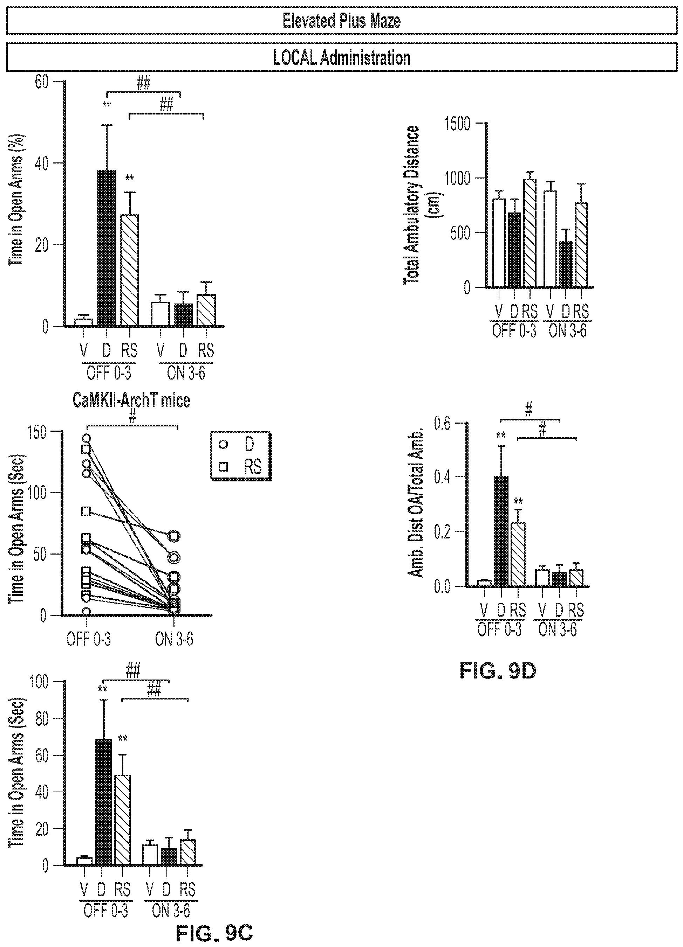

Next, we probed the effects of optogenetic inhibition of mPFC projections to the DRN after cortical infusion of RS67333 or diazepam (Figure 9A). AAV5-CaMKII-ArchT injected mice in the mPFC showed robust expression of ArchT-GFP in mPFC but also in cortical glutamatergic terminals in the DRN (Figures 9B). In the EPM, RS67333 (0.5 µg/side) and diazepam (1.5 µg/side) injected in the mPFC of CamKII-ArchT mice increased significantly time, percent time or change in the distribution of time spent in the open arms during light OFF and was reversed during a 3-min green light illumination (70 ±8 % and 85 ±5 % of inhibition for RS67333 and diazepam respectively, two-way ANOVA, **p<0.01 or ##<p0.01 vs. CaMKII*α*-ArchT or CaMKII-GFP respectively during light ON for appropriate treatment Figures 9C and inset). Similarly, acute RS67333 and diazepam administration increased the ratio of ambulatory distance in open arms divided by total distance during light OFF in comparison to controls and was blocked during light ON, confirming the anxiolytic effects of both drugs (two-way ANOVA, **p<0.01 vs. CaMKII*α*-ArchT or #<p0.05 CaMKII-GFP respectively during light OFF for appropriate treatment, Figure 9D). No changes in ambulatory distance were observed during light OFF or light ON (Figures 9D and inset). These results were also confirmed in the OF (Figure 14A-14C). Indeed, RS67333 (0.5 µg/side) and diazepam (1.5 µg/side) infused in mPFC of CamKII-ArchT mice, induced anxiolytic effects that were blocked by optogenetic inhibition of mPFC terminals in the DRN. Specifically, RS67333 and diazepam injected in the mPFC of CamKII-ArchT mice increased significantly the time spent in the center during light OFF, and this effect was reversed during a 3-min green light illumination (85 ±20 % and 80 ±22 % of inhibition for RS67333 and diazepam respectively, two-way ANOVA, *p<0.05, **p<0.01 or #p<0.05, ##<p0.01 *vs.* CaMKII*α*-ArchT or CaMKII-GFP during light ON, Figures 14B-14C and insets). These data support that the mPFC-DRN neural circuit is recruited for both RS67333 and diazepam to induce anxiolytic-like effects.

We then proceeded to investigate whether mPFC terminals targeting to the DRN circuit could be sufficient for fast anxiolytic-like effects induced by acute systemic diazepam or RS67333 treatment (Figures 9A-9B). In the EPM paradigm, as previously shown, acute systemic administration of RS67333 (1.5 mg/kg) or diazepam (1.5 mg/kg) in CaMKII-ArchT -mPFC injected mice increased time, percent time spent in open arms and ratio ambulatory distance in open arms divided by total distance eliciting an anxiolytic-like effect without affecting locomotor activity during the OFF epoch (two-way ANOVA, **p<0.01 vs. vehicle group during light OFF, Figures 9E-9F and inset). During a 3-minute green-light illumination of cortical glutamatergic terminals in the DRN, even though acute RS67333 or diazepam induced an anxiolytic-like effect, the size of the effect was attenuated in comparison to light OFF (two-way ANOVA, §§p<0.01 *vs.* vehicle group during light ON, #p<0.05 *vs.* appropriate group during light OFF, Figures 9E-9F and inset). Indeed, a significant decrease of 24 % for RS67333 and a 17 % decrease (p<0.09), for diazepam, in time spent in the open arms was observed in the EPM. The distribution of time spent in the open arms and the decrease in ambulatory distance in open arms divided by total distance between light ON after acute systemic RS67333 or diazepam administration confirmed that inhibition of mPFC pyramidal cell terminals in the DRN significantly reduced anxiolytic effects of these two compounds (two-way ANOVA, #p<0.05, ##p<0.01 vs. appropriate group during light OFF, Figures 9E-9F and inset). Overall, these data suggest that the mPFC terminals in the DRN are recruited for fast anxiolytic effects of RS67333 and diazepam.

### Evaluation of the long-term activity of a 5-HT₄ receptor agonist in BALB/cJRj mice

Having shown that RS 67333 was provided with prophylactic properties, we subsequently sought to gain knowledge of this molecule in the long term, i.e. to find out whether the anxiolytic response brought about by RS 67333 is sustainable over time. In order to achieve this, the BALB/cJRj mice were systemically injected with a single dose of RS 67333 (1.5 mg/kg) or diazepam (1.5 mg/kg), 45 minutes before performing the Splash Test. The following day, the mice underwent the EPM without having received another dose of RS, and then at the open field 24 hours later, and, finally, to NSF 24 hours after the open field (Fig. 15A).

As anticipated, RS 67333 increased the grooming time (t = 2.294; p<0.05) in the Splash Test, without affecting the number of episodes (t = 1.546; p = 0.1531) (Fig. 15B-15C), following single administration. We have not identified the anxiolytic effects expected of RS 67333 in the EPM (t = 0.4990; p = 0.6286) (Fig. 15D-15E), 24 hours after the injection. However, the RS 67333 has increased the time spent in the center of the OF (t = 1.924; p<0.05), without affecting the ratio of the distance in the center to the total walking distance (t = 1.281; p = 2292) (Fig. 15F-15G), 48 hours after the injection, and reduced the lag for feeding in the NSF (t = 2.520; p<0.05), without affecting the consumption of food in a familiar environment (t = 0.2203; p = 0.4151) (Fig. 15H-15I), 72 hours after the injection. Since diazepam does not exhibit any antidepressant activity, it is therefore normal to not identify any effect in the Splash Test. We likewise have not been able to observe any long-term activity for diazepam either.

Therefore, although this study should be repeated, the RS could have persistent effects up to 72 hours after injection.

### DISCUSSION

### Acute 5-HT₄R activation and fast anxiolytic-like effects.

Our study provides evidence in BALB/cJRj, a mouse strain with a high anxiety level, that 5-HT₄R stimulation induced fast anxiolytic-like effects similar to diazepam in three different anxiety paradigms, namely EPM, NSF and OF. Interestingly, unlike RS67333, acute systemic administration of fluoxetine did not affect anxiety-like behavior, confirming previous observations (A30). These data also suggest that rapid anxiolytic-like activity requires selective activation of some key postsynaptic receptors such as the 5-HT₄R or the 5-HT_{1A}R (A31) more than a global increase in 5-HT neurotransmission.

### Involvement of mPFC-DRN circuit for the 5-HT₄R activation-mediated rapid anxiolytic-like activity.

In both humans and also in rodents, 5-HT₄R are mainly localized in limbic areas involved in psychiatric disorders, such as anxiety (A27, A32). We explored the role of the 5-HT₄R activation expressed in mPFC in fast anxiolytic-like effects. Indeed, 5-HT₄R is expressed in excitatory pyramidal neurons of the mPFC, a region showing glutamate dysregulation in patients with generalized anxiety disorders (A33, A34). Interestingly, fast anxiolytic-like effects observed after acute systemic administration of RS67333 were reproduced by an acute infusion of this 5-HT₄R agonist in mPFC. Indeed, after a bilateral infusion with RS67333, similar to diazepam, there was an increase in time spent in open arms in the EPM and a decrease in latency to feed was observed in the NSF. Our results are in line with previous results showing that 5-HT₄R overexpression in the mPFC yields a robust anxiolytic-like behavioral phenotype (A7, A19).

Anatomical studies have shown that the prelimbic/cingulate cortices also project abundantly to DRN 5-HT neurons (A35). This connectivity has attracted great interest as a potential circuit involved in modulating stress and depressive behaviors (A22). For example, stressor exposure to inescapable shock in rodents increased cFos expression in 5-HT neurons in the middle and caudal regions of the DRN, suggesting an increased neural activation of this structure in anxiogenic situations (A36). There is also evidence that 5-HT₄R activation in the mPFC controls the firing rate of midbrain serotonergic neurons via descending inputs (A19, A29, A37). A reduction in the spontaneous activity of 5-HT neurons and a decrease in 5-HT content in the DRN of 5-HT₄R-null mice were observed (A38). Conversely, administration of RS67333 in rat, at different time points, drives effects on DRN 5-HT neuronal activity (A19, A29, A39), and increase 5-HT release at projection sites (A40). We showed that acute systemic injection of RS67333 enhances the firing rate of DRN 5-HT neurons in mice, suggesting that the fast anxiolytic-like activity of the 5-HT₄R agonist is dependent on activation of this neuronal population, despite the fact that DRN does not express 5-HT₄R (A27). In fact, the fast onset of action of the 5-HT₄R agonist could be a consequence of an increase in serotonergic output to projection areas including the mPFC (A19, A41). These results are supported by the fact that the depletion of whole brain 5-HT content by pre-treatment with systemic p-CPA prevented RS67333-induced anxiolytic-like phenotype, while p-CPA alone did not affect behavior as previously reported (A42). Interestingly, despite different pharmacological targets, the 5-HT₄R agonist and BZD shared a common anxiolytic-like activity with similar efficiency suggesting activation of 5-HT neurotransmission and possibly common neural circuit recruitment by these two drugs. Under our experimental conditions, anxiolytic-like activity of intra-mPFC infusion of diazepam was blocked by a pre-treatment with p-CPA, suggesting a participation of 5-HT system in this activity.

Knowing that 5-HT-producing neurons in the DRN are preferentially modulated by monosynaptic glutamatergic inputs from the mPFC (A35), we used a CamKII promoter to target glutamatergic pyramidal neurons and evaluate this neuronal brain circuit involved in fast anxiolytic-like effects. Illumination of ChR2-expressing terminals of mPFC neurons projecting to the DRN of BALB/cJRj mice induced an anxiolytic-like effect, measured as an increase in the time spent in the open arms, an effect similarly to intra-mPFC RS67333 and diazepam. This result emphasizes the role of the mPFC-brainstem DRN neural circuit in fast anxiolytic-like effects. Multiple studies have implicated the mPFC-DRN circuit in the regulation of behavioral response to aversive challenges. For example, deep brain stimulation (DBS) in the ventromedial PFC of chronic social defeat (SD) mice restored social interaction (A43). At the same time, 1-hour DBS in naive mice and chronic DBS in chronic SD mice, increased cFos immunoreactivity in the DRN and reversed SD-induced hypoexcitability of DRN 5-HT neurons, respectively (A43). In SD mice, chronic photoactivation of mPFC pyramidal cells increased the time spent in the open arms in the EPM (A23), whereas stimulation had no effect on anxiety-related behavior in non-stressed animals (A23, A44). Conversely, photosilencing mPFC terminals in the DRN prevented a decrease in social interaction in SD mice, suggesting a contributing role in anxiety-like behavior (A24).

To ensure that the behavioral response to local 5-HT₄R agonist infusion is in line with the idea that glutamatergic mPFC pyramidal neurons are mediators of 5-HT₄R agonist -driven effects on DRN 5-HT neuronal activity, we optogenetically silenced incoming cortical glutamatergic terminals in the DRN. Inhibition of these projections reversed the anxiolytic-like behavior induced by intra-mPFC RS67333 and diazepam administration, confirming that the cortex-raphe circuit recruitment is essential for rapid anxiolytic-like activity (A7). Interestingly, in line with our results, a recent study show that rescuing the mPFC-5-HT₄R expression in 5-HT₄R KO mice partly reduced stress levels (A28). Although these results were not surprising for the 5-HT₄R agonist, they were unexpected for diazepam. However, cortical GABA_{A} receptor activation through intra-mPFC muscimol infusion (a direct agonist of GABA_{A} receptor) has been proposed to attenuate anxiety-related behavior in adult Wistar rats (A45). These findings indicate that despite different pharmacological targets, 5-HT₄R agonist and BZD share common mechanisms to induce fast anxiolytic-like effect through prefrontal cortex-DRN brainstem neural circuit recruitment. Whether or not these glutamatergic projections might also regulate DRN activity via an effect on local interneurons should be investigated. Anatomical studies using viral anterograde tracing revealed that 5-HT neurons and GABA interneurons in the DRN receive excitatory inputs from the prelimbic part of the mPFC, with a larger proportion of inputs to DRN 5-HT neurons compared to GABAergic neurons (A35) suggesting that GABA interneurons influence may be secondary (A37).

In order to evaluate whether mPFC-DRN circuit recruitment is not only necessary but also sufficient in the fast anxiolytic-like activity related to 5-HT₄R activation, we investigated the consequences of optogenetic inhibition of the mPFC terminals in the DRN after acute systemic administration of diazepam and RS67333. Inhibition of cortical glutamatergic terminals in the DRN attenuates but does not prevent the anxiolytic effect induced by acute systemic administration with RS67333 or diazepam suggesting that other brain structures might also be involved in the fast anxiolytic-like activity of BZD and 5-HT₄R agonist. These results are not surprising since other circuits are also involved in anxiety-related behaviors such as the ventral hippocampus to prefrontal cortex (46), prefrontal cortex to basolateral amygdala (A47), basolateral amygdala to ventral hippocampus (A48) or DRN to bed nucleus of the stria terminalis (31) inputs (Figure 10). For instance, RS67333 administration into the basolateral amygdala, hippocampus, or nucleus basalis magnocellularis modulated also emotional memory formation and consolidation (A49-A51), suggesting that the anxiolytic-like effect of 5-HT₄R agonist might depend on these different limbic areas.

Interestingly, we found that silencing cortical glutamatergic terminals in the DRN attenuates also the anxiolytic-like activity of diazepam. Many other structures express the GABA_{A} receptor and the 5-HT₄R, but the mPFC-DRN circuit appears necessary but not sufficient for diazepam and RS67333-mediated fast anxiolytic-like activity. Future studies should examine how brain structures also involved in anxiety phenotype interact with the mPFC-DRN circuit for fast anxiolytic-like activity and also whether 5-HT₄R expression in the mPFC is responsible for fast anxiolytic-like effect of diazepam since diazepam have been shown to be dose-dependently inhibited by antagonists of the 5-HT₄R (A16).

Taken together, our study reveals the importance the mPFC-DRN circuit in mediating the fast anxiolytic-like effects of both 5-HT₄R agonists and BZD. Stimulating the 5-HT₄R in the mPFC or more generally the mPFC-brainstem DRN neural circuit facilitates anxiolytic effect and could represent an innovative and rapid onset therapeutic approach to treat anxiety. However, the use of 5-HT₄R agonists as a fast-acting anxiolytics may be hampered by the fact that the 5-HT₄R are expressed outside the central nervous system in the heart, gastrointestinal tract, adrenal gland, and urinary bladder (A52). It may be worth identifying other components of the mPFC-DRN circuits that are more specific and amenable to drug development.

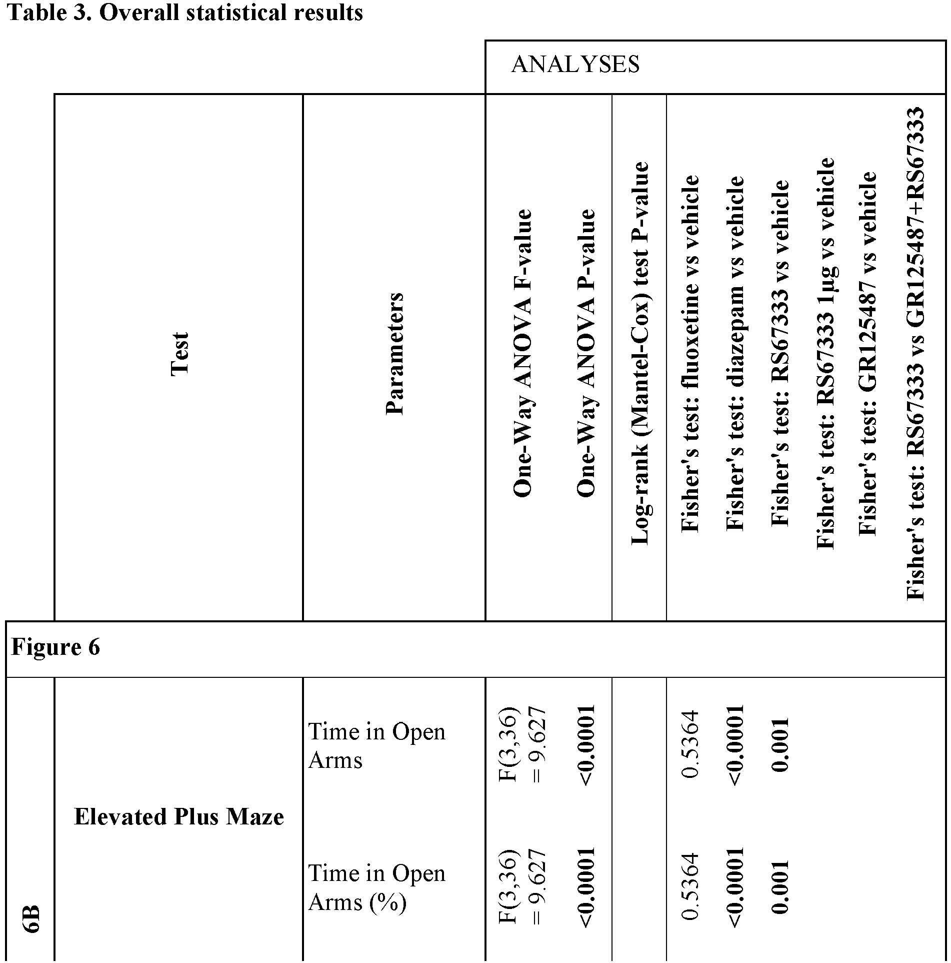

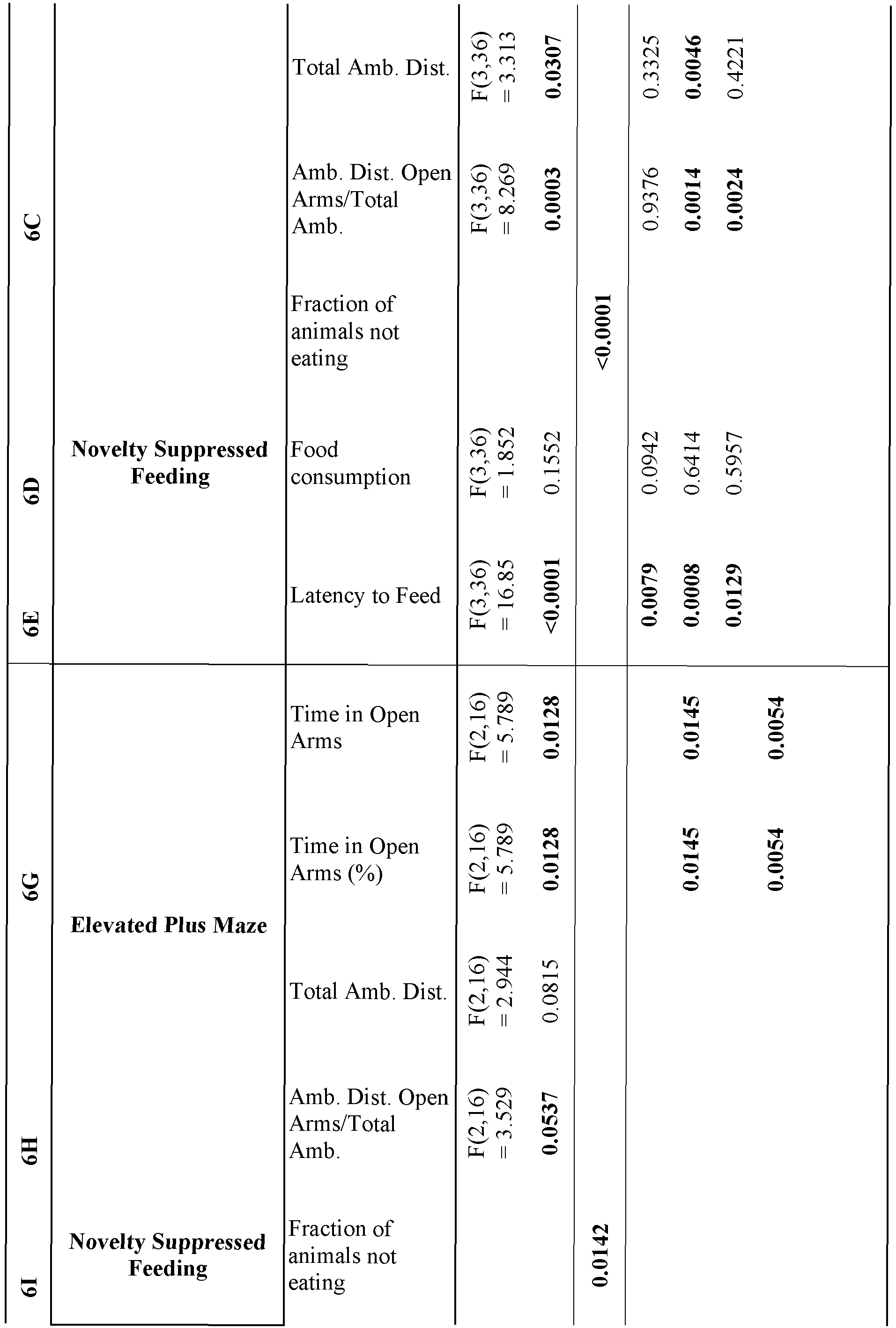

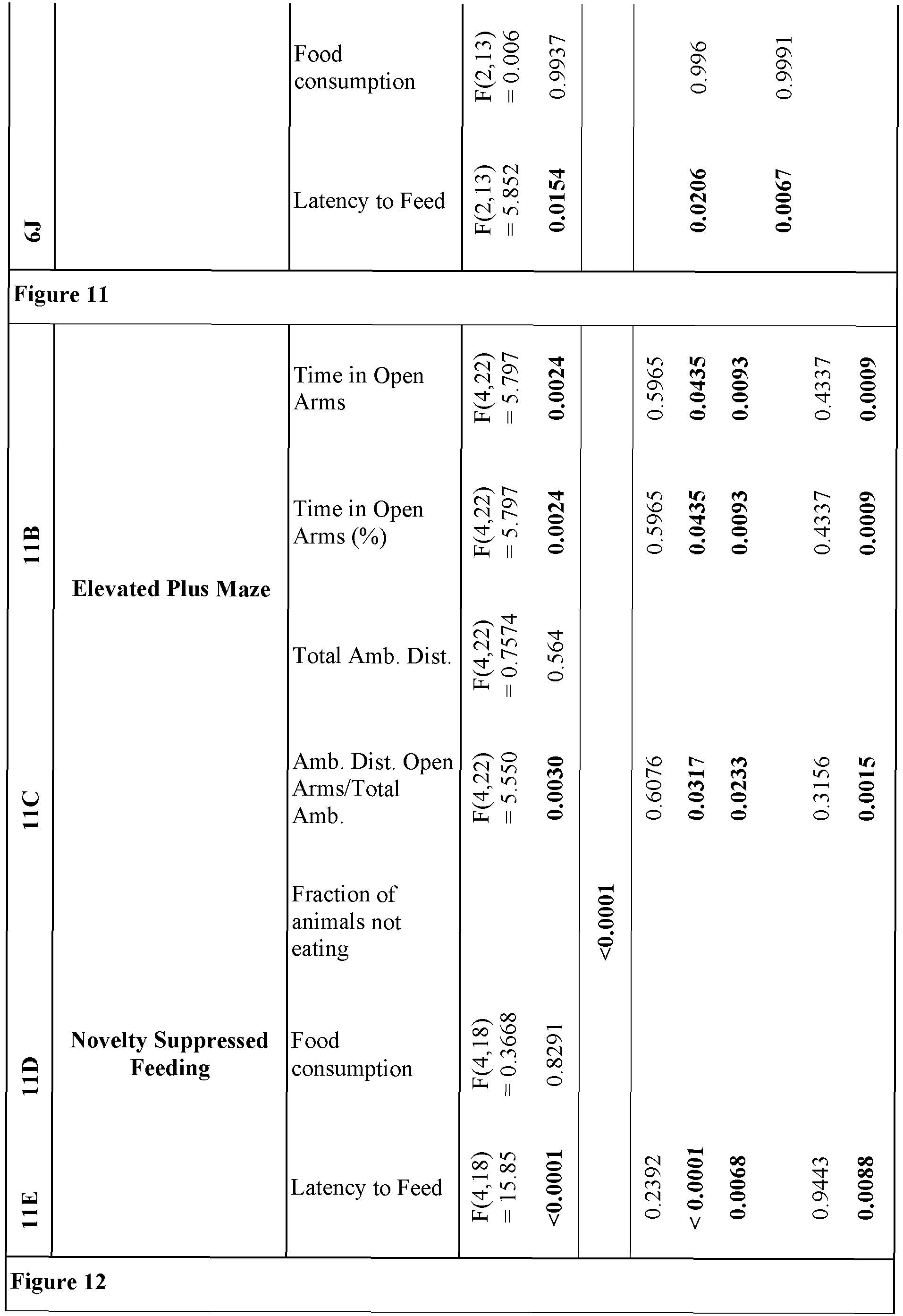

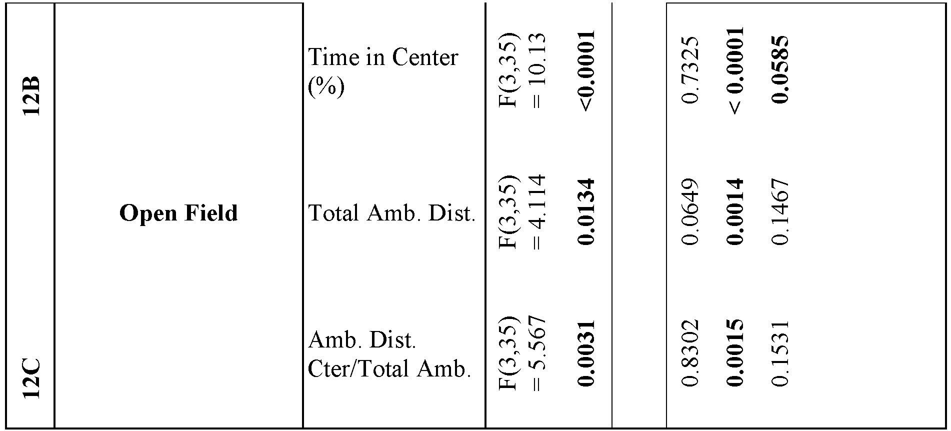

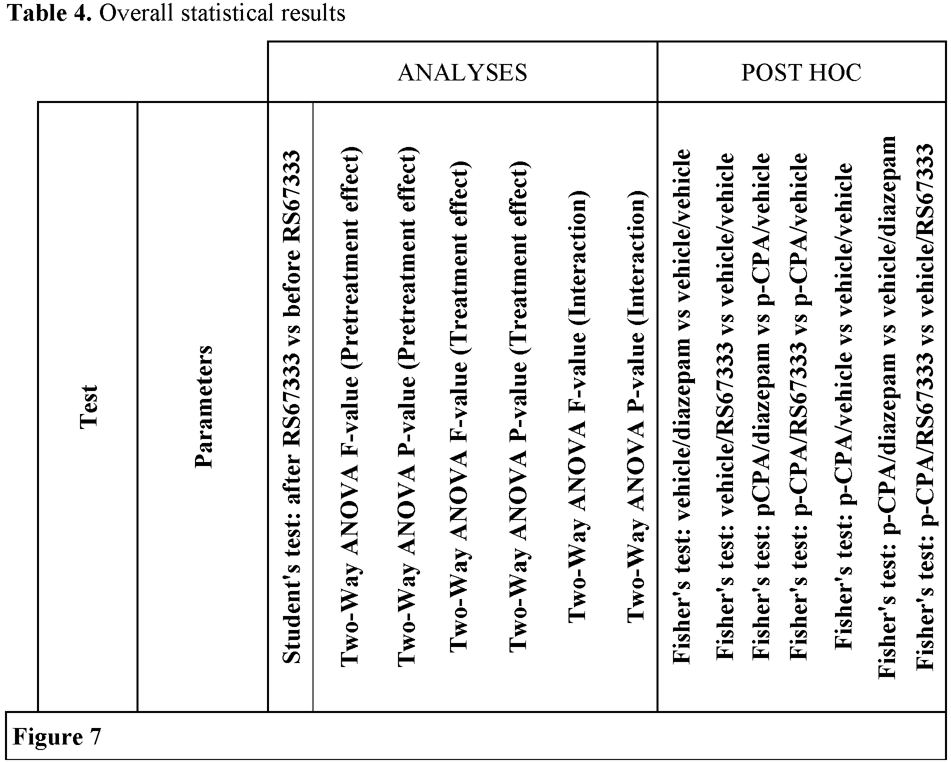

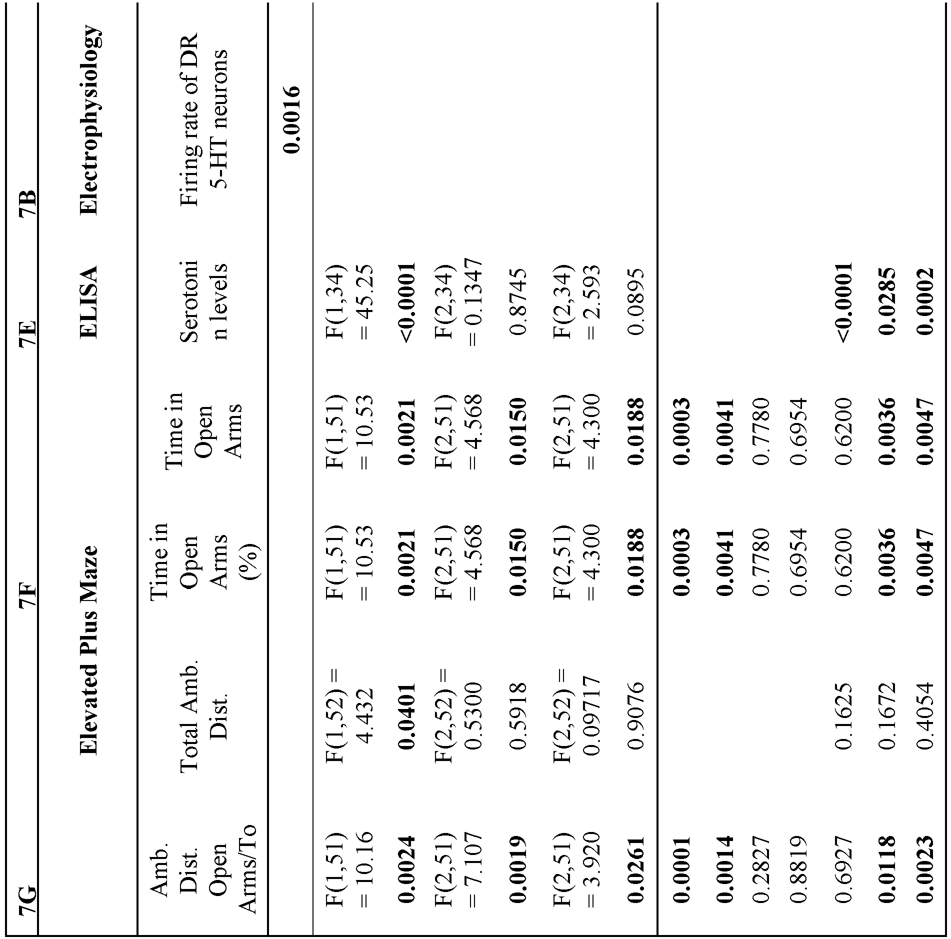

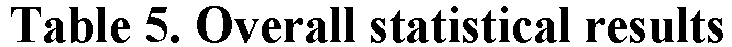

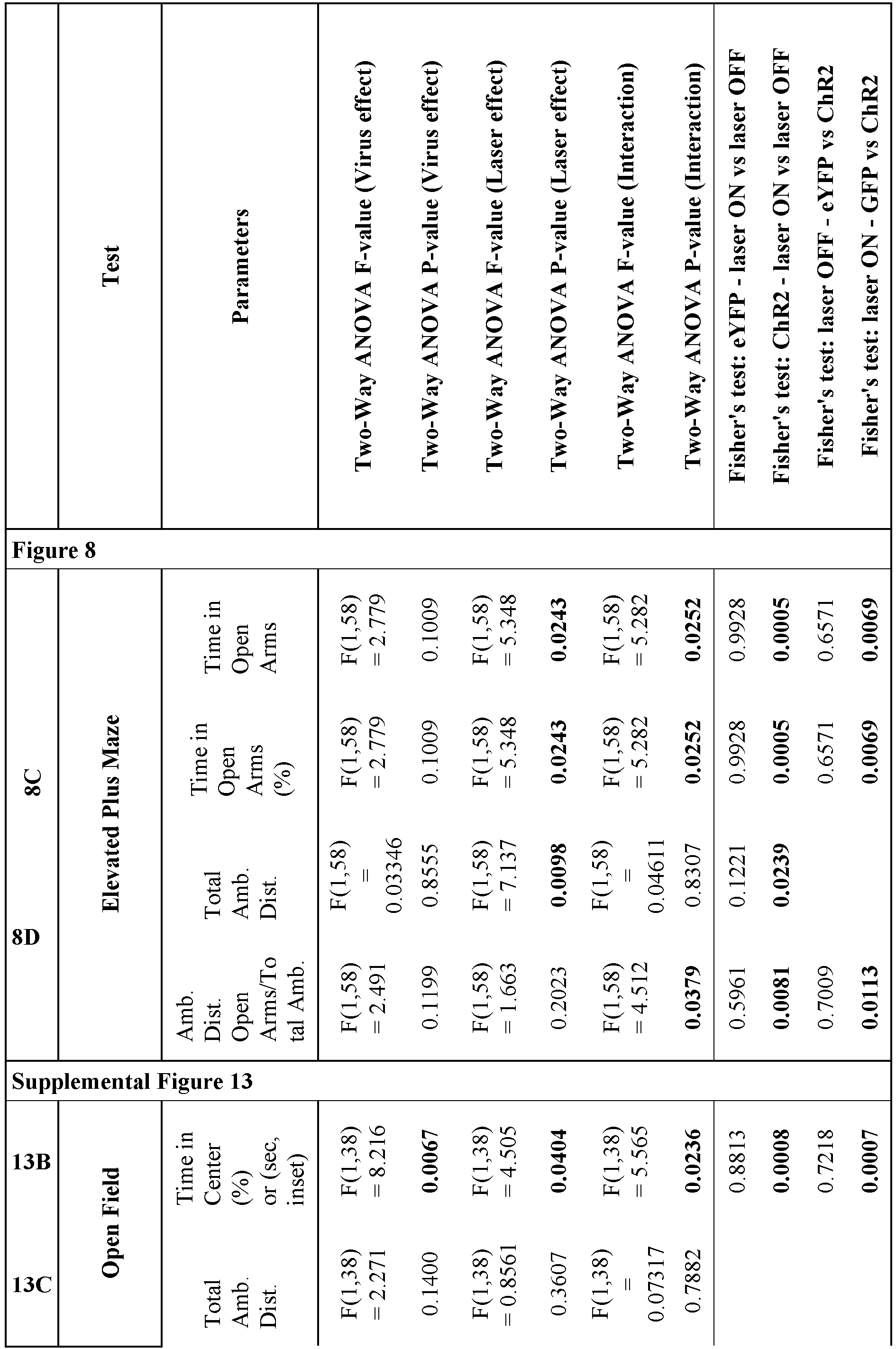

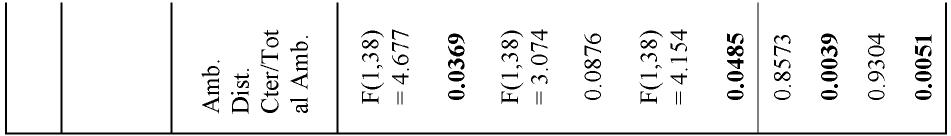

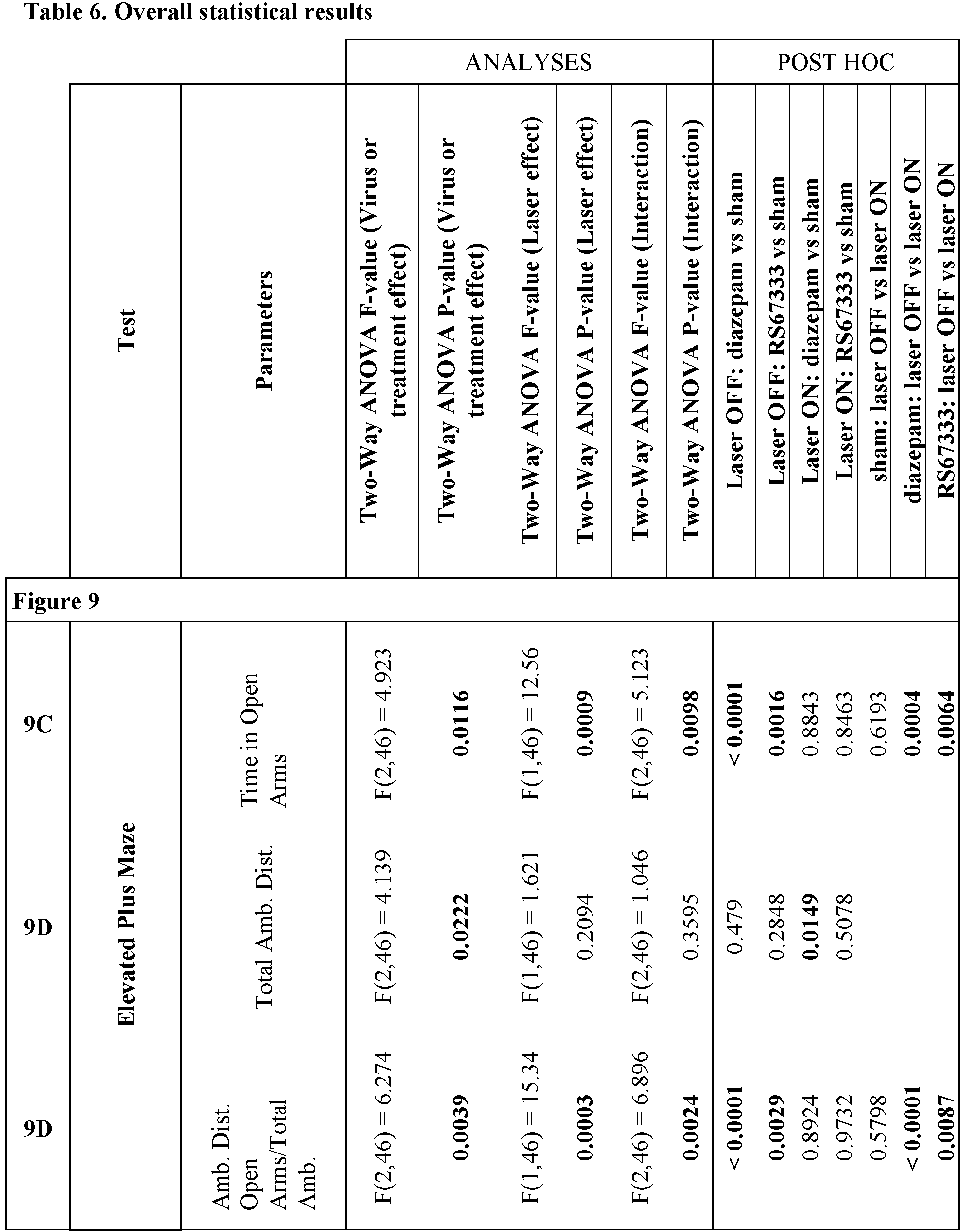

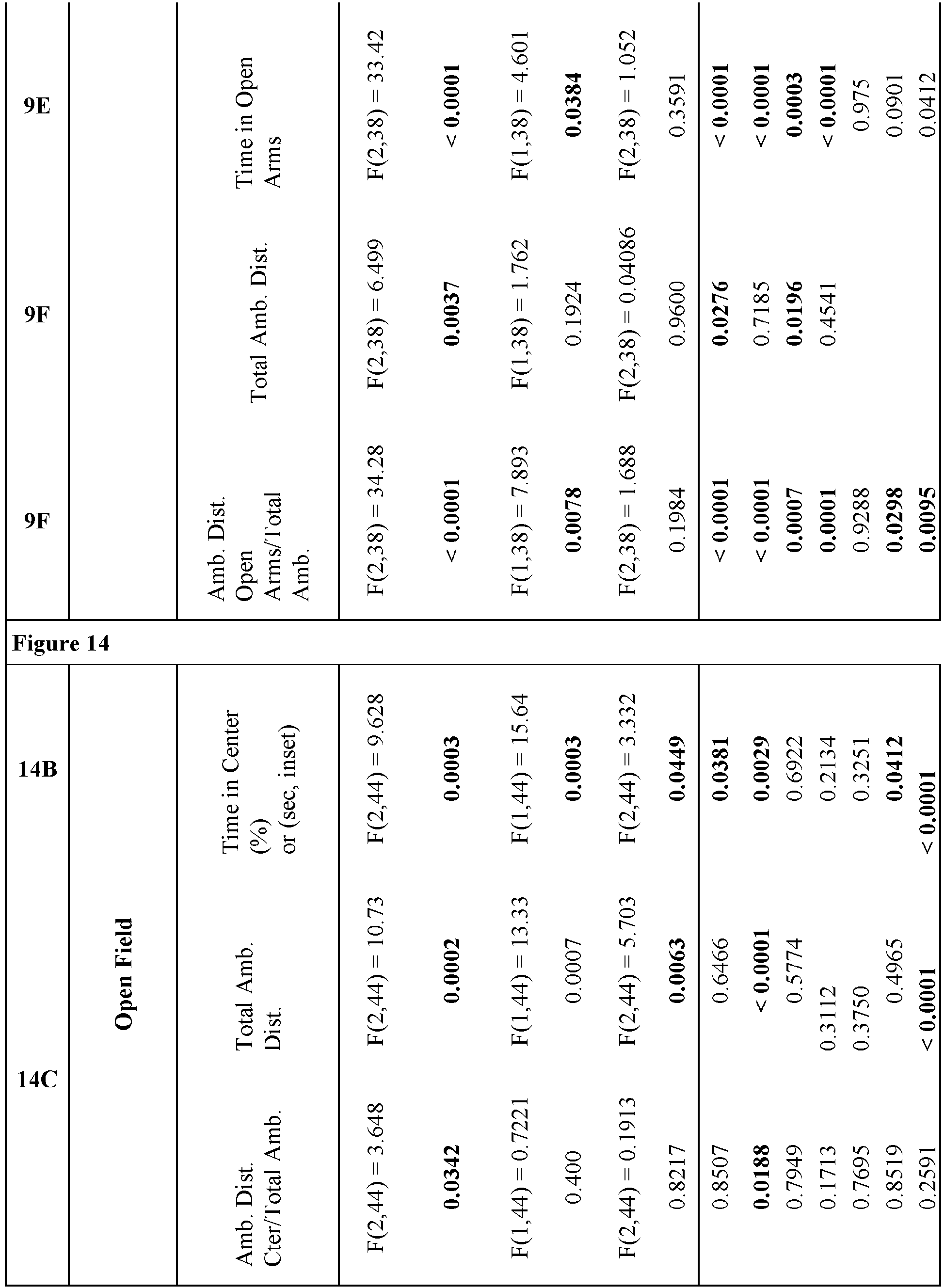

### REFERENCES

A1. Kheirbek MA, Klemenhagen KC, Sahay A, Hen R (2012): Neurogenesis and generalization: a new approach to stratify and treat anxiety disorders. Nat Neurosci. 15:1613-1620.
A2. Kessler RC, Chiu WT, Demler O, Merikangas KR, Walters EE (2005): Prevalence, severity, and comorbidity of 12-month DSM-IV disorders in the National Comorbidity Survey Replication. Arch Gen Psychiatry. 62:617-627*.*
A3. Klein E (2002): The role of extended-release benzodiazepines in the treatment of anxiety: a risk-benefit evaluation with a focus on extended-release alprazolam. J Clin Psychiatry. 63 Suppl 14:27-33.
A4. Bystritsky A (2006): Treatment-resistant anxiety disorders. Mol Psychiatry. 11:805-814.
A5. Mendez-David I, David DJ, Darcet F, Wu MV, Kerdine-Romer S, Gardier AM, et al. (2014): Rapid anxiolytic effects of a 5-HT(4) receptor agonist are mediated by a neurogenesis-independent mechanism. Neuropsychopharmacology. 39:1366-1378.
A6. Samuels BA, Mendez-David I, Faye C, David SA, Pierz KA, Gardier AM, et al. (2016): Serotonin 1A and Serotonin 4 Receptors: Essential Mediators of the Neurogenic and Behavioral Actions of Antidepressants. Neuroscientist. 22:26-45.
A7. Castello J, LeFrancois B, Flajolet M, Greengard P, Friedman E, Rebholz H (2017): CK2 regulates 5-HT4 receptor signaling and modulates depressive-like behavior. Mol Psychiatry*.*
A8. Pascual-Brazo J, Castro E, Diaz A, Valdizan EM, Pilar-Cuellar F, Vidal R, et al. (2012): Modulation of neuroplasticity pathways and antidepressant-like behavioural responses following the short-term (3 and 7 days) administration of the 5-HT(4) receptor agonist RS67333. Int J Neuropsychopharmacol. 15:631-643.
A9. Lucas G, Rymar VV, Du J, Mnie-Filali O, Bisgaard C, Manta S, et al. (2007): Serotonin(4) (5-HT(4)) receptor agonists are putative antidepressants with a rapid onset of action. Neuron. 55:712-725.
A10. Vidal R, Castro E, Pilar-Cuellar F, Pascual-Brazo J, Diaz A, Rojo ML, et al. (2014): Serotonin 5-HT4 receptors: A new strategy for developing fast acting antidepressants? Curr Pharm Des. 20:3751-3762.
A11. Rebholz H, Friedman E, Castello J (2018): Alterations of Expression of the Serotonin 5-HT4 Receptor in Brain Disorders. Int J Mol Sci. 19*.*
A12. Amigo J, Diaz A, Pilar-Cuellar F, Vidal R, Martin A, Compan V, et al. (2016): The absence of 5-HT4 receptors modulates depression- and anxiety-like responses and influences the response of fluoxetine in olfactory bulbectomised mice: Adaptive changes in hippocampal neuroplasticity markers and 5-HT1A autoreceptor. Neuropharmacology. 111:47-58.
A13. Bockaert J, Claeysen S, Compan V, Dumuis A (2004): 5-HT4 receptors. Curr Drug Targets CNS Neurol Disord. 3:39-51.
A14. Kennett GA, Bright F, Trail B, Blackburn TP, Sanger GJ (1997): Anxiolytic-like actions of the selective 5-HT4 receptor antagonists SB 204070A and SB 207266A in rats. Neuropharmacology. 36:707-712.
A15. Silvestre JS, Fernandez AG, Palacios JM (1996): Effects of 5-HT4 receptor antagonists on rat behaviour in the elevated plus-maze test. Eur J Pharmacol. 309:219-222*.*
A16. Costall B, Naylor RJ (1997): The influence of 5-HT2 and 5-HT4 receptor antagonists to modify drug induced disinhibitory effects in the mouse light/dark test. Br J Pharmacol. 122:1105-1118.
A17. Bell R, Duke AA, Gilmore PE, Page D, Begue L (2014): Anxiolytic-like effects observed in rats exposed to the elevated zero-maze following treatment with 5-HT2/5-HT3/5-HT4 ligands. Sci Rep. 4:3881.
A18. Abboussi O, Said N, Fifel K, Lakehayli S, Tazi A, El Ganouni S (2016): Behavioral effects of D3 receptor inhibition and 5-HT4 receptor activation on animals undergoing chronic cannabinoid exposure during adolescence. Metab Brain Dis. 31:321-327.
A19. Lucas G, Compan V, Charnay Y, Neve RL, Nestler EJ, Bockaert J, et al. (2005): Frontocortical 5-HT4 receptors exert positive feedback on serotonergic activity: viral transfections, subacute and chronic treatments with 5-HT4 agonists. Biol Psychiatry. 57:918-925.
A20. Davidson RJ (2002): Anxiety and affective style: role of prefrontal cortex and amygdala. Biol Psychiatry. 51:68-80.
A21. Riga D, Matos MR, Glas A, Smit AB, Spijker S, Van den Oever MC (2014): Optogenetic dissection of medial prefrontal cortex circuitry. Front Syst Neurosci. 8:230*.* A22. Warden MR, Selimbeyoglu A, Mirzabekov JJ, Lo M, Thompson KR, Kim SY, et al. (2012): A prefrontal cortex-brainstem neuronal projection that controls response to behavioural challenge. Nature. 492:428-432.
A23. Kumar S, Black SJ, Hultman R, Szabo ST, DeMaio KD, Du J, et al. (2013): Cortical control of affective networks. J Neurosci. 33:1116-1129.
A24. Challis C, Beck SG, Berton O (2014): Optogenetic modulation of descending prefrontocortical inputs to the dorsal raphe bidirectionally bias socioaffective choices after social defeat. Front Behav Neurosci. 8:43.
A25. Seo C, Guru A, Jin M, Ito B, Sleezer B, Ho YY, et al. (2019): Intense threat switches dorsal raphe serotonin neurons to a paradoxical operational mode. Science. 363:538-542.
A26. Dulawa SC, Holick KA, Gundersen B, Hen R (2004): Effects of chronic fluoxetine in animal models of anxiety and depression. Neuropsychopharmacology. 29:1321-1330.
A27. Vilaro MT, Cortes R, Mengod G (2005): Serotonin 5-HT4 receptors and their mRNAs in rat and guinea pig brain: distribution and effects of neurotoxic lesions. J Comp Neurol. 484:418-439.
A28. Jean A, Laurent L, Delaunay S, Doly S, Dusticier N, Linden D, et al. (2017): Adaptive Control of Dorsal Raphe by 5-HT4 in the Prefrontal Cortex Prevents Persistent Hypophagia following Stress. Cell Rep. 21:901-909.
A29. Moha ou Maati H, Bourcier-Lucas C, Veyssiere J, Kanzari A, Heurteaux C, Borsotto M, et al. (2016): The peptidic antidepressant spadin interacts with prefrontal 5-HT(4) and mGluR(2) receptors in the control of serotonergic function. Brain Struct Funct. 221:21-37.
A30. David DJ, Klemenhagen KC, Holick KA, Saxe MD, Mendez I, Santarelli L, et al. (2007): Efficacy of the MCHR1 antagonist N-[3-(1-{[4-(3,4-difluorophenoxy)phenyl]methyl}(4-piperidyl))-4-methylphenyl]-2-m ethylpropanamide (SNAP 94847) in mouse models of anxiety and depression following acute and chronic administration is independent of hippocampal neurogenesis. J Pharmacol Exp Ther. 321:237-248.
A31. Garcia-Garcia AL, Canetta S, Stujenske JM, Burghardt NS, Ansorge MS, Dranovsky A, et al. (2017): Serotonin inputs to the dorsal BNST modulate anxiety in a 5-HT1A receptor-dependent manner. Mol Psychiatry*.*
A32. Waeber C, Sebben M, Nieoullon A, Bockaert J, Dumuis A (1994): Regional distribution and ontogeny of 5-HT4 binding sites in rodent brain. Neuropharmacology. 33:527-541.
A33. Paulesu E, Sambugaro E, Torti T, Danelli L, Ferri F, Scialfa G, et al. (2010): Neural correlates of worry in generalized anxiety disorder and in normal controls: a functional MRI study. Psychol Med. 40:117-124.
A34. Wang Y, Chai F, Zhang H, Liu X, Xie P, Zheng L, et al. (2016): Cortical functional activity in patients with generalized anxiety disorder. BMC Psychiatry. 16:217*.*
A35. Weissbourd B, Ren J, DeLoach KE, Guenthner CJ, Miyamichi K, Luo L (2014): Presynaptic partners of dorsal raphe serotonergic and GABAergic neurons. Neuron. 83:645-662.
A36. Grahn RE, Will MJ, Hammack SE, Maswood S, McQueen MB, Watkins LR, et al. (1999): Activation of serotonin-immunoreactive cells in the dorsal raphe nucleus in rats exposed to an uncontrollable stressor. Brain Res. 826:35-43.
A37. Lucas G (2009): Serotonin receptors, type 4: a new hope? Curr Drug Targets. 10:1085-1095.
A38. Conductier G, Dusticier N, Lucas G, Cote F, Debonnel G, Daszuta A, et al. (2006): Adaptive changes in serotonin neurons of the raphe nuclei in 5-HT(4) receptor knock-out mouse. Eur J Neurosci. 24:1053-1062.
A39. Etiévant A, Lambas-Senas L, Abrial E, Bétry C, Haddjeri N, Lucas G (2011): Connection re-established: neurotransmission between the medial prefrontal cortex and serotonergic neurons o ers perspectives for fast antidepressant action. Neuropsychiatry. 1:165-177.
A40. Ge J, Barnes NM (1996): 5-HT4 receptor-mediated modulation of 5-HT release in the rat hippocampus in vivo. Br J Pharmacol. 117:1475-1480.
A41. Lucas G, Debonnel G (2002): 5-HT4 receptors exert a frequency-related facilitatory control on dorsal raphe nucleus 5-HT neuronal activity. Eur J Neurosci. 16:817-822.
A42. du Jardin KG, Liebenberg N, Muller HK, Elfving B, Sanchez C, Wegener G (2016): Differential interaction with the serotonin system by S-ketamine, vortioxetine, and fluoxetine in a genetic rat model of depression. Psychopharmacology (Berl). 233:2813-2825.
A43. Veerakumar A, Challis C, Gupta P, Da J, Upadhyay A, Beck SG, et al. (2014): Antidepressant-like effects of cortical deep brain stimulation coincide with pro-neuroplastic adaptations of serotonin systems. Biol Psychiatry. 76:203-212.
A44. Covington HE, 3rd, Lobo MK, Maze I, Vialou V, Hyman JM, Zaman S, et al. (2010): Antidepressant effect of optogenetic stimulation of the medial prefrontal cortex. J Neurosci. 30:16082-16090.
A45. Solati J, Hajikhani R, Golub Y (2013): Activation of GABAA receptors in the medial prefrontal cortex produces an anxiolytic-like response. Acta Neuropsychiatr. 25:221-226.
A46. Padilla-Coreano N, Bolkan SS, Pierce GM, Blackman DR, Hardin WD, Garcia-Garcia AL, et al. (2016): Direct Ventral Hippocampal-Prefrontal Input Is Required for Anxiety-Related Neural Activity and Behavior. Neuron. 89:857-866.
A47. Vialou V, Bagot RC, Cahill ME, Ferguson D, Robison AJ, Dietz DM, et al. (2014): Prefrontal cortical circuit for depression- and anxiety-related behaviors mediated by cholecystokinin: role of DeltaFosB. J Neurosci. 34:3878-3887.
A48. Felix-Ortiz AC, Beyeler A, Seo C, Leppla CA, Wildes CP, Tye KM (2013): BLA to vHPC inputs modulate anxiety-related behaviors. Neuron. 79:658-664.
A49. Chegini HR, Nasehi M, Zarrindast MR (2014): Differential role of the basolateral amygdala 5-HT3 and 5-HT4 serotonin receptors upon ACPA-induced anxiolytic-like behaviors and emotional memory deficit in mice. Behav Brain Res. 261:114-126.
A50. Orsetti M, Dellarole A, Ferri S, Ghi P (2003): Acquisition, retention, and recall of memory after injection of RS67333, a 5-HT(4) receptor agonist, into the nucleus basalis magnocellularis of the rat. Learn Mem. 10:420-426.
A51. Nasehi M, Tabatabaie M, Khakpai F, Zarrindast MR (2015): The effects of CA1 5HT4 receptors in MK801-induced amnesia and hyperlocomotion. Neurosci Lett. 587:73-78. A52. Tonini M, Pace F (2006): Drugs acting on serotonin receptors for the treatment of functional GI disorders. Dig Dis. 24:59-69.
A-S1. Mendez-David I, David DJ, Darcet F, Wu MV, Kerdine-Romer S, Gardier AM, et al. (2014): Rapid anxiolytic effects of a 5-HT(4) receptor agonist are mediated by a neurogenesis-independent mechanism. Neuropsychopharmacology. 39:1366-1378.
A-S2. Chegini HR, Nasehi M, Zarrindast MR (2014): Differential role of the basolateral amygdala 5-HT3 and 5-HT4 serotonin receptors upon ACPA-induced anxiolytic-like behaviors and emotional memory deficit in mice. Behav Brain Res. 261:114-126.
A-S3. McMahon LR, Cunningham KA (1999): Antagonism of 5-hydroxytryptamine(4) receptors attenuates hyperactivity induced by cocaine: putative role for 5-hydroxytryptamine(4) receptors in the nucleus accumbens shell. J Pharmacol Exp Ther. 291:300-307.
A-S4. Bockaert J, Claeysen S, Compan V, Dumuis A (2004): 5-HT4 receptors. Curr Drug Targets CNS Neurol Disord. 3:39-51.
A-S5. Eglen RM, Bonhaus DW, Johnson LG, Leung E, Clark RD (1995): Pharmacological characterization of two novel and potent 5-HT4 receptor agonists, RS 67333 and RS 67506, in vitro and in vivo. Br J Pharmacol. 115:1387-1392.
A-S6. David DJ, Klemenhagen KC, Holick KA, Saxe MD, Mendez I, Santarelli L, et al. (2007): Efficacy of the MCHR1 antagonist N-[3-(1-{[4-(3,4-difluorophenoxy)phenyl]methyl}(4-piperidyl))-4-methylphenyl]-2-m ethylpropanamide (SNAP 94847) in mouse models of anxiety and depression following acute and chronic administration is independent of hippocampal neurogenesis. J Pharmacol Exp Ther. 321:237-248.
A-S7. Petit-Demouliere B, Masse F, Cogrel N, Hascoet M, Bourin M (2009): Brain structures implicated in the four-plate test in naive and experienced Swiss mice using injection of diazepam and the 5-HT2A agonist DOI. Behav Brain Res. 204:200-205.
A-S8. Redrobe JP, Bourin M, Colombel MC, Baker GB (1998): Dose-dependent noradrenergic and serotonergic properties of venlafaxine in animal models indicative of antidepressant activity. Psychopharmacology (Berl). 138:1-8.
A-S9. Pham TH, Mendez-David I, Defaix C, Guiard BP, Tritschler L, David DJ, et al. (2017): Ketamine treatment involves medial prefrontal cortex serotonin to induce a rapid antidepressant-like activity in BALB/cJ mice. Neuropharmacology. 112:198-209.
A-S10.Bi LL, Wang J, Luo ZY, Chen SP, Geng F, Chen YH, et al. (2013): Enhanced excitability in the infralimbic cortex produces anxiety-like behaviors. Neuropharmacology. 72:148-156.
A-S11. Walf AA, Frye CA (2007): The use of the elevated plus maze as an assay of anxiety-related behavior in rodents. Nat Protoc. 2:322-328.
A-S12. David DJ, Samuels BA, Rainer Q, Wang JW, Marsteller D, Mendez I, et al. (2009): Neurogenesis-dependent and -independent effects of fluoxetine in an animal model of anxiety/depression. Neuron. 62:479-493.
A-S13. Guiard BP, Chenu F, El Mansari M, Blier P (2011): Characterization of the electrophysiological properties of triple reuptake inhibitors on monoaminergic neurons. Int J Neuropsychopharmacol. 14:211-223.
A-S14. Tritschler L, Kheirbek MA, Dantec YL, Mendez-David I, Guilloux JP, Faye C, et al. (2018): Optogenetic activation of granule cells in the dorsal dentate gyrus enhances dopaminergic neurotransmission in the Nucleus Accumbens. Neurosci Res. 134:56-60.
A-S15.Kheirbek MA, Drew LJ, Burghardt NS, Costantini DO, Tannenholz L, Ahmari SE, et al. (2013): Differential control of learning and anxiety along the dorsoventral axis of the dentate gyrus. Neuron. 77:955-968.

## Claims

1. A pharmaceutical composition comprising 4-amino-5-chloro-2,3-dihydro-N-[1-3-methoxypropyl)-4-piperidinyl]-7-benzofuran carboxamide monohydrochloride (prucalopride), 4-[4-[4-Tetrahydrofuran-3-yloxy)-benzo[d]isoxazol-3-yloxymethyl]-piperidin-1-ylmethyl]-tetrahydropyran-4-ol (PF-04995274), or a combination thereof, for use in a method for preventing a stress-induced affective disorder or stress-induced psychopathology selected from stress-induced fear and depressive-like behavior and associated affective disorders in a subject prior to a stressor, wherein the pharmaceutical composition is administered to the subject 48 hours to 3 weeks prior to the stressor.

2. The pharmaceutical composition for use according to claim 1, wherein the pharmaceutical composition is administered to the subject about 72 hours to about 2 weeks prior to the stressor.

3. The pharmaceutical composition for use according to any one of preceding claims, wherein the pharmaceutical composition is administered to the subject about 1 week prior to the stressor.

4. The pharmaceutical composition for use according to any one of preceding claims, wherein the pharmaceutical composition is administered to the subject once prior to the stressor.

5. The pharmaceutical composition for use according to any one of preceding claims, wherein the pharmaceutical composition is administered orally, intravenously, intranasally, or via injection to the subject.

6. The pharmaceutical composition for use according to any one of the preceding claims, wherein the method further comprises administering an effective amount of an antidepressant, an anxiolytic, or combinations thereof.

7. The pharmaceutical composition for use according to any one of the preceding claims, wherein the method further comprises administering an effective amount of a selective serotonin reuptake inhibitor (SSRI), or a pharmaceutically acceptable salt thereof.

8. The pharmaceutical composition for use according to any one of the preceding claims, wherein the method further comprises administering an effective amount of fluoxetine, paroxetine, sertraline, lithium, riluzole, prazosin, lamotrigine, ifenprodil, or a combination thereof.

## Patentansprüche

1. Pharmazeutische Zusammensetzung, umfassend 4-Amino-5-chlor-2,3-dihydro-N-[1-3-methoxypropyl)-4-piperidinyl]-7-benzofurancarboxamidmonohydrochlorid (Prucaloprid), 4-[4-[4-Tetrahydrofuran-3-yloxy)-benzo[d]isoxazol-3-yloxymethyl]-piperidin-1-ylmethyl]tetrahydropyran-4-ol (PF-04995274) oder eine Kombination davon, zur Verwendung in einem Verfahren zum Verhindern einer stressinduzierten affektiven Störung oder stressinduzierten Psychopathologie, ausgewählt aus stressinduzierter Angst und Depression-ähnlichem Verhalten, und damit verbundenen affektiven Störungen bei einem Subjekt vor einem Stressor, wobei die pharmazeutische Zusammensetzung dem Subjekt 48 Stunden bis 3 Wochen vor dem Stressor verabreicht wird.

2. Pharmazeutische Zusammensetzung zur Verwendung nach Anspruch 1, wobei die pharmazeutische Zusammensetzung dem Subjekt etwa 72 Stunden bis etwa 2 Wochen vor dem Stressor verabreicht wird.

3. Pharmazeutische Zusammensetzung zur Verwendung nach einem der vorhergehenden Ansprüche, wobei die pharmazeutische Zusammensetzung dem Subjekt etwa 1 Woche vor dem Stressor verabreicht wird.

4. Pharmazeutische Zusammensetzung zur Verwendung nach einem der vorhergehenden Ansprüche, wobei die pharmazeutische Zusammensetzung dem Subjekt einmal vor dem Stressor verabreicht wird.

5. Pharmazeutische Zusammensetzung zur Verwendung nach einem der vorhergehenden Ansprüche, wobei die pharmazeutische Zusammensetzung dem Subjekt oral, intravenös, intranasal oder mittels Injektion verabreicht wird.

6. Pharmazeutische Zusammensetzung zur Verwendung nach einem der vorhergehenden Ansprüche, wobei das Verfahren ferner Verabreichen einer wirksamen Menge eines Antidepressivums, eines Anxiolytikums oder Kombinationen davon umfasst.

7. Pharmazeutische Zusammensetzung zur Verwendung nach einem der vorhergehenden Ansprüche, wobei das Verfahren ferner Verabreichen einer wirksamen Menge eines selektiven Serotonin-Wiederaufnahme-Inhibitors (SSRI) oder eines pharmazeutisch unbedenklichen Salzes davon umfasst.

8. Pharmazeutische Zusammensetzung zur Verwendung nach einem der vorhergehenden Ansprüche, wobei das Verfahren ferner Verabreichen einer wirksamen Menge von Fluoxetin, Paroxetin, Sertralin, Lithium, Riluzol, Prazosin, Lamotrigin, Ifenprodil oder einer Kombination davon umfasst.

## Revendications

1. Composition pharmaceutique comprenant du monochlorhydrate de 4-amino-5-chloro-2,3-dihydro-N-[1-3-méthoxypropyl)-4-pipéridinyl]-7-benzofurane carboxamide (prucalopride), du 4-[4-[4-tétrahydrofuran-3-yloxy)-benzo[d]isoxazol-3-yloxyméthyl]-pipéridin-1-ylméthyl]tétrahydropyran-4-ol (PF-04995274), ou une combinaison de ceux-ci, destinée à être utilisée dans un procédé de prévention d'un trouble affectif induit par le stress ou d'une psychopathologie induite par le stress choisi(e) parmi la peur induite par le stress et le comportement de type dépressif et les troubles affectifs associés chez un sujet avant un facteur de stress, dans laquelle la composition pharmaceutique est administrée au sujet 48 heures à 3 semaines avant le facteur de stress.

2. Composition pharmaceutique destinée à être utilisée selon la revendication 1, dans laquelle la composition pharmaceutique est administrée au sujet environ 72 heures à environ 2 semaines avant le facteur de stress.

3. Composition pharmaceutique destinée à être utilisée selon l'une quelconque des revendications précédentes, dans laquelle la composition pharmaceutique est administrée au sujet environ 1 semaine avant le facteur de stress.

4. Composition pharmaceutique destinée à être utilisée selon l'une quelconque des revendications précédentes, dans laquelle la composition pharmaceutique est administrée au sujet une fois avant le facteur de stress.

5. Composition pharmaceutique destinée à être utilisée selon l'une quelconque des revendications précédentes, dans laquelle la composition pharmaceutique est administrée par voie orale, intraveineuse, intranasale ou par injection au sujet.

6. Composition pharmaceutique destinée à être utilisée selon l'une quelconque des revendications précédentes, dans laquelle le procédé comprend en outre l'administration d'une quantité efficace d'un antidépresseur, d'un anxiolytique ou de combinaisons de ceux-ci.

7. Composition pharmaceutique destinée à être utilisée selon l'une quelconque des revendications précédentes, dans laquelle le procédé comprend en outre l'administration d'une quantité efficace d'un inhibiteur sélectif de la recapture de la sérotonine (ISRS), ou d'un sel acceptable pharmaceutiquement de celui-ci.

8. Composition pharmaceutique destinée à être utilisée selon l'une quelconque des revendications précédentes, dans laquelle le procédé comprend en outre l'administration d'une quantité efficace de fluoxétine, de paroxétine, de sertraline, de lithium, de riluzole, de prazosine, de lamotrigine, d'ifenprodil ou d'une combinaison de ceux-ci.
